(19) Europäisches Patentamt
European Patent Office
Office européen des brevets

(11) EP 1 325 915 A1

# (12) EUROPEAN PATENT APPLICATION

(43) Date of publication:
**09.07.2003 Bulletin 2003/28**

(51) Int Cl.[7]: **C07D 217/02**, C07D 217/04

(21) Application number: **03005979.4**

(22) Date of filing: **19.07.1995**

(84) Designated Contracting States:
**AT BE CH DE DK ES FR GB GR IE IT LI LU MC NL PT SE**

(30) Priority: **22.07.1994 US 279291**
**22.07.1994 US 279339**

(62) Document number(s) of the earlier application(s) in accordance with Art. 76 EPC:
**95928091.8 / 0 772 595**

(71) Applicant: **THE UNITED STATES OF AMERICA, as represented by THE SECRETARY, Department of Health and Human Services**
**Bethesda, MD 20892-9902 (US)**

(72) Inventors:
- **Bringmann, Gerhard**
  **97074 Würzburg (DE)**
- **Harmsen, Sven**
  **23562 Lübeck (DE)**
- **Gotz, Roland**
  **91541 Rothenburg (DE)**
- **Boyd, Michael R.**
  **Ijamsville, MD 21754 (US)**

(74) Representative: **Stuart, Ian Alexander et al**
**MEWBURN ELLIS**
**York House**
**23 Kingsway**
**London WC2B 6HP (GB)**

Remarks:
This application was filed on 18 - 03 - 2003 as a divisional application to the application mentioned under INID code 62.

## (54) Monomeric and dimeric naphtylisoquinoline alkaloids and synthesis methods thereof

(57) The present invention provides methods of preparing monomeric naphthylisoquinoline alkaloids, including the antiparasitic korupensamines and related compounds, as well as non-korupensamines and other monomeric naphthylisoquinoline alkaloids. The present invention also provides methods of preparing dimeric naphthylisoquinoline alkaloids by coupling together two monomeric naphthylisoquinoline alkaloids, each of which may be the same or different, and one, both, or neither of which may possess a C-8' to C-5 naphthalene/isoquinoline linkage, to form homodimers or heterodimers, including the antiviral michellamines. The present invention further provides new, medically useful monomeric naphthylisoquinoline compounds and homodimeric and heterodimeric naphthylisoquinoline compounds and derivatives thereof.

EP 1 325 915 A1

## Description

TECHNICAL FIELD OF THE INVENTION

The present invention relates to methods of preparing known and new monomeric and dimeric naphthylisoquinoline alkaloids. The present invention also relates to new monomeric and dimeric naphthylisoquinoline alkaloids and derivatives thereof.

BACKGROUND OF THE INVENTION

Novel compounds exhibiting impressive antiviral and/or antiparasitic properties have recently been described (Manfredi et al., J. Med. Chem., 34, 3402-3405 (1991); Bringmann et al., Angew. Chem. Int. Ed. Eng., 32, 1190-1191 (1993); Boyd et al., J. Med. Chem., 37, 1740-1745 (1994); Boyd et al., PCT Application WO 94/24108; Bringmann et al., Tetrahedron, 50, 7807-7815 (1994); Hallock et al., J. Org. Chem., 59, 6349-6355 (1994); Bringmann et al., Heterocycles, 39, 503-512 (1994); Bringmann et al., Tetrahedron, 50, 9643-9648 (1994); François et al., Phytochemistry, 35, 1461-1464 (1994); François et al., PCT Application PCT/US95/01717; Boyd et al., U.S. Patent 5,409,938). These compounds are members of a general class known as naphthylisoquinoline alkaloids (Bringmann, The Alkaloids, Vol. 29 (Brossi, ed.), Academic Press, New York, 1986, pp. 141-184), and can further be characterized based on their structure as either monomeric alkaloids (or "monomers") or dimeric alkaloids (or "dimers").

Monomeric naphthylisoquinoline alkaloids include korupensamines or related monomeric naphthylisoquinoline alkaloids and derivatives thereof, which typically possess a C-8' to C-5 naphthalene/isoquinoline linkage, and non-korupensamines or other monomeric naphthylisoquinoline alkaloids and derivatives thereof, which typically lack a C-8' to C-5 naphthalene/isoquinoline linkage.

Korupensamines are novel, medically useful antiparasitic compounds which have recently been described (Hallock et al., 1994, supra; Bringmann et al., Heterocycles, 1994, supra; Boyd et al., U.S. Patent 5,409,938). A method of chemically synthesizing korupensamines and related C-8' to C-5 linked naphthylisoquinoline alkaloids has heretofore been unknown. This is significant because the only known natural source of any korupensamine is the rare tropical vine *Ancistrocladus korupensis* of Central Africa (Thomas and Gereau, Novon, 3, 494-498 (1993); Hallock et al., 1994, supra). This lack of any synthetic access to korupensamines undoubtedly has been a critical hindrance to the medical use of the compounds, and to the exploration of alternative medical uses as well.

In addition to the korupensamines, a series of other medically useful monomeric naphthylisoquinoline alkaloids and derivatives thereof has been described (François et al., 1994, supra; François et al., PCT Application PCT/US95/01717). Similar to the korupensamines, these "non-korupensamine" monomeric naphthylisoquinoline alkaloids have been in exceedingly limited supply since they also are obtained from scarce plants having a limited geographic distribution.

Dimeric naphthylisoquinoline alkaloids include michellamines, which, based on their molecular structure, are comprised of two monomeric alkaloids coupled together (e.g., two monomeric or molecular "halves"). Furthermore, a given michellamine may be either "homodimeric" (comprised of two monomeric halves which are the same) or "heterodimeric" (comprised of two monomeric halves which are different).

Dimeric naphthylisoquinoline alkaloids, as exemplified by the michellamines, have highly desirable and eminently useful medicinal properties that for the most part are distinct from the properties of the monomeric naphthylisoquinoline alkaloids which comprise their molecular halves. For example, the michellamines, such as michellamine B (Boyd et al., PCT Application WO 94/24108; Boyd et al., 1994, supra), are highly effective inhibitors of the replication and resultant destructive effects of the human immunodeficiency virus (HIV) in human immune cells. The range of anti-HIV activity observed for these dimeric alkaloids is exceptionally broad, encompassing both the major viral types, HIV-1 and HIV-2, as well as diverse HIV strains, and can be observed in different host cells (Boyd et al., 1994, supra).

Moreover, the dimeric alkaloids would appear to comprise a novel antiviral drug class in that the mechanism of action of the michellamines is distinct from any mechanism previously described. Specifically, the mechanism involves at least two components: (1) an inhibition of the viral reverse transcriptase, and (2) an inhibition of the virus-cell and cell-cell fusion processes (McMahon et al., Antimicrob. Agents Chemother., 39, 484-488 (1995)). This suggests that the dimeric alkaloids may prove effective not only in the prevention of nascent viral infection, but also in the prevention of the replication and spread of the virus *in vivo* and in the prevention of syncytia formation which has been observed *in vitro* and which may mediate the depletion of T4 immune cells which occurs *in vivo.*

In addition to possessing medicinally desirable properties, dimeric alkaloids are also quite attractive from a pharmacological and toxicological standpoint. *In vivo* doses of michellamine B that are non-toxic result in a level of the drug in the blood which is well in excess of its effective antiviral concentration (Supko et al., Anal. Biochem., 216, 52-60 (1994); Supko et al., Antimicrob. Agents Chemother., 39, 9-14 (1995).

In contrast, the monomeric naphthylisoquinoline alkaloids appear to be devoid of anti-HIV activity. However,

the monomeric alkaloids instead have potent antiparasitic properties as exhibited by their bacteriocidal activity against strains of malaria. In this respect, it is interesting to speculate that a trace of this antiparasitic activity may be imparted to the alkaloid dimer by its constituent monomeric halves, as a few of the dimeric naphthylisoquinoline alkaloids (e.g., the michellamines) also appear weakly antiparasitic (Boyd et al., U.S. Patent 5,409,938; François et al., PCT Application PCT/US95/01717; François et al., 1994, supra).

Unfortunately, attempts by researchers to maximally exploit the potential of the dimeric alkaloids through development of antiviral and antiparasitic therapy and unprecedented uses for the alkaloids have been hindered by the lack of significant access to the dimeric alkaloids. To date, the only known natural source of the dimeric alkaloids is the rare tropical vine *Ancistrocladus korupensis* (Thomas and Gereau, 1993, supra; Boyd et al., 1994, supra; Hallock et al., 1994, supra). Monomeric naphthylisoquinoline alkaloids do not spontaneously combine or couple together to form the dimeric alkaloids, and a method of converting (e.g., coupling) monomeric alkaloids or derivatives thereof to form a dimeric alkaloid or derivative has heretofore been unknown. Indeed, the naturally occurring michellamines A, B, and C (Boyd et al., 1994, supra) and frustratingly simple derivatives prepared directly therefrom (see, e.g., Boyd et al., PCT Application WO 94/24108) have been the only known dimeric naphthylisoquinoline alkaloids from any source. Alternative dimeric alkaloids, if such be obtained, might present particularly advantageous medicinal properties, such as increased potency, increased host range, increased range of therapeutic action, and the like.

Accordingly, it is an object of the present invention to provide methods of chemically synthesizing known and new monomeric naphthylisoquinoline alkaloids, including korupensamines, derivatives thereof, and other monomeric naphthylisoquinolines (i.e., those lacking a C-8' to C-5 naphthalene/isoquinoline linkage).

Correspondingly, it is another object of the present invention to provide new monomeric naphthylisoquinoline compounds. Such compounds have particular use as therapeutic agents, for instance, as antiparasitic agents, and/or may serve as precursors or building blocks for the synthesis of dimeric naphthylisoquinoline compounds, such as exemplified by the michellamines (Bringmann et al., Tetrahedron, 1994, supra; Boyd et al., 1994, supra), which have proven to be medically useful.

In addition, it is an object of the present invention to provide methods of synthesizing known and new dimeric alkaloids, including homodimeric and heterodimeric naphthylisoquinoline alkaloids.

Correspondingly, it is another object of the present invention to provide new dimeric naphthylisoquinoline alkaloids. Such compounds have particular use as therapeutic agents, for instance, as antiviral and antiparasitic agents, as exemplified by the michellamines (Bringmann et al., Tetrahedron, 1994, supra; Boyd et al., 1994, supra; François et al., PCT Application PCT/US95/01717).

These and other objects and advantages of the present invention, as well as additional inventive features, will be apparent from the description of the invention provided herein.

## BRIEF SUMMARY OF THE INVENTION

The present invention provides a method of preparing a monomeric naphthylisoquinoline alkaloid comprising (a) preparing a naphthalene building block with at least one activation group at a coupling site and, optionally, with at least one protective group as a precursor to a free hydroxyl group desired in the monomeric naphthylisoquinoline alkaloid, (b) preparing an isoquinoline building block with at least one activation group at a coupling site and, optionally, with at least one protective group as a precursor to a free hydroxyl group desired in the monomeric naphthylisoquinoline alkaloid, wherein the isoquinoline building block is a tetrahydroisoquinoline, dihydroisoquinoline, or fully aromatic isoquinoline building block, and (c) coupling the naphthalene and isoquinoline building blocks at the coupling sites to form the monomeric naphthylisoquinoline alkaloid. The present invention also provides new monomeric naphthylisoquinoline alkaloids and derivatives thereof.

The present invention further provides a method of preparing a compound which comprises (a) selecting first and second naphthylisoquinoline alkaloid monomers, which are either the same or different, (b) optionally introducing protective group(s) at desired site(s) in the monomers, (c) introducing activation group(s) at the desired coupling site (s) of the monomers if needed for coupling of the monomers, (d) coupling the first and second monomers to form a dimeric naphthylisoquinoline alkaloid, and (e) optionally removing the protective group(s) from the dimeric naphthylisoquinoline alkaloid. In addition, the present invention provides new dimeric naphthylisoquinoline alkaloids, as well as derivatives thereof.

## BRIEF DESCRIPTION OF THE DRAWINGS

**Figure 1** illustrates the structures of non-korupensamines and other monomeric naphthylisoquinoline alkaloids lacking a C-8' to C-5 naphthalene/isoquinoline linkage.

**Figure 2** illustrates the preparation of the naphthalene building block 1-bromo-4-isopropoxy-5-methoxy-7-methylnaphthalene, which comprises the first stage of the method for preparation of korupensamines A and B. Reaction conditions: (a) NaH, THF, 24 h, 0°C RT, 61%; (b) $CF_3CO_2H/H_2O$, 10 h, RT, 99%; (c) $Ac_2O$, 14 h reflux; (d) EtOH/EtONa, THF, 1 h, 95% from **3**; (e) $Me_2SO_4$, acetone, $K_2CO_3$, reflux, 15 h, 99%; (f) $LiAlH_4$, THF, 1 h, 0°C, 95%; (g) $(BrCCl_2)_2$ (Bringmann and Schneider, Synthesis, 139-141 (1983)), $CH_2Cl_2$, 30 min, RT, 95%; (h) L-selectride, $CH_2Cl_2$, 2.5h, 0°C, 98%.

**Figure 3** illustrates the preparation of the isoquinoline building block **7**, which comprises the second stage of the method for preparation of korupensamines A and B. Reaction conditions: (a) BnBr, 2N NaOH, $CH_2Cl_2$, $nBu_3NBnCl$, 5 h, RT; (b) *i*PrSNa, DMF, NaOH 150°C, 5 h, 56% from **6;** (c) BnBr, 2 N NaOH, $CH_2Cl_2$, $nBu_3NBnCl$, 2 h, RT, 91%; (d) $Br_2$, DMF, 3 d, RT, 94%.

**Figure 4** illustrates the convergent construction of the monomeric naphthylisoquinoline alkaloids, which comprise the third and final stage of the method for preparation of korupensamines A and B. Reaction conditions: (a) tBuLi, $Bu_3SnCl$, THF, 2 h, -78°C, RT, 89%; (b) $PdCl_2(PPh_3)_2$, $PPh_3$, LiCl, Cu(I)Br, DMF, 40 h, 135°C, 15%; (c) $BCl_3$, $CH_2Cl_2$, 10 min, RT; (d) $H_2$, Pd/C, MeOH; (e) atropodiastereomer separation according to Hallock et al. (1994, supra).

**Figure 5** illustrates the synthesis of ancistrobrevine B using the intermolecular coupling strategy of the present invention as described in Example 2. Reaction conditions: (a) $PdCl_2(PPh_3)_2$, LiCl, Cu(I)Br, DMF, 135°C; (b) $H_2$, Pd/C, MeOH.

**Figure 6** illustrates the synthesis of dioncopeltine A using the intermolecular coupling strategy of the present invention. Reaction conditions: (a) $PdCl_2(PPh_3)_2$, LiCl, Cu(I)Br, DMF, 135°C; (b) $BCl_3$ c) $H_2$, Pd/C, MeOH.

**Figure 7** illustrates the synthesis of dioncophylline C using the intermolecular coupling strategy of the present invention. Reaction conditions: (a) $PdCl_2(PPh_3)_2$, LiCl, Cu(I)Br, DMF, 135°C; (b) $BCl_3$; (c) $H_2$, Pd/C, MeOH.

**Figure 8** illustrates some oxidative coupling procedures to make a medically useful dimeric naphthylisoquinoline alkaloid from a monomeric naphthylisoquinoline precursor.

**Figure 9** illustrates an electrochemical coupling procedure to make a medically useful dimeric naphthylisoquinoline alkaloid from a monomeric naphthylisoquinoline precursor.

**Figure 10** illustrates a chemical reductive coupling procedure to make a medically useful dimeric naphthylisoquinoline alkaloid from a monomeric naphthylisoquinoline precursor.

**Figure 11** illustrates a "redox-neutral" chemical coupling procedure to make a medically useful dimeric naphthylisoquinoline alkaloid from a monomeric naphthylisoquinoline precursor. Due to the electrophilic/nucleophilic character of the two appropriately prepared monomeric naphthylisoquinoline alkaloids, this procedure is particularly suited for the directed cross-coupling of two different monomeric alkaloids to give heterodimeric naphthylisoquinoline compounds.

**Figure 12** illustrates a method for preparation of michellamine A through the oxidative dimerization of korupensamine A (**10a**) via its derivative **11**. Reaction conditions: (a) $(CH_3)_3CO_2CHO$, $CH_2Cl_2$ 20°C; (b) $CH_3COCl$, $Et_3N$, cat. DMAP, $CH_2Cl_2$, 92% from **10a**; (c) $Ag_2O$, 0.2% $Et_3N$ in $CHCl_3$, 73%; (d) $NaBH_4$, *i*PrOH, 25°C; (e) MeOH/HCl, reflux, 67% from **12.**

**Figure 13** illustrates some representative examples of variations in structures of the dimeric naphthylisoquinoline alkaloids which can be obtained according to the methods of the present invention.

**Figure 14** illustrates a method for preparation of a representative new dimeric naphthylisoquinoline alkaloid. Shown specifically is a homodimer comprised of two monomeric dioncopeltine A "halves". Each of the halves lacks a C-8' to C-5 naphthalene/isoquinoline linkage.

**Figure 15** illustrates some other representative examples of variations in the structures of the dimeric naphthylisoquinoline alkaloids which can be obtained according to the methods of the present invention.

**Figure 16** illustrates a synthetic scheme for the preparation of isoquinoline-coupled versus naphthalene-coupled dimers of dioncophylline A.

**Figure 17** illustrates a synthetic scheme for the preparation of isoquinoline-coupled versus naphthalene-coupled dimers of ancistrocladine.

**Figure 18** illustrates the structures of various michellamines.

## DETAILED DESCRIPTION OF THE INVENTION

The present invention provides methods of preparing both known and new monomeric and dimeric naphthylisoquinoline alkaloids and derivatives thereof. The present invention also provides new monomeric and dimeric naphthylisoquinoline alkaloids and derivatives thereof.

Definitions

For clarification of the chemical structures described herein, the following definitions apply.

By "korupensamine" or "related monomeric naphthylisoquinoline alkaloids" is meant a monomeric naphthylisoquinoline alkaloid possessing a C-8' to C-5 naphthalene/isoquinoline linkage.

By "non-korupensamine" or "other monomeric naphthylisoquinoline alkaloids" is meant a monomeric naphthylisoquinoline alkaloid which lacks a C-8' to C-5 naphthalene/isoquinoline linkage.

By naphthylisoquinoline homodimers is meant a dimeric alkaloid containing two monomeric naphthylisoquinoline halves, wherein each half is the same.

By naphthylisoquinoline heterodimers is meant a dimeric alkaloid containing two monomeric naphthylisoquinoline halves, wherein each half is different.

By $C_1$-$C_6$ alkyl is meant straight or branched-chain $C_1$-$C_6$ alkyl groups. Examples include, but are not limited to, methyl, ethyl, n-propyl, isopropyl, n-butyl, isobutyl, *sec*-butyl, tertiary-butyl, n-pentyl, isopentyl, and n-hexyl.

By aryl is meant an organic radical derived from an aromatic hydrocarbon. Examples of aryl groups include phenyl and *o*-, *m*-, and *p*-hydroxyphenyl.

By aliphatic is meant an organic radical derived from an open hydrocarbon chain. Examples of aliphatic radicals include alkanes, alkenes, and alkynes. Specific examples of aliphatic radicals which can be used in the present invention include, but are not limited to, $C_1$-$C_6$ alkyl radicals, straight or branched.

Structures

To make it easier to compare naphthylisoquinoline alkaloids of the present invention of different coupling types, contrary to IUPAC numbering conventions, and consistent with previous work by the inventors (Bringmann et al., Phytochemistry, 30, 3845-3847 (1991)), the naphthalene portion of the alkaloids is hereinafter numbered in the same way. In other words, there is always attributed a 2-methyl-4,5-dioxy-substitution pattern to the naphthalene, independent from the site of the axis.

Medical Uses

The new monomeric and dimeric naphthylisoquinoline alkaloids and derivatives thereof are expected to have at least those medicinal properties possessed by the previously known monomeric and dimeric naphthylisoquinoline alkaloids. (see, e.g., Boyd et al., PCT Application WO 94/24108; Boyd et al., 1994, supra). However, depending upon the particular disease and host to be treated, a compound of the present invention will be distinctly advantageous in a given situation.

The medically useful properties of the new compounds of the present invention can be readily confirmed by one knowledgeable and skilled in the art by use of any of a variety of methods which have been published or otherwise disclosed elsewhere. For example, antiviral properties, particularly anti-HIV properties, can be confirmed as described in Boyd et al., J. Med. Chem., 1994, supra, and Boyd et al., PCT Application WO 94/24108. Also, for example, *in vitro* and *in vivo* antimalarial activity may be confirmed as described in François et al., Phytochemistry, 35, 1461-1464 (1994), Gulakowski et al., J. Virol. Methods, 33, 309-313 (1991), François et al., PCT Application PCT/US95/01717, and Boyd et al., U.S. Patent 5,409,938.

Synthesis of Monomeric Naphthylisoquinoline Alkaloids

The present inventive method of preparing a monomeric naphthylisoquinoline alkaloid comprises:

(a) preparing a naphthalene building block with at least one activation group at a coupling site and, optionally, with at least one protective group as a precursor to a free hydroxyl group desired in the monomeric naphthylisoquinoline alkaloid,

(b) preparing an isoquinoline building block with at least one activation group at a coupling site and, optionally, with at least one protective group as a precursor to a free hydroxyl group desired in the monomeric naphthylisoquinoline alkaloid, wherein the isoquinoline building block is a tetrahydroisoquinoline, dihydroisoquinoline, or fully aromatic isoquinoline building block, and

(c) coupling the naphthalene and isoquinoline building blocks at the coupling sites to form the monomeric naphthylisoquinoline alkaloid.

Preferably, the present inventive method further comprises:

(d) removing the protective groups from the monomeric naphthylisoquinoline alkaloid, and
(e) purifying the monomeric naphthylisoquinoline alkaloid.

Any suitable building blocks can be utilized. The building blocks can have the configurations at any chiral centers as desired in the monomeric naphthylisoquinoline alkaloid. When the isoquinoline building block is a tetrahydroisoquinoline building block, the tetrahydroisoquinoline building block preferably has methyl groups at C-1 and C-3 and can have the *R*-configuration at C-1 and the *R*-configuration at C-3, the *S*-configuration at C-1 and the *R*-configuration at C-3, the *R*-configuration at C-1 and the *S*-configuration at C-3, or the *S*-configuration at C-1 and the *S*-configuration at C-3. Similarly, when the isoquinoline building block is a dihydroisoquinoline building block, the dihydroisoquinoline building block preferably has a methyl group at C-3 and can have either the *S*-configuration or the *R*-configuration at C-3.

Any suitable activation and protective groups can be utilized with respect to the building blocks. One of the activation groups, either on the naphthalene or isoquinoline building block, will generally be a nucleophilic activation group, while the other of the activation groups, on the other building block, will generally be an electrophilic activation group. The nucleophilic activation group is preferably selected from the group consisting boronic acid and trialkylstannyl groups. The electrophilic activation group is preferably selected from the group consisting of halogen and 0-triflate leaving groups. The protective group is preferably an isopropyl group.

The aforementioned process steps can be carried out by any suitable means. Thus, for example, the coupling can be effected by several means, such as by transition metal catalysis, especially by using Pd. Also, the monomeric naphthylisoquinoline alkaloid can be purified by a variety of means, preferably by HPLC. The purification of the monomeric naphthylisoquinoline alkaloid is more preferably carried out by HPLC on an amino-bonded or other phase column so as to obtain a pure atropodiastereomer. Moreover, while the purification process is preferably carried out after removal of the protective groups, the purification process can be carried out either before or after the removal of the protective groups.

The distinct novelty of the aforementioned synthetic strategy includes both the intermolecular biaryl coupling of intact isoquinolines and naphthalenes (only Sargent in Australia did intermolecular coupling, but never managed to do that with the intact halves, cp. Rizzacasa and Sargent, J. Chem. Soc. Chem. Commun., 301 (1989)) and also the use of the isopropyl and related protective groups as a precursor to the free OH-group on the naphthalene portion of the alkaloid. The latter is the basis for the synthesis of the naphthalene portion of monomeric alkaloids which possess the OH/OMe-pattern on the naphthalene portion of the alkaloid.

The preparation of a korupensamine or related monomeric naphthylisoquinoline alkaloid containing the C-8' to C-5 naphthalene/isoquinoline linkage comprises the aforedescribed general method wherein the coupling of the naphthalene and isoquinoline building blocks is by forming a C-8' to C-5 naphthalene/isoquinoline linkage, preferably wherein the activation group for the naphthalene building block is trialkylstannyl, the activation group for the isoquinoline building block is bromine, and the protective group is an isopropyl group. Similarly, the preparation of a non-korupensamine or other monomeric naphthylisoquinoline alkaloid which does not contain the C-8' to C-5 naphthalene/isoquinoline linkage comprises the aforedescribed general method wherein the coupling of the naphthalene and isoquinoline building blocks is by forming a naphthalene/isoquinoline linkage other than a C-8' to C-5 naphthalene/isoquinoline linkage, preferably wherein the activation group for the naphthalene building block is trialkylstannyl or a boronic acid group, the activation group for the isoquinoline building block is a halogen or an *O*-triflate leaving group, and the protective group is an isopropyl group.

## Korupensamines, Related Monomeric Naphthylisoquinoline Alkaloids, and Derivatives Thereof

The korupensamines and korupensamine derivatives of the present invention are chemically unique in several respects. Their basic structure comprises a biaryl system consisting of a tetrahydrogenated isoquinoline moiety with an unprecedented methyl group at C-3. Moreover, these alkaloids display atropisomerism due to the bulky ortho-substituents adjacent to the biaryl axis. Such highly unusual structures presumably result from an unprecedented biogenetic origin, for which a polyketide pathway has been implicated (Bringmann, The Alkaloids, Vol 29 (Brossi, ed.), Academic Press, New York, 1986, pp. 141-184; Bringmann et al., Planta Med., 57, suppl. 2, 98 (1991)). The korupensamines and korupensamine derivatives of the present invention are unique among all heretofore known naphthylisoquinoline alkaloids in containing only one C-8' to C-5 linkage of a naphthalene group and a tetrahydroisoquinoline group, an *R* configuration at C-1, and an exceptionally high polarity.

Korupensamines and derivatives are medically useful, for example, as antimalarial agents (François et al., PCT Application PCT/US95/01717; Boyd et al., U.S. Patent 5,409,938). Also, such "monomeric" compounds are chemically useful as precursors to medically useful (e.g. antiviral) "dimeric" compounds, such as michellamines (Boyd et al., J. Med. Chem., 37, 1740-1745 (1994); Boyd et al., PCT Application WO 94/24108). Previously, the only known source of korupensamines was a rare African plant (see, e.g., Hallock et al., 1994, supra; Boyd et al., 1994, supra).

The following synthetic procedure for korupensamine A and B provides a specific illustration of the preparation of monomeric naphthylisoquinoline alkaloids having a C-8' to C-5 naphthalene/isoquinoline linkage. Characteristic structural demands for the synthesis of the korupensamines are not only the high degree of free OH and NH functions (and thus the need for appropriate protective groups), but also the unusual position of the biaryl axis. In contrast to the various related alkaloids previously prepared by the "lactone methodology" (see, e.g., Bringmann et al., Angew. Chem. Int. Ed. Engl., 25, 913-915 (1986); Bringmann and Reuscher, Angew. Chem. Int. Ed. Engl., 28, 1672-1673 (1989); Bringmann and Jansen, Synthesis, 825-827 (1991)), the monomeric naphthylisoquinolines, korupensamines A and B with the 8',5-coupling type do not have a $C_1$ side-chain in an *ortho*-position next to the axis. Consequently the efficient "lactone methodology" is not applicable to this target biaryl. Accordingly, the present invention provides an intermolecular coupling strategy for the construction of the axis. As a precursor to the isocyclic moiety of the korupensamines, with the free hydroxy function specifically only at C-5' and the methylether at C-4', the naphthalene building block **4**, with an *0*-isopropyl substituent as a protective group and the bromo substituent at the scheduled coupling position, is used. The method for construction of **4** is related to the preparation of related dimethoxy analogs except for the protective group strategy and is outlined in **Figure 2** and further described in Example 1.

**Figure 3** outlines the preparation of the next building block in the synthetic route to korupensamines A and B, the isoquinoline moiety. As a precursor for the heterocyclic moiety of the target molecules, the tetrahydroisoquinoline **7,** i.e., with benzyl groups for the protection of both N-and O-functionalities, and again a bromine substituent at the site of the scheduled biaryl coupling, is used. For its preparation, the correctly 1*R*.3*R*-configurated tetrahydroisoquinoline **6,** the directed synthesis of which from the aryl-propanone **5** has already been described (Bringmann et al., Liebigs Ann. Chem., 877-888 (1993)), is used. Whereas *O*-demethylation of **6** and subsequent double benzylation turns out to be very tedious, better results can be obtained by a stepwise *O*-benzylation/*O*-demethylation/*O*-benzylation sequence, followed by a regioselective bromination in the 5-position of the tetrahydroisoquinoline, to give **7**. Additional details are included in Example 1.

**Figure 4** outlines the Pd(II) catalyzed intermolecular biaryl coupling of the suitably protected and activated building blocks. For the activation of the coupling sites, considering options such as halogen or *O*-triflate leaving groups on the electrophilic partner and boronic acid or trialkylstannyl groups on the nucleophilic partner, the use of a brominated isoquinoline **7** and a stannylated naphthalene **8** turns out to be the combination of choice in the case of korupensamines A and B. The tribenzyl derivative **7** can be coupled with **8** in good yields, giving a mixture of the two atropodiastereomers **9a** and **9b**, which without resolution may then be immediately deprotected by treatment with $BCl_3$ to cleave the isopropyl and benzyl ether functions, and subsequently treated by catalytic hydrogenation to set free the amino group, giving a mixture of **10a** and **10b**. Final atropodiastereomeric separation can be achieved by HPLC on an amino-bonded phase column, as previously published (Hallock et al., 1994, supra). Additional details of the synthesis are found in Example 1.

The present inventive method can be used to prepare monomeric naphthylisoquinoline alkaloids other than korupensamines. For example, **Figure 5** outlines a potential route of the synthesis of ancistrobrevine B using the intermolecular coupling strategy of the present invention.

One skilled in the art will readily appreciate that certain chemical modifications can be incorporated as desired into the precursors in the aforementioned synthetic method and/or can be used to modify the end product thereof to obtain a useful new korupensamine or korupensamine derivative with modified biological properties. Such modified properties may include one or more of the following: greater therapeutic potency against a particular disease or disease-causing organism such as a parasite, particularly antimalarial potency, a broader spectrum of therapeutic activity against diverse diseases or disease-causing organisms such as a parasite, particularly against parasitic strains of malaria, enhanced oral bioavailability, less toxicity in a particular host mammal, more advantageous pharmacokinetics and/or tissue distribution in a given host mammal, and the like.

For example, a useful modified compound may be obtained by the methods of the present invention by selecting and constructing, and incorporating in the coupling step, an appropriately protected tetrahydroisoquinoline, dihydroisoquinoline, or fully aromatic isoquinoline building block having configurations at one or both of the chiral centers that are different from the corresponding naturally occurring korupensamines.

Moreover, by applying one or more chemical reactions (as disclosed in François et al., PCT Application PCT/US95/01717 and Boyd et al., U.S. Patent 5,409,938) to a given korupensamine or korupensamine derivative, a useful new derivative may be obtained wherein one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, a secondary amine site may instead be an amide, sulfonamide, tertiary amine, or alkyl quaternary ammonium salt or corresponding Hoffmann elimination product thereof, a tertiary amine site may instead be a secondary amine, and one or more aromatic hydrogen substituent (s) may instead be halo, nitro, amino, hydroxyl, thiol, or cyano.

Furthermore, by applying chemical reactions as disclosed herein to a given korupensamine or korupensamine derivative, a useful new derivative may be obtained wherein one or more aromatic hydrogen substituent(s) may instead be an acyl or $C_1$-$C_6$ alkyl substituent, and $CH_3$ may instead be H. These methods of derivatization are further described

in Example 2.

Accordingly, the present invention provides a compound of the formula

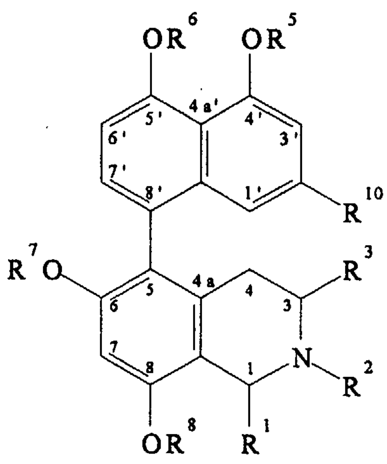

or

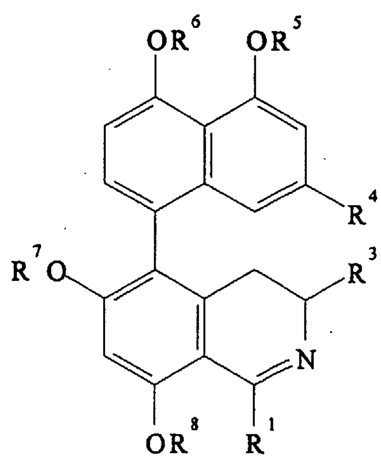

or

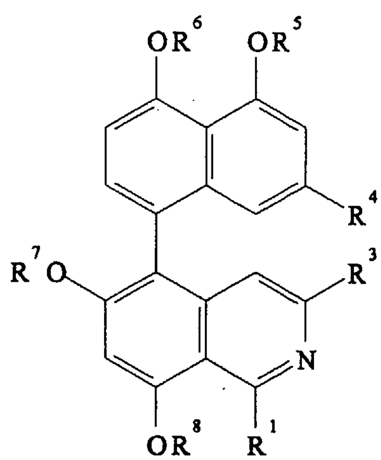

wherein $R^1$, $R^3$, $R^4$, and $R^{10}$ may be the same or different and each may be H or $C_1$-$C_6$ alkyl, $R^2$, $R^5$, $R^6$, $R^7$, and $R^8$ may be the same or different and each may be H, $C_1$-$C_6$ alkyl, $R^9CH_2$-, $R^9CO$-, or $R^9SO_2$-, $R^9$ may be H, $C_1$-$C_6$ alkyl, or aryl, and one or more of the ring positions 1, 3, 4, 1', 2', 3', 4', 5', 6', 7', 6, 7, and 8 may be substituted with halo, nitro, amino, hydroxyl, thiol, acyl, $C_1$-$C_6$ alkyl, or cyano, with the proviso that $R^{10}$ is not methyl when $R^1$ and $R^3$ are methyl.

The present invention also provides a compound of the same above-described structural formula wherein (a) $R^1$, $R^3$, $R^4$, and $R^{10}$ may be the same or different and each may be H or $C_1$-$C_6$ alkyl, $R^2$, $R^5$, $R^6$, $R^7$, and $R^8$ may be the same or different and each may be H, $C_1$-$C_6$ alkyl, $R^9CH_2$-, $R^9CO$-, or $R^9SO_2$-, $R^9$ may be H, $C_1$-$C_6$ alkyl, or aryl, and one or more of the ring positions 1, 3, 4, 1', 2', 3', 4' , 5' , 6' , 7' , 6, 7, and 8 may be substituted with halo, nitro, amino, hydroxyl, thiol, acyl, $C_1$-$C_6$ alkyl, or cyano, (b) one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, one or more secondary amine site (s) may instead be an amide, sulfonamide, tertiary amine, alkyl quaternary ammonium salt or corresponding Hoffmann elimination product thereof, a tertiary amine site may instead be a secondary amine, and one or more aromatic hydrogen substituent(s) may instead be halo, nitro, amino, hydroxyl, thiol, or cyano, and (c) one or more aromatic hydrogen substituent(s) is instead an acyl or $C_1$-$C_6$ alkyl substituent. With respect to such a compound, $R^{10}$ may or may not be methyl when $R^1$ and $R^3$ are methyl.

Non-Korupensamines, Other Monomeric Naphthylisoquinoline Alkaloids, and Derivatives Thereof

Numerous other naturally occurring, medically useful monomeric naphthylisoquinoline alkaloids and semisynthetic derivatives thereof have been identified (see, e.g., François et al., PCT Application PCT/US95/01717 and references therein). These other alkaloids generally differ from korupensamines in their lack of a C-8' to C-5 naphthalene/isoquinoline linkage. As for the korupensamines, a practical, synthetic route of access to many of such compounds has not previously been available.

The above generally applicable method, having certain specific modifications or adaptations introduced on a case-by-case basis, may be used to prepare by chemical synthesis naturally occurring monomeric naphthylisoquinoline alkaloids lacking a C-8' to C-5 naphthalene/isoquinoline linkage, such as but not limited to the compounds identified in **Table 1**.

Table 1.

| **Literature references reporting the chemical structures of naphthylisoquinoline alkaloids.** | |
|---|---|
| Compound Name | Reference Citation |
| Dioncophylline B | Bringmann et al., Phytochemistry, 30, 3845-3847 (1991) |
| Dioncopeltine A | Bringmann et al., Phytochemistry, 30, 1691-1696 (1991) |
| Dioncophylline C | Bringmann et al., Phytochemistry, 31, 4019-4024 (1992) |
| Dioncolactone A | Bringmann et al., Phytochemistry, 30, 1691-1696 (1991) |
| Ancistrobrevine D | Bringmann et al., Planta Med., 58 (suppl 1), 703-704 (1992) |
| 5'-O-Demethyl-8-*O*-methyl-7-*epi*-dioncophylline A | Bringmann et al., Phytochemistry, 36, 1057-1061 (1994) |
| 5'-*O*-Demethyl-7-*epi* dioncophylline A | Bringmann et al., Planta Med., 59 (suppl), 621-622 (1993) |
| (±)-Dioncophyllacine A | Bringmann et al., Phytochemistry, 31, 4015-4018 (1992) |
| Ancistrobrevine A | Bringmann et al., Planta Med., 58 (suppl 1), 703-704 (1992) |
| 6-*O*-Demethyl-ancistrobrevine A | (unpublished) |
| Ancistrobarterine A (6-*O*-Demethyl-8-*O*-methyl-7-*epi*-ancistrobrevine C) | Bringmann et al., Planta Med., 59 (suppl), 623-624 (1993) |
| *N*-Formyl-*O*,*O*-dimethyl-dioncophylline C | Bringmann et al., Phytochemistry, 31, 4019-4024 (1992) |
| *N*-Formyl-dioncophylline C | Bringmann et al., Phytochemistry, 31, 4019- |

Table 1. (continued)

| Literature references reporting the chemical structures of naphthylisoquinoline alkaloids. | |
|---|---|
| Compound Name | Reference Citation |
| | 4024 (1992) |
| *N*-Formyl-8-*O*-benzyl-dioncophylline C | Francois et al., PCT Application PCT/US95/01717 |
| *N*-Formyl-8-*O*-methyl-dioncophylline C Francois et al., PCT Application | |
| | PCT/US95/01717 |
| *N*-Formyl-8-*O*-pivaloyl-dioncophylline C | Francois et al., PCT Application PCT/US95/01717 |
| *N*-Formyl-8-*O*-acetyl-dioncophylline C | Francois et al., PCT Application PCT/US95/01717 |
| *N*-Formyl-8-*O*-benzoyl-dioncophylline C | Francois et al., PCT Application PCT/US95/01717 |
| 8-*O*-Methyl-dioncophylline C | Francois et al., PCT Application PCT/US95/01717 |

The structures of these compounds identified in **Table 1**, for which no synthetic method of preparation has heretofore been published or otherwise disclosed, are shown in **Figure 1**. Those compounds for which a synthetic method of preparation has heretofore been proposed, albeit quite different from the present inventive method, are identified in **Table 2**. The structures of these compounds are also depicted in **Figure 1.**

| Table 2. Literature references reporting the chemical structures of naphthylisoquinoline alkaloids. | |
|---|---|
| Compound Name | Reference Citation |
| Dioncophylline A | Bringmann et al., Tetrahedron Lett., 31, 639-642 (1990); Bringmann et al., Tetrahedron Lett., 31, 643-646 (1990) |
| N-Methyldioncophylline A | Bringmann et al., Phytochemistry, 30, 1307-1310 (1991) |
| Ancistrocladine | Bringmann, The Alkaloids, 29, 141-184 (1986) (and literature cited therein) |
| N-Methyldioncophylline A (atropisomers) | Bringmann et al., Phytochemistry, 30, 1307-1310 (1991) |
| Dioncophylleine A | Fleischhauer et al., Z. Naturforsch, 48b, 140-148 (1993) |
| Hamatine | Bringmann et al., The Alkaloids, 29, 141-184 (1986); Bringmann et al., Angew Chem., 25, 913 (1986); Bringmann et al., Heterocycles, 28, 137 (1989) (and literature cited therein) |
| 7-*epi*-Dioncophylline A | Bringmann et al., Tetrahedron Lett., 31, 643-646 (1990) |
| *N*-Formylancistrocladine | Bringmann et al., Phytochemistry, 31, 4019-4024 (1992) |
| *N*-Methylancistrocladine | Bringmann et al., Phytochemistry, 31, 4019-4024 (1992) (and literature cited therein) |
| 6-Deoxy-*N*-methylancistrocladine | Bringmann et al., Phytochemistry, 31, 4019-4024 (1992) |

Thus, the present invention provides a method of preparing a compound having a formula selected from the group consisting of dioncophylline B, dioncopeltine A, dioncophylline A, dioncophylline C, dioncolactone A, *N*-methyl-dioncophylline A, ancistrobrevine D, ancistrocladine, 5'-*O*-demethyl-8-*O*-methyl-7-*epi*-dioncophylline A, 5'-*O*-demethyl-7-*epi*-dioncophylline A, dioncophylleine A, (±)-dioncophyllacine A, hamatine, ancistrobrevine A, 6-*O*-demethyl-ancistrobrevine A, ancistrobarterine A, 7-*epi*-dioncophylline A, *N*-formyl ancistrocladine, *N*-methyl-ancistrocladine,

6-deoxy-*N*-methyl-ancistrocladine, *N*-formyl-*O*,*O*-dimethyldioncophylline C, *N*-formyl-dioncophylline C, *N*-formyl-8-*O*-benzyl-dioncophylline C, *N*-formyl-8-*O*-methyldioncophylline C, *N*-formyl-8-*O*-pivaloyl-dioncophylline C, *N*-formyl-8-*O*-acetyl-dioncophylline C, *N*-formyl-8-*O*-benzoyl-dioncophylline C, and 8-*O*-methyl-dioncophylline C, and related alkaloids and derivatives thereof wherein the configurations at C-1 and C-3 may instead be the same or different and each may be *R* or *S*, the coupling points. comprising the naphthalene/isoquinoline axis may be different, and the configuration about the axis may be different, i.e., *M* or *P*.

As more specific examples of the synthetic method of preparation, **Figures 6** and **7** illustrate potential routes of the synthesis of dioncopeltine A and dioncophylline C, respectively, using the intermolecular coupling strategy of the present invention. Similar to the korupensamine synthesis, the desired appropriately protected and activated naphthalene and isoquinoline building blocks are subjected to the conditions giving the desired intermolecular biaryl coupling, then followed by deprotection and separation, or separation then deprotection, to give the desired product.

Moreover, by applying one or more chemical reactions (as disclosed in François et al., PCT Application PCT/US95/01717 and Boyd et al., U.S. Patent 5,409,938) to a given non-korupensamine or non-korupensamine derivative, a useful new derivative may be obtained wherein one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, a secondary amine site may instead be an amide, sulfonamide, tertiary amine, or alkyl quaternary ammonium salt or corresponding Hoffmann elimination product thereof, a tertiary amine site may instead be a secondary amine, and one or more aromatic hydrogen substituent (s) may instead be halo, nitro, amino, hydroxyl, thiol, or cyano. By way of the present invention, new compounds may be obtained, wherein, with respect to the aforementioned compounds, one or more aromatic hydrogen substituent(s) is instead an acyl or $C_1$-$C_6$ alkyl substituent, at least one $CH_3$ is instead H, and/or the tetrahydroisoquinoline is instead a dihydroisoquinoline or a fully aromatic isoquinoline. These methods of derivatization are described in Example 2.

Accordingly, the present invention provides a compound having a formula selected from the group consisting of dioncophylline B, dioncopeltine A, dioncophylline A, dioncophylline C, dioncolactone A, *N*-methyl-dioncophylline A, ancistrobrevine D, ancistrocladine, 5'-*O*-demethyl-8-*O*-methyl-7-*epi*-dioncophylline A, 5'-*O*-demethyl-7-*epi*-dioncophylline A, dioncophylleine A, (±)-dioncophyllacine A, hamatine, ancistrobrevine A, 6-*O*-demethyl-ancistrobrevine A, ancistrobarterine A, 7-*epi*-dioncophylline A, *N*-formyl ancistrocladine, *N*-methyl-ancistrocladine, 6-deoxy-*N*-methyl-ancistrocladine, *N*-formyl-*O*,*O*-dimethyl-dioncophylline C, *N*-formyl-dioncophylline C, *N*-formyl-8-*O*-benzyl-dioncophylline C, *N*-formyl-8-*O*-methyl-dioncophylline C, *N*-formyl-8-*O*-pivaloyl-dioncophylline C, *N*-formyl-8-*O*-acetyl-dioncophylline C, *N*-formyl-8-*O*-benzoyl-dioncophylline C, and 8-*O*-methyl-dioncophylline C, wherein (a) the configurations at C-1 and C-3 may instead be the same or different and each may be *R* or *S,* the coupling points comprising the naphthalene/isoquinoline axis may be different, the configuration about the axis may be different, one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, and one or more secondary amine site(s) may instead be an amide, sulfonamide, tertiary amine, alkyl quaternary ammonium salt or corresponding Hoffmann elimination product thereof, one or more tertiary amine site(s) may instead be a secondary amine, and one or more aromatic hydrogen substituent(s) may instead be halo, nitro, amino, hydroxyl, thiol, or cyano, and (b) one or more aromatic hydrogen substituent(s) is instead an acyl or $C_1$-$C_6$ alkyl substituent, at least one $CH_3$ is instead H, and/or the tetrahydroisoquinoline is instead a dihydroisoquinoline or a fully aromatic isoquinoline.

## Monomeric Naphthylisoquinoline Monomers Employed as Precursors to Prepare Dimeric Naphthylisoquinoline Alkaloids

Methods have been described above for preparing synthetic korupensamines and related C-8' to C-5 linked monomeric naphthylisoquinoline alkaloids, as well as non-korupensamines and other monomeric naphthylisoquinoline alkaloids which lack a C-8' to C-5 naphthylisoquinoline linkage, and derivatives thereof, all of which may be employed as synthetic precursors in the present invention for the preparation of dimeric naphthylisoquinoline alkaloids. Numerous other naturally occurring, monomeric naphthylisoquinoline alkaloids and semisynthetic derivatives thereof are also known (see, for example, Boyd et al., U.S. Patent 5,409,938; François et al., PCT Application PCT/US95/01717), and these likewise may be employed as synthetic precursors in the present invention.

A practical synthetic route of access to most of such naturally occurring compounds had not heretofore been available. However, as described above, such monomeric alkaloids can now be chemically synthesized and are likewise useful as synthetic precursors for the methods and compounds of the present invention in the preparation of dimeric naphthylisoquinoline alkaloids. Examples of such precursor monomers which may now be obtained as synthetic, partially synthetic or natural products, include but are not limited to the monomers

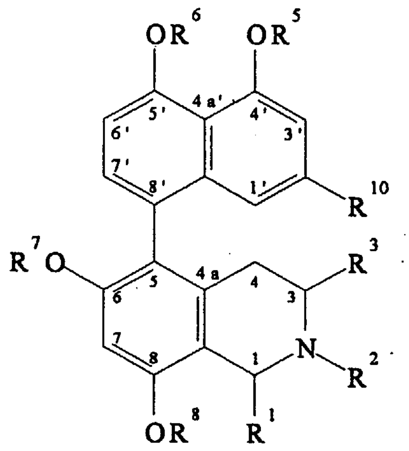

or

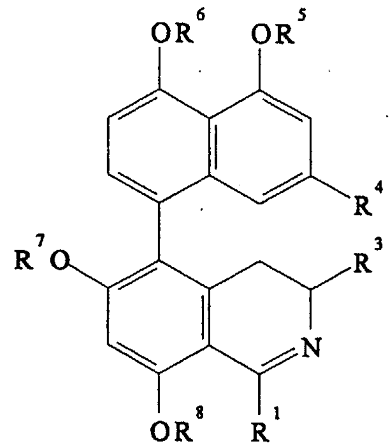

or

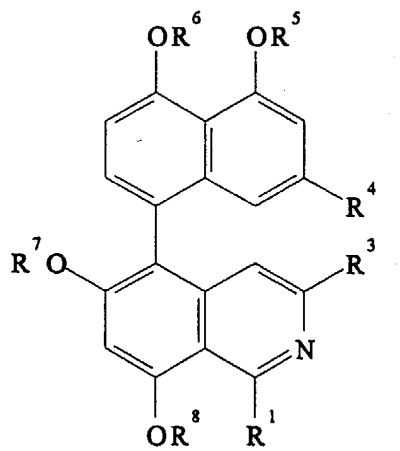

wherein $R^1$, $R^3$, $R^4$, and $R^{10}$ may be the same or different and each may be H or $C_1$-$C_6$ alkyl, $R^2$, $R^5$, $R^6$, $R^7$, and $R^8$ may be the same or different and each may be H, $C_1$-$C_6$ alkyl, $R^9CH_2$-, $R^9CO$-, or $R^9SO_2$-, $R^9$ may be H, $C_1$-$C_6$ alkyl, or aryl, as well as monomers having the chemical formulae depicted in **Figure 1** (see also Tables 1 and 2) and derivatives thereof.

Synthesis of Dimeric Naphthylisoquinoline Alkaloids

The present invention provides a method of preparing a compound which comprises:

(a) selecting first and second naphthylisoquinoline alkaloid monomers, which are either the same or different,
(b) optionally introducing protective group(s) at desired site(s) in the monomers,
(c) introducing activation group(s) at the desired coupling site(s) of the monomers if needed for coupling of the monomers,
(d) coupling the first and second monomers to form a dimeric naphthylisoquinoline alkaloid, and
(e) optionally removing the protective group(s) from the dimeric naphthylisoquinoline alkaloid.

The monomeric naphthylisoquinoline alkaloids which can be employed as precursors or building blocks for the synthesis of these dimeric naphthylisoquinoline compounds can include korupensamines and related C-8' to C-5 linked compounds, as well as non-korupensamines and other monomeric naphthylisoquinoline alkaloids and derivatives thereof. Such monomeric naphthylisoquinoline alkaloids can be known or novel and can be naturally occurring or prepared in accordance with the disclosure above.

When the first and second naphthylisoquinoline alkaloid monomers are the same, then their coupling results

in a homodimeric naphthylisoquinoline alkaloid. Alternatively, when the first and second monomers are different, then their coupling results in a heterodimeric naphthylisoquinoline alkaloid.

The protective group(s) can be removed from the dimeric naphthylisoquinoline alkaloid by any suitable means, preferably by using methanolic HCl. Also, following synthesis, the dimeric naphthylisoquinoline alkaloid can be purified by any suitable means, preferably by HPLC, and especially on an amino-bonded or other phase column.

The present invention further comprises preferably introducing an OH substituent, or modification of an existing substituent to give an OH substituent, at the naphthalene ring position adjacent to the coupling site prior to the introduction of the protective group(s).

Any suitable protective and activation group(s) can be employed in the context of the present invention. The protective group(s) is/are preferably introduced by consecutive *N*-formylation then *O*-acetylation at all sites except the OH located immediately adjacent to the desired coupling site in each monomer. However, as described herein, the method of synthesis can be carried out wherein the protective group(s) is/are not introduced at all such sites.

The activation group will generally be nucleophilic on one monomer and electrophilic on the other monomer. Thus, the activation group for the first monomer is preferably selected from the group consisting of trialkylstannyl and boronic acid derivatives (nucleophilic group), while the activation group for the second monomer is preferably selected from the group of halogens, particularly bromine, and *O*-triflate leaving groups (electrophilic group). Introduction of the activation group may be accomplished by any suitable means, for example, by metallation followed by conversion to an activation group, such as trialkylstannyl or a boronic acid derivative.

The aforementioned process steps can be carried out by any suitable means. Thus, for example and as described further herein, the coupling can be effected by several means, including but not limited to: electrochemically; by transition metal catalysis, especially using Pd; by enzyme catalysis, particularly wherein the enzyme is selected from the group consisting of laccase, peroxidase, tyrosinase, and mixtures thereof, or wherein the enzyme is from the *Ancistrocladus korupensis* plant, either obtained by recombinant means, or purified directly from this source; oxidatively, especially wherein an oxidant, particularly $Ag_2O$, is used to give the corresponding binaphthyl, biaryl, or binaphthylidendione centered quateraryl derivative; reductively, especially wherein the coupling is done by introducing a halogen (in particular, bromine) at the coupling site(s), and then performing an Ullmann reaction; and by a "redox-neutral process", particularly wherein the activation group for the first monomer is trialkylstannyl or a boronic acid derivative, and the activation group for the second monomer is a halogen (especially bromine) or an *O*-triflate leaving group, particularly when the coupling is done by transition metal catalysis.

In a preferred method of the present invention, the protective group(s) is/are introduced by consecutive *N*-formylation then *O*-acetylation at all sites except the at the site of the OH located immediately adjacent to the desired coupling site in each monomer, and the coupling is effected using oxidants, preferably $Ag_2O$, to give the corresponding binaphthyl, biaryl, or biarylidendione centered quateraryl derivative, which is followed by photochemical or chemical reduction, to give the corresponding protected binaphthyl or biaryl derivative.

One skilled in the art will readily appreciate that certain chemical modifications can be incorporated as desired into the aforementioned synthetic method and/or can be used to modify the end product thereof to obtain a useful new synthetic dimeric naphthylisoquinoline alkaloid derivative. Such modified properties may include greater therapeutic potency against a particular disease or disease-causing organism, a broader spectrum of therapeutic activity against diverse diseases or disease-causing organisms, enhanced oral bioavailability, less toxicity in a particular host mammal, more advantageous pharmacokinetics and/or tissue distribution in a given host mammal, and the like. For example, by applying one or more well known chemical reactions to a given dimeric naphthylisoquinoline alkaloid, prepared according to the aforementioned method, a useful new derivative may be obtained wherein one or more phenolic hydroxyl group(s) may instead be replaced by an ester, sulfonate ester, or ether group; one or more methyl ether group (s) may instead be replaced by a phenolic hydroxyl group; one or more phenolic hydroxyl group(s) may instead be replaced by an aromatic hydrogen substituent; a secondary amine site may instead be replaced by an amide, sulfonamide, tertiary amine, or alkyl quaternary ammonium salt or corresponding Hoffmann elimination product thereof; a tertiary amine site may instead be replaced be a secondary amine; and one or more aromatic hydrogen substituent(s) may instead be replaced by a halogen, nitro, amino; hydroxyl, thiol, acyl, $C_1$-$C_6$ alkyl, or cyano substituent, and $CH_3$ may be replaced by H. Alternatively, if a "modified" dimeric alkaloid is desired, such modifications may be contained within the monomeric alkaloids used to synthesize the dimer.

**Figure 8** schematically exemplifies some of the oxidative coupling approaches. Biosynthetic coupling may be achieved, for example, by catalysis using the whole *Ancistrocladus korupensis* plant (e.g., in the form of a homogenate) or subcellular fraction thereof, or other appropriate catalytic constituent(s), isolated or prepared therefrom. Such a constituent can be, for example, an enzyme which catalyzes the desired aforementioned coupling step.

An enzyme used for the aforementioned enzymatic coupling can be that isolated and purified directly from *Ancistrocladus korupensis* or, alternatively, a recombinant enzyme protein produced by well established genetic engineering techniques (see, e.g., see Nicholl, in An Introduction to Genetic Engineering, Cambridge University Press, Cambridge, 1994, pp. 1-5 & 127-130; Steinberg et al., in Recombinant DNA Technology Concepts and Biomedical

Applications, Prentice Hall, Englewood Cliffs, NJ, 1993, pp. 81-124 & 150-162; Sofer, Introduction to Genetic Engineering, Butterworth-Heinemann, Stoneham, MA, 1991, pp. 1-21 & 103-126; Old and Primrose in Principles of Gene Manipulation, Blackwell Scientific Publishers, London, 1992, pp. 1-13 & 108-221). For example, an *Ancistrocladus korupensis* gene or cDNA coding for such a coupling enzyme can be identified and subcloned. The gene or cDNA can then be delivered, using an appropriate expression vector, into an appropriate protein-synthesizing organism (e.g., E. *coli, S. cerevisiae,* insect cells, or mammalian cells) wherein the gene, under the control of an endogenous or exogenous promoter, can be appropriately transcribed and translated. Such expression vectors (including, but not limited to, phage, cosmid, viral, and plasmid vectors) are known to those skilled in the art, as are reagents and techniques appropriate for gene transfer (e.g., transfection, electroporation, transduction, microinjection transformation, etc.). Subsequently, the desired recombinantly produced protein can be isolated and purified using standard techniques known in the art for the purification of proteins (e.g., chromatography, centrifugation, differential solubility, isoelectric focusing, etc.), and used for the aforementioned enzymatic coupling reaction.

Alternatively, the desired native enzymatic protein for the aforementioned coupling reaction can be isolated, purified from *Ancistrocladus korupensis* by conventional (i.e., non-recombinant) methods, and sequenced by conventional techniques. The sequence can then be used to synthesize the corresponding DNA, which may then be subcloned into an appropriate expression vector, and delivered into a protein-producing cell for *en mass* recombinant production of the desired coupling enzyme.

In both cases, the DNA or cDNA encoding the coupling enzyme may code for either the entirety or a portion of the coupling protein. Where the DNA or cDNA does not comprise the entire coding sequence, the DNA or cDNA may be subcloned as part of a gene fusion. In a transcriptional gene fusion, the DNA or cDNA will contain its own control sequence directing appropriate production of protein (e.g., ribosome binding site, translation initiation codon, etc.), and the transcriptional control sequences (e.g., promoter elements and/or enhancers) will be provided by the vector. In a translational gene fusion, transcriptional control sequences as well as least some of the translational control sequences (i.e., the translation initiation codon) will be provided by the vector. In the case of a translational gene fusion, a chimeric protein will be produced.

Enzymes from sources other than *Ancistrocladus korupensis* can be used instead to catalyze the aforementioned coupling reaction. Examples of such enzymes include but are not limited to laccases (e.g., Benfield et al., Phytochemistry, 3, 79-88 (1964); Flaig et al., Planta Med., 9, 123-139 (1961)), peroxidases (e.g., Saunders, Peroxidase, Butterworth, London, 1964, pp. 1-52; Scott, Quart. Rev. (London), 19, 1-35 (1965)), and tyrosinases (e.g., Robb, Phytochemistry, 4, 731-740 (1965); Harel, Phytochemistry, 4, 783-790 (1965)). This approach is further illustrated, for example, in recent reviews (e.g., Holland, Organic Synthesis with Oxidative Enzymes, (VCH Weinheim, ed.), 1992, p. 341; Whiting, in Comprehensive Organic Synthesis (Trost and Fleming, eds.), Peragamon Press, Oxford, 1991, VII, p. 659).

One skilled in the art will also appreciate that the aforementioned essential coupling step can be also performed electrochemically. For example, a solution of N-formyl-6,8-0-bisacetyl korupensamine A (**11**) may be prepared and subjected to electrolysis, generally according to the procedure of Bobbitt et al. (J. Am. Chem. Soc., 93, 3551-3552 (1971)), to give the corresponding dimer. This is further illustrated schematically in **Figure 9**.

The skilled artisan will further appreciate that the aforementioned essential coupling step can be likewise performed chemically by reduction, for example by reductive dimerization. In this instance, as is typical of non-oxidative coupling procedures in general, an activation of the coupling position(s) is required. For example, a halogen such as bromine may be introduced at the desired coupling position, followed by an Ullmann reaction to join the desired monomeric "halves". Thus, the *N*-formyl-6,8-*O*-bisacetyl korupensamine A can be selectively monobrominated by reaction with $Br_2$, then subsequently protected at the site of the last remaining free OH group by acetylation under more rigorous reaction conditions. The reductive coupling step by the Ullmann reaction is performed in a suitable solvent by heating (e.g., to 200°C) with activated copper powder. Usual workup and cleavage of the protective groups provides michellamine A. This is further exemplified schematically in **Figure 10**. Alternatively, korupensamine A may be benzylated at *N*-2, *O*-6, and *O*-8 to give *N*-benzyl-6,8-di-*O*-benzylkorupensamine which is protected with the MOM-group at 0-5 and regioselectively halogenated by *ortho*-directed lithiation and treatment with an appropriate halogen source.

The aforementioned essential coupling step can also be efficiently accomplished by a "redox-neutral" coupling process. Thus, the *N*-benzyl-6,8-*O*-benzylkorupensamine A is metallated and converted into an appropriate activation group such as the corresponding trialkylstannyl or boronic acid derivative, which is then reacted with the corresponding bromo-substituted derivative with Pd-catalysis. Ultimate cleavage of the protective group (if desired) gives the corresponding dimeric target molecule. This is further exemplified in **Figure 11**. This "redox-neutral" procedure is highly efficient in the preparation of heterodimeric naphthylisoquinoline alkaloids, because the electrophilic and nucleophilic reaction partners may be derived from different monomeric naphthylisoquinoline alkaloids appropriately prepared by the corresponding protective group strategy.

The aforementioned essential coupling step can also be very efficiently and effectively accomplished chemically by an oxidative dimerization process. Consequently, the present invention is directed yet more specifically to

methods of preparing diverse medically useful dimeric naphthylisoquinoline alkaloids, particularly by employing an oxidative coupling step to join the desired monomeric halves.

Dimeric Naphthylisoquinoline Alkaloids Containing a C-8' to C-5 Naphthalene/Isoquinoline Linkage Within Both Molecular Halves and Derivatives Thereof

The michellamines are characterized by the presence of no less than 6 free phenolic hydroxy groups and 2 secondary amino functions and, stereochemically, by the existence of 4 stereocenters and 3 axes, one of which is configuratively unstable, and the other two of which are stereogenic due to restricted rotation. As illustrated in **Figure 18,** michellamines A and C in particular are $C_2$-symmetric homodimers consisting of two constitutionally and stereochemically identical halves, whereas michellamine B is a heterodimer consisting of two atropodiastereomeric parts.

The present invention provides a novel method of chemical synthesis whereby one can build up the dimeric naphthylisoquinoline alkaloid framework through coupling of first and second naphthylisoquinoline monomers which represent the corresponding monomeric "halves" of the dimer. The present invention encompasses the aforementioned method wherein the first and second monomers are the same or different, especially wherein the monomers possess a C-8' to C-5 naphthalene/isoquinoline linkage, and preferably wherein the monomers are compounds of the formula

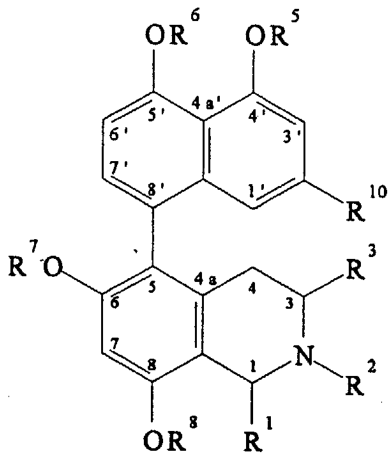

or

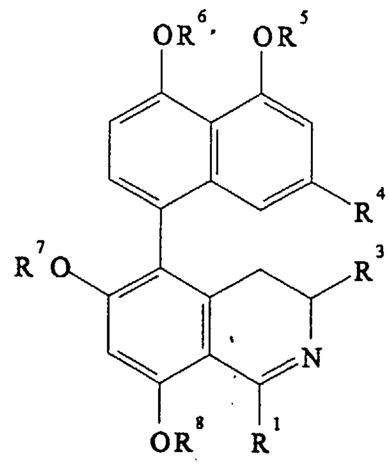

or

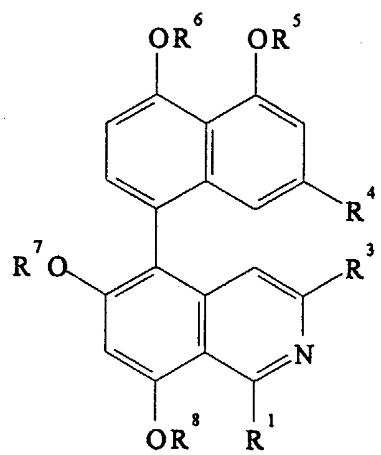

wherein $R^1$, $R^3$, $R^4$, and $R^{10}$ may be the same or different and each may be H or $C_1$-$C_6$ alkyl, $R^2$, $R^5$, $R^6$, $R^7$, and $R^8$ may be the same or different and each may be H, $C_1$-$C_6$ alkyl, $R^9CH_2$-, $R^9CO$-, or $R^9SO_2$-, $R^9$ may be H, $C_1$-$C_6$ alkyl or aryl, and one or more of the ring positions 1, 3, 4, 1', 2', 3', 4', 5', 6', 7', 6, 7, and 8 may be substituted with halo, nitro, amino, hydroxyl, thiol, acyl, $C_1$-$C_6$ alkyl, or cyano, one or more phenolic hydroxyl group(s) may instead be an

ester, sulfonate ester, or ether group, one or more methyl ether group(s) may instead be a phenolic hydroxyl group, one or more phenolic hydroxyl group(s) may instead be an aromatic hydrogen substituent, one or more secondary amine site(s) may instead be an amide, sulfonamide, tertiary amine, alkyl quaternary ammonium salt or corresponding Hoffmann elimination product thereof, and one or more tertiary amine site(s) may instead be a secondary amine.

The synthetic scheme for michellamine A provides a specific illustration of the method of synthesis of a homodimeric alkaloid containing a C-8' to C-5 naphthalene/isoquinoline linkage, and is set forth in **Figure 12.** From the structure of the given precursor **10a** (i.e., korupensamine A), with its phenolic oxygen functions and the secondary amino group, undesired byproducts might be expected for the oxidation step. Also, such polar compounds are more difficult to handle. Consequently, it is necessary to guarantee selectivity for the coupling reaction already on the level of a specific protection of all these functionalities except for 5'-OH, i.e., the oxygen function next to the required coupling site. Thus, consecutive *N*-formylation with pivalic formic anhydride (Vlietstra et al., J.R. Neth. Chem. Soc., 101, 460-462 (1982)) and subsequent treatment with acetyl chloride clearly allows differentiation of the "free" hydroxy functions at C-6 and C-8 from the chelated one at C-5', specifically giving the partially protected monophenolic derivative **11** in most satisfactory (e.g., 90%) yields.

With the key monomeric precursor **11** thus in hand, the crucial dimerization step can be achieved with optimum yields (e.g., 90-95%) when using conditions elaborated in Laatsch et al., Liebigs Ann. Chem., 1321-1347 (1980), for the dimerization of related naphthol precursors. Thus, treatment of **11** with $Ag_2O$ in $CHCl_3$ in the presence of 0.2% triethylamine leads directly to the formation of corresponding binaphthylidendione **12**, without the necessity of stopping the reaction on the level of an intermediate binaphthol. Still, the easy detection of the violet-colored diquinone **12** and its satisfying stability makes this compound a convenient intermediate that can be fully characterized. Subsequent cautious reduction of **12,** e.g., with sodium borohydride or with methanol/hv, gives the corresponding substituted binaphthol, with the central biaryl axis then in the correct position and oxidation level. No complications by the formation of atropodiastereomeric products needs to be taken into consideration, due to the low isomerization barrier at the central axis. Subsequent cleavage of all the 6 protective groups in the multifold derivatized michellamine A is then performed in a single step, by treatment with methanolic HCl, to give the pure michellamine A (**13**) in good yields (e.g., 60% from **12**) with cleanup as necessary or desired by HPLC on an amino-bonded phase column. The synthetic product can be shown to be identical to the corresponding natural product by a comparison of its physicochemical, spectral, and biological properties to an authentic sample of the natural product (Manfredi et al., 1991, supra; Boyd et al., 1994, supra). Additional details can be found in Example 3.

It is also noteworthy that the oxidative phenolic coupling reaction can be, in certain favorable instances, successfully accomplished (albeit generally with somewhat less efficiency) using less fully protected or even unprotected monomers. For example, *N*-formyl-korupensamine A (i.e., not having further *O*-protective groups) can be coupled and deprotected in accordance with the immediately aforementioned procedure to give michellamine A. However, this synthetic route typically results in much poorer yields than in the aforementioned procedure which includes the use of *O*-protective groups. Likewise, it can be anticipated that coupling can be accomplished with completely unprotected monomers (e.g., lacking also the *N*-protective functionality), however, with still expected worse yields than when any protection/deprotection strategy is employed.

Certain naturally occurring michellamine compounds, and derivatives prepared directly therefrom, are described in PCT Applications WO 92/18125 and WO 94/24108. As part of the present invention, those michellamine compounds and derivatives can now be obtained by chemical synthesis, which may be partially or entirely independent of the aforementioned natural products, by use of the aforementioned method and by employing synthetic C-8' to C-5 linked korupensamines or derivatives prepared according to the method disclosed above. Likewise, unprecedented new synthetic dimers, in which both of the monomeric halves retain a C-8' to C-5 naphthalene/isoquinoline linkage, yet which otherwise differ (e.g., in the chirality of stereocenter(s) or biaryl axis (axes), in the nature of the isoquinoline group(s), or in the position of coupling points of the monomeric halves) from the michellamines known to occur in nature, can be prepared.

Some specific examples of variations in structure of such dimeric naphthylisoquinoline alkaloids which can be prepared according to the present invention are provided in **Figure 13.** Structure **A** is michellamine A; structure **B** exemplifies a corresponding dimer wherein one of the korupensamine halves is instead replaced by a monomer which retains a C-8' to C-5 naphthalene/isoquinoline linkage and has different stereochemistry at C-1 and/or C-3; structure **C** exemplifies a corresponding compound having one or both tetrahydroquinolines replaced by a dihydroisoquinoline; structure **D** exemplifies a corresponding compound having one or both tetrahydroisoquinoline(s) or dihydroisoquinoline (s) replaced by a fully aromatic isoquinoline. In addition to, or instead of, variations such as exemplified by these structures, there may be other variations, such as different configurations (e.g., *M* or *P*) about the axis of coupling, different coupling positions, and/or different substituents on the naphthalene and/or isoquinoline portion(s) of the molecule.

Accordingly, the present invention provides new compounds, particularly dimeric michellamines and other dimeric naphthylisoquinoline compounds and derivatives thereof which possess a C-8' to C-5 naphthalene/isoquinoline

linkage within both molecular halves. More specifically, the present invention provides a dimeric naphthylisoquinoline compound, or derivative thereof, wherein each monomeric half is the same or different and is comprised of a monomeric naphthylisoquinoline alkaloid which possesses a C-8' to C-5 naphthalene/isoquinoline linkage.

Thus, the present invention provides a dimeric naphthylisoquinoline alkaloid comprised of coupled first and second naphthylisoquinoline monomers which are the same or different, wherein said first and second monomers are compounds of the formula

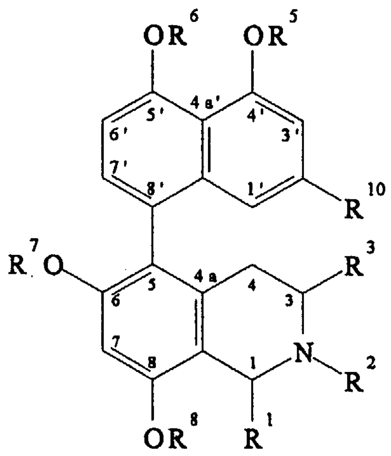

or

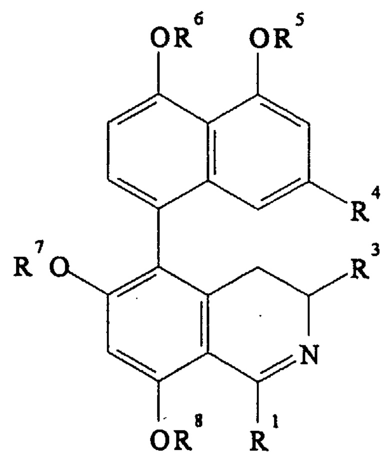

or

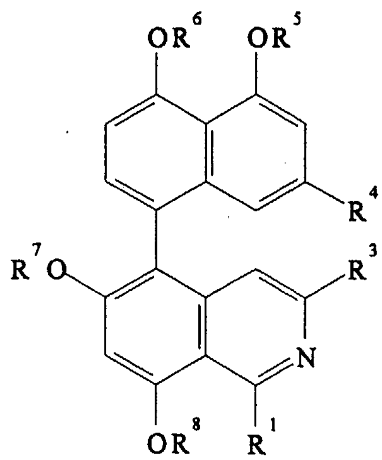

wherein $R^1$, $R^3$, $R^4$, and $R^{10}$ may be the same or different and each may be H or $C_1$-$C_6$ alkyl, $R^2$, $R^5$, $R^6$, $R^7$, and $R^8$ may be the same or different and each may be H, $C_1$-$C_6$ alkyl, $R^9CH_2$-, $R^9CO$-, or $R^9SO_2$-, $R^9$ may be H, $C_1$-$C_6$ alkyl or aryl, and one or more of the ring positions 1, 3, 4, 1' , 2' , 3' , 4' , 5' , 6' , 7' , 6, 7, and 8 may be substituted with halo, nitro, amino, hydroxyl, thiol, acyl, $C_1$-$C_6$ alkyl, or cyano, one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, one or more secondary amine site(s) may instead be an amide, sulfonamide, tertiary amine, alkyl quaternary ammonium salt or corresponding Hoffmann elimination product thereof, and one or more tertiary amine site(s) may instead be a secondary amine, with the proviso that $R^{10}$ is not methyl in both first and second monomers when $R^1$ and $R^3$ are methyl in both first and second monomers.

Dimeric Naphthylisoquinoline Alkaloids Lacking a C-8' to C-5 Naphthalene/Isoquinoline Linkage Within One or Both of the Monomeric Halves

The aforementioned method of coupling korupensamines and related naphthylisoquinoline alkaloids to provide dimers (e.g., michellamines) can be further applied, extended, and adapted as appropriate to provide yet other heretofore unknown dimeric naphthylisoquinoline alkaloids. For example, one or both of the monomeric halves comprising such a dimer may contain a non-korupensamine which lacks a C-8' to C-5 naphthalene/isoquinoline linkage that typifies the korupensamines and "korupensamine-like" monomers. Thus, for instance, one can select a monomeric compound of Table 1 or 2 to couple with the same or a different monomeric compound of Table 1 or 2, or with a korupensamine or related C-8' to C-5 linked monomer, to provide a new homodimeric or heterodimeric naphthylisoquinoline alkaloid. The choice of monomers for coupling into dimers is exemplified by, but is not limited to, the korupensamines and derivatives thereof and the compounds and derivatives thereof of Tables 1 and 2. Still yet other suitable precursor monomers may be found in nature. In this respect, other diverse monomers having different linkages and/or chirality can be prepared by the methods of synthesis disclosed above, and such monomers can serve likewise as precursors for coupling into novel dimers.

Thus, the present invention encompasses a method of preparing a homodimeric or heterodimeric naphthylisoquinoline alkaloid which lacks a C-8' to C-5 naphthalene/isoquinoline linkage within one or both of the monomeric halves. The present inventive method of preparing a dimeric naphthylisoquinoline alkaloid through coupling of first and second monomers which form the corresponding monomeric halves of the dimeric naphthylisoquinoline alkaloid may also be employed wherein the first and second monomers are the same or different, and one or both of the first and second monomers lack a C-8' to C-5 naphthalene/isoquinoline linkage.

Suitable such monomers which lack a C-8' to C-5 naphthalene/isoquinoline linkage include the group consisting of dioncophylline B, dioncopeltine A, dioncophylline A, dioncophylline C, dioncolactone A, *N*-methyl-dioncophylline A, ancistrobrevine D, ancistrocladine, 5'-*O*-demethyl-8-*O*-methyl-7-*epi*-dioncophylline A, 5'-*O*-demethyl-7-*epi*-dioncophylline A, dioncophylleine A, (±)-dioncophyllacine A, hamatine, ancistrobrevine A, 6-*O*-demethyl-ancistrobrevine A, ancistrobarterine A, 7-*epi*-dioncophylline A, *N*-formyl ancistrocladine, *N*-methyl-ancistrocladine, 6-deoxy-*N*-methyl-ancistrocladine, *N*-formyl-*O*,*O*-dimethyl-dioncophylline C, *N*-formyl-dioncophylline C, *N*-formyl-8-*O*-benzyl-dioncophylline C, *N*-formyl-8-*O*-methyl-dioncophylline C, *N*-formyl-8-*O*-pivaloyl-dioncophylline C, *N*-formyl-8-*O*-acetyl-dioncophylline C, *N*-formyl-8-*O*-benzoyl-dioncophylline C, and 8-*O*-methyl-dioncophylline C, wherein the configurations at C-1 and C-3 may instead be the same or different and each may be *R* or *S*, the coupling points comprising the naphthalene/isoquinoline axis may be different, the configuration about the axis may be different, one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, and one or more secondary amine site(s) may instead be an amide, sulfonamide, tertiary amine, alkyl quaternary ammonium salt or corresponding Hoffmann elimination product thereof, one or more tertiary amine site(s) may instead be a secondary amine, one or more aromatic hydrogen substituent(s) may instead be a halogen, nitro, amino, hydroxyl, thiol, acyl, $C_1$-$C_6$ alkyl, or cyano substituent, $CH_3$ may instead be H, and the tetrahydroisoquinoline may instead be a dihydroisoquinoline or a fully aromatic isoquinoline.

When only one of the monomers lacks a C-8' to C-5 naphthalene/isoquinoline linkage and the other monomer possesses a C-8' to C-5 naphthalene/isoquinoline linkage, the monomer possessing a C-8' to C-5 naphthalene/isoquinoline linkage can be a compound of the formula

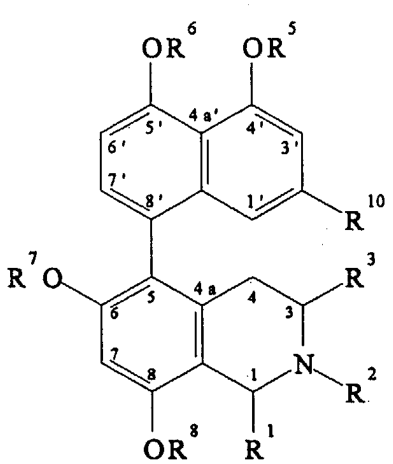

or

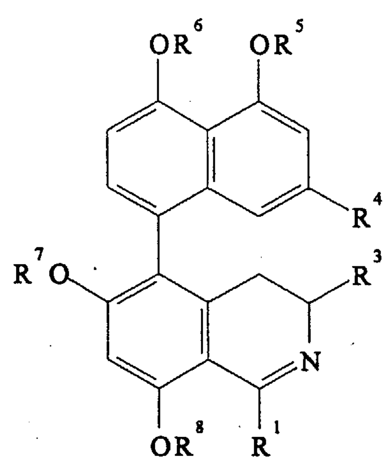

or

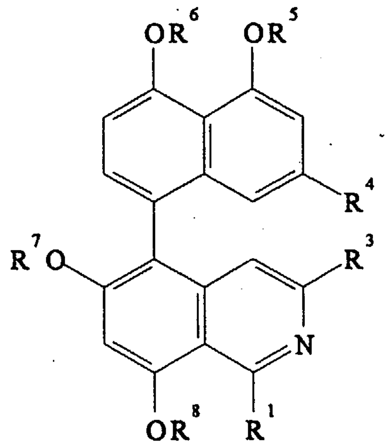

wherein $R^1$, $R^3$, $R^4$ and $R^{10}$ may be the same or different and each may be H or $C_1$-$C_6$ alkyl, $R^2$, $R^5$, $R^6$, $R^7$, and $R^8$ may be the same or different and each may be H, $C_1$-$C_6$ alkyl, $R^9CH_2$-, $R^9CO$-, or $R^9SO_2$-, $R^9$ may be H, $C_1$-$C_6$ alkyl or aryl, and one or more of the ring positions 1, 3, 4, 1' , 2' , 3' , 4' , 5' , 6' , 7' , 6, 7, and 8 may be substituted with halo, nitro, amino, hydroxyl, thiol, acyl, $C_1$-$C_6$ alkyl, or cyano, one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, one or more secondary amine site(s) may instead be an amide, sulfonamide, tertiary amine, alkyl quaternary ammonium salt or corresponding Hoffmann elimination product thereof, and one or more tertiary amine site(s) may instead be a secondary amine.

As a yet more specific example, the aforementioned method can be applied to the synthesis of a heretofore unknown dimeric naphthylisoquinoline alkaloid **17** comprised of two dioncopeltine A "halves", as illustrated in **Figure 14**. As before in the case of korupensamine A, the naturally occurring alkaloid dioncopeltine A (**14**), as isolated from *Triphyophyllum peltatum* (Bringmann et al., Phytochemistry, 30, 1691-1696 (1991)), can subsequently be protected by N-formylation with pivalic formic anhydride and subsequent O-acetylation with AcCl, thus protecting all the nucleophilic functionalities except for the OH group at C-5' to give **15.** Subsequent cautious oxidation with $Ag_2O$ gives the dimeric compound **16,** which may be deprotected as in the aforementioned synthesis of michellamine A to give the novel dimer 17. Further details are provided in Example 4.

**Figure 15** illustrates some other selected examples of variations in the dimeric naphthylisoquinoline alkaloids which can be obtained according to the methods of the present invention. Structure **A** is a heterodimer comprised of one korupensamine monomeric half (which possesses a C-8' to C-5 naphthalene/isoquinoline linkage) coupled to a dioncophylline C monomeric half (which lacks a C-8' to C-5 naphthalene/isoquinoline linkage); structure **B** exemplifies a corresponding dimer wherein one or both of the monomeric halves instead has (have) a different configuration at C-1 and/or C-3; structure **C** exemplifies a corresponding compound having one or both tetrahydroisoquinolines replaced by a dihydroisoquinoline; structure **D** exemplifies a corresponding compound having one or both tetrahydroisoquinoline (s) or dihydroisoquinoline(s) replaced by a fully aromatic isoquinoline. In addition to, or instead of, variations such as exemplified by these structures, there may be other variations, such as different configurations (e.g., *M* or *P*) about the axis of coupling, different coupling positions, and/or different substituents on the naphthalene and/or isoquinoline portion(s) of the molecule.

In view of the present disclosures, one skilled in the art will appreciate that in certain instances with certain compounds there will be the opportunity of directly coupling, immediately following *N*-formylation, of selected naphthylisoquinoline monomers which do not have the free OH group on the naphthalene portion. This may be, for example, exploited to obtain dimers that are coupled at the isocyclic ring of the isoquinoline, giving rise to a quaterary in which the naphthalene (N) and isoquinoline (IQ) parts are linked together with the connectivity "N-IQ-IQ-N", i.e., in a manner different than the usual (e.g., michellamine-type) naphthalene-coupled array ("IQ-N-N-IQ"). This is further exemplified in **Figures 16** and **17**, which schematically illustrate methods leading to isoquinoline-coupled or, optionally, to naphthalene-coupled dimers of dioncophylline A and ancistrocladine, respectively.

Accordingly, the present invention provides new dimeric naphthylisoquinoline alkaloids and derivatives thereof. In particular, the present invention provides a dimeric naphthylisoquinoline alkaloid comprised of coupLed first and second naphthylisoquinoline monomers which are the same or different and wherein one or both of the monomers lacks a C-8' to C-5 naphthalene/isoquinoline linkage, particularly wherein the monomers which lack a C-8' to C-5 naphthalene/isoquinoline linkage are selected from the group consisting of dioncophylline B, dioncopeltine A, dioncophylline A, dioncophylline C, dioncolactone A, *N*-methyl-dioncophylline A, ancistrobrevine D, ancistrocladine, 5'-*O*-demethyl-8-*O*-methyl-7-*epi*-dioncophylline A, 5'-*O*-demethyl-7-*epi*-dioncophylline A, dioncophylleine A, (±)-dioncophyllacine A, hamatine, ancistrobrevine A, 6-*O*-demethyl-ancistrobrevine A, ancistrobarterine A, 7-*epi*-dioncophylline A, *N*-formyl ancistrocladine, *N*-methyl-ancistrocladine, 6-deoxy-*N*-methyl-ancistrocladine, *N*-formyl-*O*,*O*-dimethyl-dioncophylline C, *N*-formyl-dioncophylline C, *N*-formyl-8-*O*-benzyl-dioncophylline C, *N*-formyl-8-*O*-methyl-dioncophylline C, *N*-formyl-8-*O*-pivaloyl-dioncophylline C, *N*-formyl-8-*O*-acetyl-dioncophylline C, *N*-formyl-8-*O*-benzoyl-dioncophylline C, and 8-*O*-methyl-dioncophylline C, wherein the configurations at C-1 and C-3 may instead be the same or different and each may be *R* or *S*, the coupling points comprising the naphthalene/isoquinoline axis may be different, the configuration about the axis may be different, one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, and one or more secondary amine site(s) may instead be an amide, sulfonamide, tertiary amine, alkyl quaternary ammonium salt or corresponding Hoffmann elimination product thereof, one or more tertiary amine site(s) may instead be a secondary amine, one or more aromatic hydrogen substituent(s) may instead be a halogen, nitro, amino, hydroxyl, thiol, acyl, $C_1$-$C_6$ alkyl, or cyano substituent, $CH_3$ may instead be H, and the tetrahydroisoquinoline may instead be a dihydroisoquinoline or a fully aromatic isoquinoline, while the monomer, if utilized, which possesses a C-8' to C-5 naphthalene/isoquinoline linkage is a compound of the formula

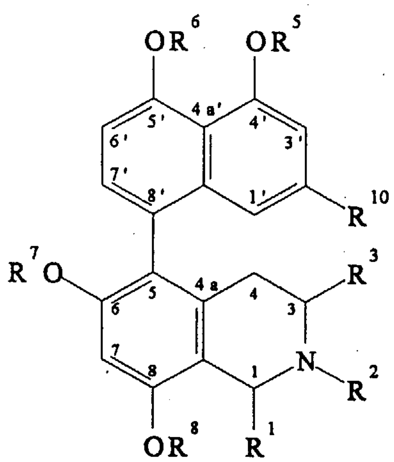

or

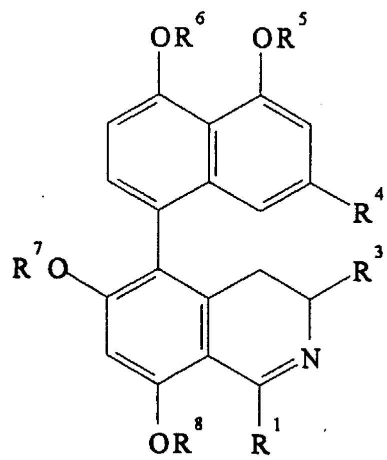

or

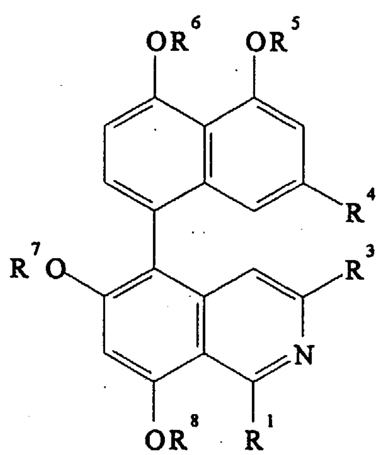

wherein $R^1$, $R^3$, $R^4$ and $R^{10}$ may be the same or different and each may be H or $C_1$-$C_6$ alkyl, $R^2$, $R^5$, $R^6$, $R^7$, and $R^8$ may.be the same or different and each may be H, $C_1$-$C_6$ alkyl, $R^9CH_2$-, $R^9CO$-, or $R^9SO_2$-, $R^9$ may be H, $C_1$-$C_6$ alkyl or aryl, and one or more of the ring positions 1, 3, 4, 1', 2', 3', 4', 5', 6', 7', 6, 7, and 8 may be substituted with halo, nitro, amino, hydroxyl, thiol, acyl, $C_1$-$C_6$ alkyl, or cyano, one or more phenolic hydroxyl group(s) may instead be an ester, sulfonate ester, or ether group, one or more methyl ether group(s) may instead be a phenolic hydroxyl group, one or more phenolic hydroxyl group(s) may instead be an aromatic hydrogen substituent, one or more secondary amine site(s) may instead be an amide, sulfonamide, tertiary amine, alkyl quaternary ammonium salt or corresponding

Hoffmann elimination product thereof, and one or more tertiary amine site(s) may instead be a secondary amine.

Examples

The following examples further illustrate the present invention, but, of course, should not be construed as in any way limiting its scope.

EXAMPLE 1: Synthesis of Monomeric Alkaloids

This example provides a description of the method of preparing monomeric alkaloids. In particular, this example provides a specific description of the preparation of korupensamine A (**Figure 4; 10a**) and korupensamine B (**Figure 4; 10b**), both of which contain the characteristic C-8' to C-5 naphthalene/tetrahydro-isoquinoline linkage. The reaction for other monomeric alkaloids is quite similar.

The first stage of the method corresponding to preparation of the naphthalene building block is illustrated in **Figure 2**; the reaction conditions and yields are summarized in the corresponding legend. The synthesis starts with **1** (pale yellow oil), prepared by *O*-isopropylation (Pr-Br, acetone, 88%) and subsequent bromination ($Br_2 \cdot NaOAc$, $CH_2Cl_2$, 62%) of 3-hydroxybenzaldehyde. For the annulation of the second ring, the introduction of the missing $C_4$-unit by Wittig reaction proved to give higher yields than the Stobbe approach frequently used in the literature (Handford and Whalley, J. Chem. Soc., 3896-3897 (1963)). From the known building block **2** (Owton et al., Syn. Commun., 23, 2119-2125 (1993), and literature cited therein) was obtained, after selective cleavage of the tert-butylester group with $CF_3CO_2H$, the $\alpha,\beta$-unsaturated monoester **3** (m.p. 86°C), which then was cyclized, *O*-deacetylated, and *O*-methylated. Transformation of the ester functionality into the required methyl substituent was brought about by LAH-reduction and subsequent deoxygenation of the primary alcohol group by hydroxy/halogen exchange (Bringmann et al., Angew. Chem. Int. Ed. Eng., 25, 913-915 (1986); Bringmann and Schneider, Synthesis, 139-141 (1983)) followed by further reduction to give **4** (m.p. 78°C).

**2-Bromo-5-isopropoxy-benzaldehyde (1).** To a suspension of 50.0 g (110 mmol) 3-hydroxybenzaldehyde and 100 g (725 mmol) dry $K_2CO_3$ in 100 ml dry DMF, 61.5 g (500 mmol) isopropylbromide was added at room temperature. The reaction mixture was heated for 17 h at 90°C. The solvent was removed under reduced pressure and the residue was partitioned between 2N NaOH and toluene. The collected organic layers were dried ($MgSO_4$), filtered and the solvent removed under pressure. The residue was distilled *in vacuo* to give 58.8 g (88%) colorless liquid. To a solution of 57.0 g (347 mmol) 3-isopropoxybenzaldehyde in 200 ml dichloromethane 30.5 g (372 mmol) NaOAc was added. After that a solution of 55.5 g (347 mmol) bromine in 50 ml dichloromethane was added dropwise and the reaction mixture was stirred overnight. The reaction mixture was extracted with saturated aqueous $Na_2SO_3$ and the combined organic phases were dried ($MgSO_4$), filtered and concentrated under reduced pressure. The residue was distilled *in vacuo* to give **1** (52.1 g, 62%) as a yellow oil.

**2-*tert*-Butoxycarbonyl-1-ethoxycarbonylethyl-idendiethoxyphosphorane (2).** To a suspension of 5.50 g (231 mmol) NaH in 300 ml dry THF, a solution of 49.3 g (220 mmol) triethylphosphonoacetate in 150 ml dry THF under Ar at 0°C was added dropwise. The reaction mixture was stirred and allowed to warm to room temperature overnight. 45.0 g (231 mmol) tert-butyl-bromoacetate was added dropwise at 0°C over 30 min and the reaction mixture was allowed to warm to room temperature. After 28 h the reaction mixture was concentrated under reduced pressure and partitioned between water and ethyl acetate. The organic phase was dried ($MgSO_4$), filtered and concentrated under reduced pressure. The crude product was distilled *in vacuo* to give 54.0 g (73%) 2 as a viscous oil.

**(*E*)-3-Carboethoxy-4-(2'-bromo-5'-isopropylphenyl)-3-butenic Acid (3).** To a suspension of 1.85 g (77.1 mmol) NaH in 130 ml dry THF, 25.4 g (75.1 mmol) *tert*-butoxycarbonyl-1-ethoxycarbonylethylidendiethoxy-phosphorane (2) was added dropwise at 0°C, The reaction mixture was stirred for 6 h at 0°C under Ar. This solution was added under Ar dropwise at 0°C to 15.5 g (63.5 mmol) 2-bromo-5-isopropoxy-benzaldehyde (1), dissolved in 80 ml dry THF. The cooling bath was removed and the reaction mixture was stirred overnight at room temperature. 20 ml water was added and the solvent was removed under reduced pressure. The residue was partitioned between water and dichloromethane. The organic layers were dried ($MgSO_4$), filtered and the solvent was removed under reduced pressure to afford 16.5 g (61%) (*E*)-3-carboethoxy-4-(2'-bromo-5-isopropylphenyl)-3-butenic acid-*tert*-butyl ester as a yellow oil.

15.0g (35.1 mmol) of the crude (*E*)-3-carboethoxy-4-(2'-5-isopropylphenyl)-3-butenic acid-*tert*-butyl ester was dissolved in 70 ml 90% aqueous trifluoroacetic acid and stirred for 3 h. Removal of trifluoroacetic acid *in vacuo* afforded 12.8 g (99%) **3** as a pale yellow solid (m.p. 86°C).

**1-Bromo-4-isopropoxy-5-methoxy-7-methylnaphthalene (4)**. 12.9 g (34.8 mmol) (*E*)-3-carboethoxy-4-(2'-bromo-5'-isopropylphenyl)-3-butenic acid (**3**) were dissolved in 340 acetic anhydride and heated for 6 h in reflux. After cooling to room temperature, a solution of 200 g ice, 200 g water and 90 g $K_2CO_3$ were added. After extraction with dichloromethane, the combined organic layers were dried ($MgSO_4$), filtered and concentrated under reduced pressure. The dried residue was dissolved in a solution of 8.40 g (389 mmol) sodium in 300 ml dry ethanol and stirred for 4 h at

room temperature under Ar. The solvent was removed *in vacuo* and the residue was partitioned between 100 ml dichloromethane and 30 ml 2N HCl-solution. The organic phases were collected, dried ($MgSO_4$) and concentrated under reduced pressure. The residue was recrystallized from isopropanol to give 11.7 g (95%) ethyl-8-bromo-4-hydroxy-5-isopropoxy-2-naphthoate as yellow crystals (m.p. 106°C).

To a solution of 4.92 g (13.9 mmol) ethyl-8-bromo-4-hydroxy-5-isopropoxy-2-naphthoate in 150 ml acetone 4.80 g (34.8 mmol) $K_2CO_3$ and dropwise 4.90 g (38.7 mmol) dimethylsulfate at room temperature were added. Subsequently the reaction mixture was refluxed for 10 h and after that 60 ml of concentrated ammonia was added. Solvent was removed under vacuum and the residue was partitioned between water and dichloromethane. The combined organic phases were washed three times with 2N NaOH solution and two times with water. The organic phase was dried ($MgSO_4$) and concentrated under vacuum. The residue was recrystallized from diethylether to give 5.00 g (99%) ethyl-8-bromo-5-isopropoxy-4-methoxy-2-naphthoate as colorless needles (m.p. 62°C).

A solution of 4.80 g (13.1 mmol) ethyl-8-bromo-5-isopropoxy-4-methoxy-2-naphthoate in 100 ml dry THF was cooled to 0°C under Ar. A suspension of 498 mg (13.1 mmol) $LiAlH_4$ in 60 ml dry THF was cooled to 0°C and added to the ester over a period of 45 min. After stirring for 30 min at 0°C, 10 ml water and 10 ml 2N HCl were added carefully. The THF was concentrated *in vacuo* and the residue was extracted with dichloromethane, dried ($MgSO_4$) and concentrated *in vacuo*. Column chromatography (dichloromethane/acetone, 95:5) afforded 4.00 g (95%) 1-bromo-7-hydroxymethyl-4-isopropoxy-5-methoxy-naphthalene as colorless needles (m.p. 120-121°C).

To a solution of 4.00 g (12.3 mmol) 1-bromo-7-hydroxymethyl-4-isopropoxy-5-methoxy-naphthalene in 40 ml dry dichloromethane, 3.24 g (12.3 mmol) triphenyl-phosphane and 4.00 g (12.3 mmol) 1,2-dibromotetrachloroethane were added under Ar. After stirring for 30 min at room temperature, the solvent was removed and subsequently the residue was filtered over a short silica gel column (petroleum/ethyl acetate, 4:1) to give 5.00 g (95%) 1-bromo-7-bromomethyl-4-isopropoxy-5-methoxy-naphthalene as a colorless solid (m.p. 126°C).

To a 0°C cold solution of 7.60 g (19.6 mmol) 1-bromo-7-bromomethyl-4-isopropoxy-5-methoxy-naphthalene in 150 ml dry dichloromethane, 20.0 ml (20.0 mmol) of a 1M L-selectride-solution was added under Ar. After 2 h, further 2.50 ml (2.50 mmol) 1M *L*-selectride-solution was added. After that the solvent was removed *in vacuo* and the residue was filtered over a short silica gel column (petroleum/acetone, 30:1). After removal of the solvent *in vacuo*, the residue was recrystallized from acetone to give 5.90 g (98%) of **4** as colorless needles (m.p. 78°C).

The second stage of the synthesis corresponding to preparation of the isoquinoline building block is shown in **Figure 3.** The reaction conditions and yields are summarized in the corresponding legend. **7:** A solution of the isoquinoline **6** (1.50 g, 5.07 mmol), benzyl bromide (0.66 ml, 5.55 mmol) and benzyl tri-*n*-butyl ammonium chloride (160 mg, 0.51 mmol) in dichloromethane (20 ml) and 2 N NaOH (8 ml) was stirred vigorously for 5 h at 20°C. The layers were separated, the aqueous layer was extracted with dichloromethane and dried over sodium sulfate. Chromatography on deactivated (5% $NH_3$) silica gel with petroleum ether/*tert*-butyl methyl ether/ethyl acetate (100:0:0 → 90:6:3) as eluent afforded the crude benzylated isoquinoline (1.58 g) as a yellow oil. (characterized as HBr salt; m.p. 232-234°C, $[\alpha]_D^{20}$ = +23° (c = 1.1 in ethanol)). To a suspension of sodium hydride (480 mg, 20.0 mmol) in dimethyl formamide (20 ml) 1-methyl ethanethiol (1.86 ml, 20.0 mmol) was added. The mixture was stirred for 0.5 h at 40°C and then added to the crude benzylated isoquinoline. The resulting solution was heated to 150°C for 5 h. At 20°C, water (50 ml) and saturated ammonium chloride solution (20 ml) were added. After stirring for 10 min, the phases were separated, and the aqueous layer was extracted with dichloromethane. Chromatography on deactivated (5% $NH_3$) silica gel with petroleum ether/*tert*-butyl methyl ether/ethyl acetate (100:0:0 → 70:20:10) as eluent yielded the starting material **6** (539 mg, 1.81 mmol) and the 6-hydroxy isoquinoline (688 mg, 1.84 mmol, 36%, 56% based on recovered starting material, characterized as HBr salt; m.p. 239°C, $[\alpha]_D^{20}$ = +24° (c = 0.53 in ethanol)). A solution of the 6-hydroxyisoquinoline (137 mg, 0.37 mmol), benzyl bromide (48 μl, 0.40 mmol) and benzyl tri-n-butyl ammonium chloride (11 mg, 37 μmol) in dichloromethane (6 ml) and 2 N NaOH (3 ml) was stirred vigorously for 2 h at 20°C. The aqueous layer was extracted with dichloromethane. Chromatography on deactivated (5% $NH_3$) silica gel with petroleum ether/tert-butyl methyl ether/ethyl acetate (100:0:0 → 85:10:5) as eluent afforded the tribenzylisoquinoline (155 mg, 0.334 mmol, 91%) as a pale yellow oil. A solution of this oil (36 mg, 78 μmol) and bromine 7 μl, 130 μmol) in dimethyl formamide (1 ml) was stirred for 3 d at 20°C. The solution was diluted with dichloromethane, and bisulfite solution was added. Extraction with dichloromethane and chromatography on deactivated (5% $NH_3$) silica gel with petroleum ether/tert-butyl methyl ether/ethyl acetate (100:0:0 → 85:10:5) as eluent yielded the isoquinoline 7 (42 mg, 73 μmol, 94%) as a yellow oil. The resulting product **7** (characterized as its HCl salt: mp 130°C, $[\alpha]_D$+36°, c = 0.49 in acetone), provided the necessary precursor for the heterocyclic part of the target molecules.

The third stage of the synthesis corresponding to convergent construction of the alkaloids is shown in **Figure 3.** The reaction conditions and yields are summarized in the corresponding legend. The coupling of the dibenzyl derivative **7** with **8** gave a mixture of the two atropodiastereomers **9a** and **9b** in a 1.4:1 ratio. Since both diastereomeric target molecules **10a** and **10b** occur in nature, and also since both were desired for subsequent synthesis of michellamines (e.g., see Example 3), the precursors **9a** and **9b** were not necessarily resolved, but were immediately deprotected by treatment with $BCl_3$ to cleave the isopropyl and benzyl ether functions. Subsequent catalytic hydrogenation gave the free amino forms of **10a** and **10b,** which were then isolated and purified individually by HPLC on an amino-bonded phase column as published (Hallock et al., supra). All compounds in all three of the above synthetic stages were verified to have the appropriate physicochemical, spectral and other analytical data.

The method of synthesis for other monomeric alkaloids (i.e., in particular, non-korupensamine alkaloids) is done using the same or similar chemical principles as is illustrated for korupensamine A and B. More detail regarding this technology is set forth in **Figures 5-7.**

EXAMPLE 2: Synthesis of Monomeric Alkaloid Derivatives

This example further illustrates methods for the preparation of medically useful new derivatives of naphthylisoquinoline alkaloids which are either naturally occurring or are prepared according to the aforementioned methods of the present invention and which either may be a korupensamine, a related monomeric naphthylisoquinoline alkaloid, a non-korupensamine, or other monomeric naphthylisoquinoline alkaloid.

Using standard organic chemical methodology (particularly as set forth in François et al., PCT Application PCT/US95/01717 and Boyd et al., U.S. Patent 5,409,938, as well as set forth herein), one or more structural modifications of the aforementioned synthetic naphthylisoquinoline alkaloids can be made to provide derivatives with modified biological properties which may advantageously be useful for treatment of certain host mammal species and/or against certain pathogenic agents, particularly parasitic species such as strains of malaria. Such properties may, for example, include one or more of the following: greater therapeutic potency, particularly antimalarial potency; broader spectrum of therapeutic activity, particularly antimalarial activity; enhanced oral bioavailability; less host toxicity; and more advantageous pharmacokinetics and/or tissue distribution.

Depending on the stoichiometric amount of the particular reactant, the naphthylisoquinoline compound can be substituted at one, some, or all of the respective available positions. For example, when such a compound is reacted with a certain amount of $CH_3COCl$, acetate can be introduced at one, some, or all the available OH or NH positions.

Examples of these include, but are not limited to:

1. Conversion to ester, sulfonate ester, and ether substituents at one or more phenolic hydroxyl positions in the naphthylisoquinoline compound.

For example, for preparation of esters or sulfonate esters, the selected naphthylisoquinoline compound can be reacted with an acid halide (RCOX or $RSO_2X$, where X = Cl, Br, or I, and R is an $C_1$-$C_6$ aliphatic or aromatic radical) in anhydrous pyridine or triethylamine.

Alternatively, the selected compound may be reacted with an acid ($RCO_2H$ or $RSO_3H$ wherein R is an aliphatic or aromatic radical) and dicyclohexylcarbodiimide in triethylamine to prepare the ester or sulfonate ester.

For preparation of ethers, the selected naphthylisoquinoline compound is reacted with an organic halide (e.g., RX, $RCH_2$-X (where X = Cl, Br, or I), OTf, or OTs, and R is a $C_1$-$C_6$ aliphatic or aromatic radical) in anhydrous acetone with anhydrous potassium carbonate or with phase transfer catalysis.

For instance:

dioncophylline C $\xrightarrow[\text{pyridine}]{CH_3COCl}$ "dioncophylline C acetate"

dioncophylline C $\xrightarrow[K_2CO_3\ \text{acetone}]{CH_3I}$ "O-methyl dioncophylline C"

2. Removal of (an) ether methyl group(s) to provide a phenolic hydroxyl functionality and/or conversion of that moiety to an ester, sulfonate, or other ether.

For example, for hydrolytic cleavage of the methyl ether and conversion to phenolic hydroxyl, the selected naphthylisoquinoline compound is reacted with $BBr_3$, $BX_3{\cdot}(CH_3)_2S$ in $CH_2Cl_2$ (where X = F, Cl or Br), FtS, or other ether cleaving reactants. The resulting phenol can be converted to esters, sulfonate esters or ethers as described above.

For instance:

dioncophylline C $\xrightarrow[CH_2Cl_2]{BF_3 \cdot (CH_3)_2S}$ "O-demethyl dioncophylline C"

3. Preparation of amide or sulfonamide derivatives at the amine site in a selected naphthylisoquinoline compound.

For example, for preparation of amide or sulfonamide derivatives, the same general procedures described above (in 1) apply. In either case (1 or 3), an appropriate functional group protection strategy (blocking/deblocking of selected groups) is applied.

For instance:

dioncophylline C $\xrightarrow[Et_3N]{\text{benzenesulfonyl chloride}}$ "dioncophylline C benzene-sulfonamide"

4. Conversion of the secondary amine functionality to an alkyl quaternary ammonium salt or to a tertiary amine.

For example, for preparation of tertiary amines, the selected naphthylisoquinoline alkaloid is reacted with an aldehyde and the resulting product reduced with $NaBH_4$.

Alternatively, for preparation of an alkyl ammonium salt, the selected naphthylisoquinoline alkaloid is reacted with an alkyl halide (RX, where X = Cl, Br or I, and R is an $C_1$-$C_6$ aliphatic radical) in anhydrous aprotic solvent.

For instance:

dioncophylline C $\xrightarrow[CH_2Cl_2]{CH_3I}$ "dioncophylline C dimethyl-ammonium iodide"

5. Conversion of the tertiary amine function to a secondary amine.

For example, for preparation of a secondary amine, a selected N-alkyl naphthylisoquinoline compound is reacted with cyanogen bromide to give the corresponding cyanamide, which is then treated with $LiAlH_4$.

For instance:

N-methyl-6-deoxyancistrocladine $\xrightarrow{BrCN}$ "6-deoxyancistrocladine cyanamide"

6-deoxyancistrocladine cyanamide $\xrightarrow[THF]{LiAlH_4}$ "6-deoxyancistrocladine"

6. Conversion of one or more phenolic hydroxyl groups to an aromatic hydrogen substituent.

For example, the selected naphthylisoquinoline compound is converted (after suitable protection of the amine function if necessary) to the triflic ester, followed by reductive deoxygenation of the triflic ester to give the corresponding derivative.

For instance:

N-methylancistrocladine $\xrightarrow[Tf_2O]{TlOEt\text{-}CH_2Cl_2}$ "N-methylancistrocladine, triflic ester"

N-methylancistrocladine, triflic ester $\xrightarrow[HCO_2H,\ dppp,\ DMF]{(Ph_3P)_2\ PdCl_2,\ nBu_3N,}$ "6-deoxy-N-methylancistrocladine"

7. Substitution of one or more hydrogen substituents on the aryl systems by halogen, nitro, amino, hydroxyl, thiol, or cyano groups.

For example, for preparation of bromine-substituted derivatives, the selected naphthylisoquinoline compound is reacted with $Br_2$ in $H_2O$. For preparation of other substituted derivatives, the selected naphthylisoquinoline compound is treated with $HNO_3$/HOAc to provide nitro-substituted ($-NO_2$) derivatives. In turn, the nitro derivative can be reduced to the amino derivative. The amino-derivative is the point of origin of the chloro, iodo, cyano, thiol, and hydroxyl substitution via well known and practiced diazonium substitution reactions.

For instance:

dioncophylline C $\xrightarrow[H_2O]{Br_2}$ "bromodioncophylline C"

dioncophylline C $\xrightarrow[HOAc]{HNO_3}$ "nitro dioncophylline C" $\xrightarrow{[H]}$

"aminodioncophylline C" $\xrightarrow[HCl]{NaNO_2}$ "diazodioncophylline C"

"diazodioncophylline C" $\xrightarrow{CuCl}$ "chlorodioncophylline C"

"diazodioncophylline C" $\xrightarrow{CuCN}$ "cyanodioncophylline C"

"diazodioncophylline C" $\xrightarrow{H_2O}$ "hydroxydioncophylline C"

Additionally, the following new modifications are disclosed herein:

1. Substitution of one or more hydrogen substituents on the aryl systems by acyl or $C_1$-$C_6$ alkyl.

For example, for preparation of an acyl derivative, the suitably protected (e.g., *N*-benzylated) naphthylisoquinoline is reacted with RCOCl and $AlCl_3$ to give a corresponding acyl derivative, which can then be deprotected (e.g., by *N*-debenzylation) if desired. For preparation of the corresponding alkyl naphthylisoquinoline, the acyl naphthylisoquinoline is treated with $LiAlH_4/AlCl_3$.

2. Replacement of a methyl group with a hydrogen substituent.

For example, a methyl-substituted naphthylisoquinoline may be oxidized to give a corresponding carboxyl-substituted naphthylisoquinoline, which may then be decarboxylated to give the final desired (demethylated) naphthylisoquinoline.

EXAMPLE 3: Synthesis of Michellamines

This example describes more fully the synthesis of a michellamine, specifically michellamine A (**13**). The method is summarized in **Figure 12**; the reaction conditions and yields are summarized in the corresponding legend.

**11:** A mixture of korupensamine A (**10a**) (50.0 mg, 0.13 mmol), pivalic formic anhydride (26 µl, 0.16 mmol) and dry $CH_2Cl_2$ (10 ml) was stirred at 20°C for 2 h. Removal of the solvent in vacuum afforded a brown solid, which was dissolved in dry $CH_2Cl_2$ (10 ml). After addition of acetyl chloride (24 µl, 0.33 mmol), $NEt_3$ (46 µl, 0.33 mmol) and a catalytic amount of DMAP, the reaction mixture was stirred for 5 h. After treatment with aqueous $NH_4Cl$ (2M, 5 ml), the organic layer was filtered through deactivated (5% $NH_3$) silica gel. Crystallization from $CH_2Cl_2$/diethyl ether/petroleum ether afforded **11** (58.4 mg, 0.12 mmol, 90%; m.p. 159°C, $[\alpha]_D^{20}$ = + 9.3 (c = 0.50 in $CHCl_3$).

**12:** A solution of **11** (40.1 mg, 0.82 mmol) in dry $CHCl_3$ (50 ml) containing 0.2% $NEt_3$ was treated with $Ag_2O$ (401 mg, 1.73 mmol). After 5d stirring at 20°C, the solvent was removed and the residue purified by chromatography on deactivated (5% $NH_3$) silica gel with $CH_2Cl_2$/methanol (95:5) as eluent. The crude product was crystallized from $CH_2Cl_2$/diethylether/petroleum ether, to give **12** (33.9 mg, 5.20 µmol, 85%) as a deep-violet colored powder (m.p. decomp. >230°C, $[\alpha]_D^{20}$ = +31 (c = 0.0023 in $CHCl_3$)). The E-configuration at the central double bond was not established, but is plausible for steric reasons and in analogy to Laatsch (supra).

**13:** A solution of **12** (5.10 mg, 5.20 µmol) was treated with $NaBH_4$ (1.00 mg, 26.4 µmol) in dry *i*PrOH (1 ml) for 10 min at 20°C. The solvent was evaporated and the residue was dissolved in diethyl ether and extracted several times with water. The organic phases were dried over $Na_2SO_4$ and the solvent was evaporated under vacuum. A solution of the resulting oil in dry methanol (2 ml) was treated with portions (1 ml) of cold-saturated methanolic HCl over a period of 24 h while gently refluxing. After removal of the solvent, HPLC on a semi-preparative amino-bonded phase column (Rainin Dynamax-60A) with $CH_2Cl_2$/methanol/$(NH_4)_2CO_3$ (90:10:0.01) as eluent afforded **13** (2.64 mg, 3.48 µmol, 67%), which was characterized as its diacetate salt as described previously (Manfredi et al., supra; Boyd, et al., supra).

EXAMPLE 4: Synthesis of Other Dimeric Naphthylisoquinoline Alkaloids

This example sets forth in further detail the synthesis of a representative new dimeric naphthylisoquinoline alkaloid, specifically, in this instance, a homodimer comprised of two monomeric dioncopeltine A "halves", which lack a C-8' to C-5 naphthalene/tetrahydroisoquinoline linkage. The method is outlined in **Figure 14**; the reaction conditions and yields are summarized in the corresponding legend. Analogous to the above-mentioned procedure for korupensamine A dioncopeltine A (**14**) was submitted to the partial protection and dimerization sequence:

**15:** A suspension of **14** (190 mg, 0.50 mmol), $K_2CO_3$ (2070 mg, 1.50 mmol) and benzyl bromide (0.25 ml, 359 mg, 2.09 mmol) in acetone was refluxed for 4 h. Excessive $K_2CO_3$ was filtered off and the solvent was removed under vacuum. The crude product was crystallized from dichloromethane/petroleum ether to afford the corresponding *O*,*N*-dibenzyldioncopeltine A (269 mg, 0.48 mmol) as colorless crystals (m.p. 150-151°C $[\alpha]_D^{20}$ = 61.8° (c = 0.5 in chloroform).

To a solution of this dibenzyldioncopeltine A (257 mg, 0.46 mmol) in dichloromethane (55 ml), $NEt_3$ (100 µl, 0.54 mmol) and a catalytic amount of DMAP were added. After stirring for 5 min at 20°C, acetyl chloride (55 µl, 0.77 mmol) was added. After stirring for 2h, the reaction mixture was quenched with saturated $NH_4Cl$-solution and the layers were separated. The solvent was evaporated and the residue was filtered over deactivated (7.5% $NH_3$) silica gel to afford **15** (273 mg, 0.45 mmol, 99%) as a light yellow oil.

**16:** A solution of **15** (20 mg, 0.33 mmol) in dry chloroform (2 ml) containing 0.2% triethylamine was treated with $Ag_2O$ (86 mg, 0.37 mmol). After 6h stirring at 20°C, the catalyst was filtered over deactivated (5% $NH_3$) silica gel to afford **16** as a deep violet amorphous powder.

**17:** A solution of **16** in methanol was irradiated ($\lambda_{max}$ = 400 nm) at 20°C for 2h. A catalytic amount of Pd-C (10%) was added and the suspension was hydrogenated at ambient $H_2$-pressure. The catalyst was filtered off (Celite) with methanol as eluent. The product was purified by HPLC.

EXAMPLE 5: Synthesis of Derivatives of Dimeric Naphthylisoquinoline Alkaloids

This example more fully illustrates methods for obtaining medically useful new derivatives of naphthylisoquinoline alkaloids prepared according to the aforementioned methods of the present invention.

Using standard organic chemical methodology, one or more structural modifications of the aforementioned dimeric naphthylisoquinoline alkaloids prepared according to the present invention can be made to provide derivatives with modified biological properties which may be advantageously useful for treatment of certain host mammal species

and/or against certain pathogenic agents. Such properties may, for example, include one or more of the following: greater therapeutic potency, broader spectrum of therapeutic activity, enhanced oral bioavailability, less host toxicity, more advantageous pharmacokinetics and/or tissue distribution.

Such methods were previously set forth by Boyd et al. (PCT Application WO 94/24108) for michellamines. However, since the novel compounds of the present invention were not known at that time, these methods were not applied to these compounds. Accordingly, this example illustrates the modification of dimeric naphthylisoquinoline alkaloids other than the michellamines, and the derivatives resulting therefrom. Furthermore, this example sets forth novel methods of modification not disclosed therein.

Depending on the stoichiometric amount of the particular reactant, the naphthylisoquinoline compound can be substituted at one, some, or all of the respective available positions. For example, when such a compound is reacted with a certain amount of $CH_3COCl$, acetate can be introduced at one, some, or all the available OH or NH positions.

Examples of these include, but are not limited to:

1. Conversion to ester, sulfonate ester, and ether substituents at one or more phenolic hydroxyl positions in the naphthylisoquinoline compound.

For example, for preparation of esters or sulfonate esters, the selected naphthylisoquinoline compound can be reacted with an acid halide (RCOX or $RSO_2X$, where X = Cl, Br, or I, and R is an $C_1$-$C_6$ aliphatic or aromatic radical) in anhydrous pyridine or triethylamine.

Alternatively, the selected compound may be reacted with an acid ($RCO_2H$ or $RSO_3H$ wherein R is an aliphatic or aromatic radical) and dicyclohexylcarbodiimide in triethylamine to prepare the ester or sulfonate ester.

For preparation of ethers, the selected naphthylisoquinoline compound is reacted with an organic halide (e.g., RX, or $RCH_2$-X, where X = Cl, Br, I, OTf, or OTs, and R is a $C_1$-$C_6$ aliphatic or aromatic radical) in anhydrous acetone with anhydrous potassium carbonate or with phase transfer catalysis.

For instance:

michellamine B --------► ($CH_3COCl$ / pyridine) "michellamine B acetate"

michellamine B --------► ($CH_3I$ / $K_2CO_3$ acetone) "O-methyl michellamine B"

2. Removal of (an) ether methyl group(s) to provide a phenolic hydroxyl functionality and/or conversion of that moiety to an ester, sulfonate, or other ether.

For example, for hydrolytic cleavage of the methyl ether and conversion to phenolic hydroxyl, the selected naphthylisoquinoline compound is reacted with $BBr_3$, $BX_3 \cdot (CH_3)_2S$ in $CH_2Cl_2$ (where X = F, Cl, or Br), FtS, or other ether cleaving reactants. The resulting phenol can be converted to esters, sulfonate esters or ethers as described above.

For instance:

michellamine B ---------------► ($BF_3 \bullet (CH_3)_2S$ / $CH_2Cl_2$) "O-demethyl michellamine B"

3. Preparation of amide or sulfonamide derivatives at the amine site in a selected naphthylisoquinoline compound.

For example, for preparation of amide or sulfonamide derivatives, the same general procedures described above (in 1) apply. In either case (1 or 3), an appropriate functional group protection strategy (blocking/deblocking of

selected groups) is applied.

For instance:

$$\text{michellamine B} \xrightarrow[Et_3N]{\text{benzenesulfonyl chloride}} \text{"michellamine B benzene-sulfonamide"}$$

4. Conversion of the secondary amine functionality to an alkyl quaternary ammonium salt or to a tertiary amine.

For example, for preparation of tertiary amines, the selected naphthylisoquinoline alkaloid is reacted with an aldehyde and the resulting product reduced with $NaBH_4$.

Alternatively, for preparation of an alkyl ammonium salt, the selected naphthylisoquinoline alkaloid is reacted with an alkyl halide (RX, where X = Cl, Br, or I, and R is an $C_1$-$C_6$ aliphatic radical) in anhydrous aprotic solvent.

For instance:

$$\text{michellamine B} \xrightarrow[CH_2Cl_2]{CH_3I} \text{"michellamine B dimethyl-ammonium iodide"}$$

5. Substitution of one or more hydrogen substituents on the aryl systems by halogen, nitro, amino, hydroxyl, thiol, or cyano groups.

For example, for preparation of bromine-substituted derivatives, the selected naphthylisoquinoline compound is reacted with $Br_2$ in $H_2O$, HOAc, or $CHCl_3$. For preparation of other substituted derivatives, the selected naphthylisoquinoline compound is treated with $HNO_3$/HOAc to provide nitro-substituted ($-NO_2$) derivatives. In turn, the nitro derivative can be reduced to the amino derivative. The amino-derivative is the point of origin of the chloro, iodo, cyano, thiol, and hydroxyl substitution via well known and practiced diazonium substitution reactions.

For instance:

michellamine B —$Br_2$, $H_2O$→ "bromomichellamine B"

michellamine B —$HNO_3$, HOAc→ "nitro michellamine B" —[H]→

"aminomichellamine B" —$NaNO_2$, HCl→ "diazomichellamine B"

"diazomichellamine B" —CuCl→ "chloromichellamine B"

"diazomichellamine B" —CuCN→ "cyanomichellamine B"

"diazomichellamine B" —$H_2O$→ "hydroxymichellamine B"

Additionally, the following new modifications are disclosed herein:

1. Conversion of the tertiary amine function (which may be prepared by reaction (4)) to a secondary amine.

For example, for preparation of a secondary amine, a selected N-alkyl naphthylisoquinoline compound is reacted with cyanogen bromide to give the corresponding cyanamide, which is then treated with $LiAlH_4$.

For instance:

N-methyl-michellamine B —BrCN→ "michellamine B cyanamide"

Michellamine B cyanamide —$LiAlH_4$, THF→ "michellamine B"

2. Conversion of one or more phenolic hydroxyl groups to an aromatic hydrogen substituent.

For example, the selected naphthylisoquinoline compound is converted (after suitable protection of the amine function if necessary) to the triflic ester, followed by reductive deoxygenation of the triflic ester to give the corresponding 6-deoxykorupensamine.

For instance:

$TlOEt-CH_2Cl_2$

N-methyl-michellamine B --------------▶ "N-methyl-michellamine B triflic ester"

$Tf_2O$

$(Ph_3P)_2\ PdCl_2$, $nBu_3N$,

N-methyl-michellamine B ----------------------▶ "deoxy-N-triflic ester methyl-michellamine B"

$HCO_2H$, dppp, DMF

3. Substitution of one or more hydrogen substituents on the aryl systems by acyl or alkyl.

For example, for preparation of an acyl derivative, the suitably protected (e.g., *N*-benzylated) naphthylisoquinoline is reacted with RCOCl and $AlCl_3$ to give a corresponding acyl derivative, which can then be deprotected (e.g., by *N*-debenzylation) if desired. For preparation of the corresponding alkyl naphthylisoquinoline, the acyl naphthylisoquinoline is treated with $LiAlH_4/AlCl_3$.

4. Replacement of a methyl group with a hydrogen substituent.

For example, a methyl-substituted naphthylisoquinoline may be oxidized to give a corresponding carboxyl-substituted naphthylisoquinoline, which may then be decarboxylated to give the final desired (demethylated) naphthylisoquinoline.

All of the references cited herein, including patents, patent applications, literature publications, and the like, are hereby incorporated in their entireties by reference.

While this invention has been described with an emphasis upon preferred embodiments, it will be obvious to those of ordinary skill in the art that variations of the preferred compounds and methods may be used and that it is intended that the invention may be practiced otherwise than as specifically described herein. Accordingly, this invention includes all modifications encompassed within the spirit and scope of the invention as defined by the following claims.

## Claims

**1.** A compound of the formula

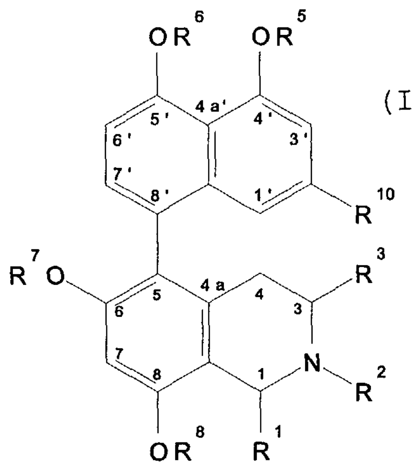

(I)

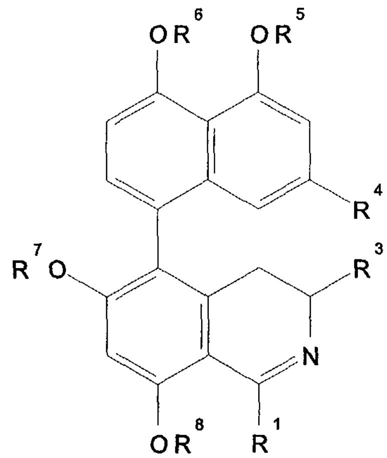

(II)

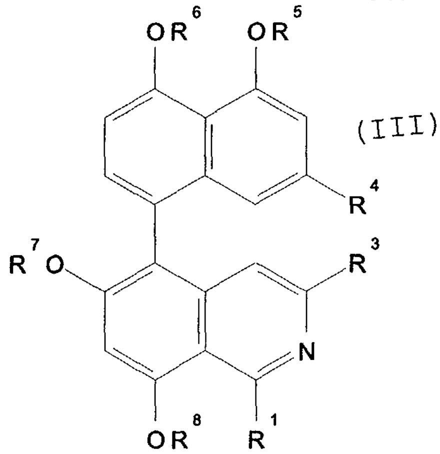

(III)

wherein (a) $R^1$, $R^3$, $R^4$, and $R^{10}$ may be the same or different and each may be H or $C_1$-$C_6$ alkyl, $R^2$, $R^5$, $R^6$, $R^7$, and $R^8$ may be the same or different and each may be H, $C_1$-$C_6$ alkyl, $R^9CH_2$-, $R^9CO$-, or $R^9SO_2$-, $R^9$ may be H, $C_1$-$C_6$ alkyl, or aryl, and one or more of the ring positions 1, 3, 4, 1', 2', 3', 4', 5', 6', 7', 6, 7, and 8 may be substituted with halo, nitro, amino, hydroxyl, thiol, acyl, $C_1$-$C_6$ alkyl, or cyano, (b) one or more phenolic hydroxyl group(s) may instead be a sulfonate ester, one or more methyl ether group(s) may instead be a phenolic hydroxyl group, one or more phenolic hydroxyl group(s) may instead be an aromatic hydrogen substituent, and one or more secondary amine site(s) may instead be an amide, sulfonamide, tertiary amine, alkyl quaternary ammonium salt or corresponding Hoffmann elimination product thereof, one or more tertiary amine site(s) may instead be a secondary amine, and one or more aromatic hydrogen substituent(s) may instead be halo, nitro, amino, hydroxyl, thiol, cyano, acyl or $C_1$-$C_6$ alkyl substituent; with the proviso that $R^{10}$ is not methyl when $R^1$ and $R^3$ are methyl.

**2.** A dimeric naphthylisoquinoline alkaloid comprised of coupled first and second naphthylisoquinoline alkaloid monomers which are the same or different, wherein said first and second monomers are selected from the compounds of claim 1.

**3.** The dimeric alkaloid of claim 2 wherein said first and second monomers are selected from compounds of formulae (II) and (III) as set out in claim 1.

**4.** The dimeric alkaloid of claim 2 wherein said first and second monomers are selected from compounds of formula (I) as set out in claim 1, wherein $R^1$ and $R^3$ are H.

**5.** The dimeric alkaloid of claim 2 wherein said first and second monomers are selected from compounds of formula (I) as set out in claim 1 wherein $R^{10-}$ is H.

**6.** A dimeric naphthylisoquinoline alkaloid comprised of coupled first and second naphthylisoquinoline alkaloid monomers which are the same or different, wherein said first monomer lacks a C-8' to C-5 naphthalene/isoquinoline

linkage, and said second monomer may or may not possess a C-8' to C-5 naphthalene/isoquinoline linkage.

**7.** The dimeric naphthylisoquinoline alkaloid of claim 6, wherein said first monomer is selected from the group consisting of dioncophylline B, dioncopeltine A, dioncophylline A, dioncophylline C, dioncolactone A, *N*-methyl-dioncophylline A, ancistrobrevine D, ancistrocladine, 5'-*O*-demethyl-8-*O*-methyl-7-*epi*-dioncophylline A, 5'-*O*-demethyl-7-*epi*-dioncophylline A, dioncophylleine A, (±)-dioncophyllacine A, hamatine, ancistrobrevine A, 6-*O*-demethyl-ancistrobrevine A, ancistrobarterine A, 7-*epi*-dioncophylline A, *N*-formyl ancistrocladine, *N*-methyl-ancistrocladine, 6-deoxy-*N*-methyl-ancistrocladine, *N*-formyl-*O,O*-dimethyl-dioncophylline C, *N*-formyl-dioncophylline C, *N*-formyl-8-*O*-benzyl-dioncophylline C, *N*-formyl-8-*O*-methyl-dioncophylline C, *N*-formyl-8-*O*-pivaloyl-dioncophylline C, *N*-formyl-8-*O*-acetyl-dioncophylline C, *N*-formyl-8-*O*-benzoyl-dioncophylline C, and 8-*O*-methyl-dioncophylline C, wherein the configurations at C-1 and C-3 may instead be the same or different and each may be *R* or *S*, the coupling points comprising the naphthalene/isoquinoline axis may be different, the configuration about the axis may be different, one or more phenolic hydroxyl group(s) may instead be a sulfonate ester, one or more methyl ether group(s) may instead be a phenolic hydroxyl group, one or more phenolic hydroxyl group(s) may instead be an aromatic hydrogen substituent, and one or more secondary amine site(s) may instead be an amide, sulfonamide, tertiary amine, alkyl quaternary ammonium salt or corresponding Hoffmann elimination product thereof, one or more tertiary amine site(s) may instead be a secondary amine, one or more aromatic hydrogen substituent(s) may instead be a halogen, nitro, amino, hydroxyl, thiol, acyl, $C_1$-$C_6$ alkyl, or cyano substituent, $CH_3$ may instead be H, and the tetrahydroisoquinoline may instead be a dihydroisoquinoline or a fully aromatic isoquinoline.

**8.** The dimeric naphthylisoquinoline alkaloid of claim 6, or wherein said second monomer is a compound of the formula (I), (II), or (III) as set out in claim 1.

**9.** The dimeric naphthylisoquinoline alkaloid of claim 6, wherein said first and second monomers are different, and second monomer is selected from the group consisting of dioncophylline B, dioncopeltine A, dioncophylline A, dioncophylline C, dioncolactone A, *N*-methyl-dioncophylline A, ancistrobrevine D, ancistrocladine, 5'-*O*-demethyl-8-*O*-methyl-7-*epi*-dioncophylline A, 5'-*O*-demethyl-7-*epi*-dioncophylline A, dioncophylleine A, (±)-dioncophyllacine A, hamatine, ancistrobrevine A, 6-*O*-demethyl-ancistrobrevine A, ancistrobarterine A, 7-*epi*-dioncophylline A, *N*-formyl ancistrocladine, *N*-methyl-ancistrocladine, 6-deoxy-*N*-methyl-ancistrocladine, *N*-formyl-*O*,*O*-dimethyl-dioncophylline C, *N*-formyl-dioncophylline C, *N*-formyl-8-*O*-benzyl-dioncophylline C, *N*-formyl-8-*O*-methyl-dioncophylline C, *N*-formyl-8-*O*-pivaloyl-dioncophylline C, *N*-formyl-8-*O*-acetyl-dioncophylline C, *N*-formyl-8-*O*-benzoyl-dioncophylline C, and 8-*O*-methyl-dioncophylline C, wherein the configurations at C-1 and C-3 may instead be the same or different and each may be *R* or *S*, the coupling points comprising the naphthalene/isoquinoline axis may be different, the configuration about the axis may be different, one or more phenolic hydroxyl group(s) may instead be a sulfonate ester, one or more methyl ether group(s) may instead be a phenolic hydroxyl group, one or more phenolic hydroxyl group(s) may instead be an aromatic hydrogen substituent, and one or more secondary amine site(s) may instead be an amide, sulfonamide, tertiary amine, alkyl quaternary ammonium salt or corresponding Hoffmann elimination product thereof, one or more tertiary amine site(s) may instead be a secondary amine, one or more aromatic hydrogen substituent(s) may instead be a halogen, nitro, amino, hydroxyl, thiol, acyl, $C_1$-$C_6$ alkyl, or cyano substituent, $CH_3$ may instead be H, and the tetrahydroisoquinoline may instead be a dihydroisoquinoline or a fully aromatic isoquinoline.

**10.** The dimeric alkaloid of claim 6 or claim 7 wherein said first and second monomers are the same, both lacking a $C-8^1$ to C-5 naphthalene/isoquinoline linkage, and are coupled through a carbon-carbon bond.

**11.** The dimeric alkaloid of claim 6 wherein one of the monomers is as defined in claim 7, the other of said monomers is different and is as defined in claim 8, and said monomers are coupled through a carbon-carbon bond.

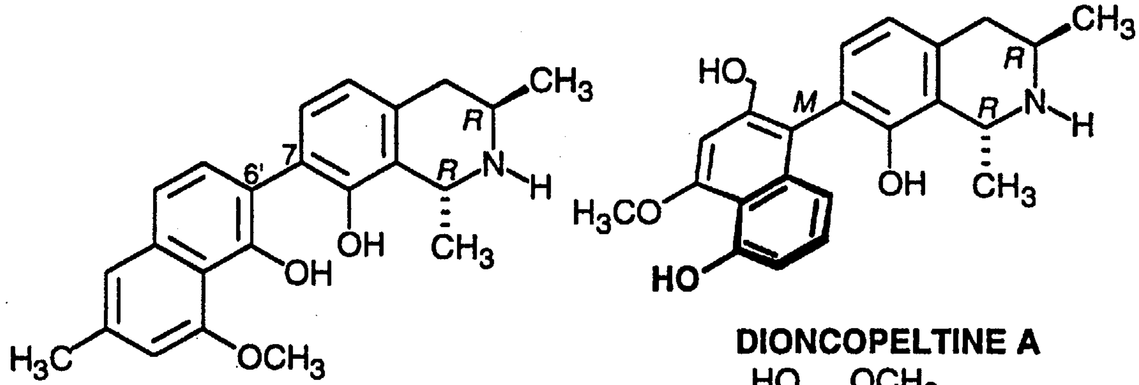

DIONCOPHYLLINE B

DIONCOPELTINE A

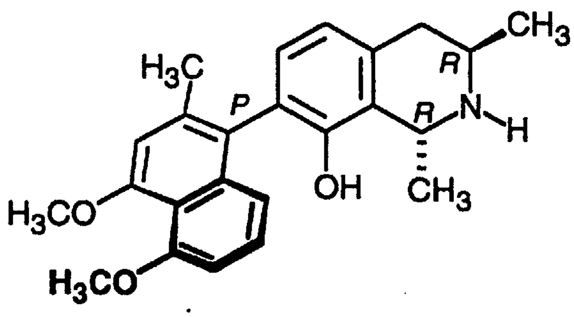

DIONCOPHYLLINE A

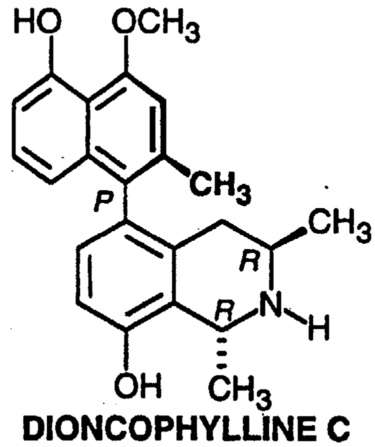

DIONCOPHYLLINE C

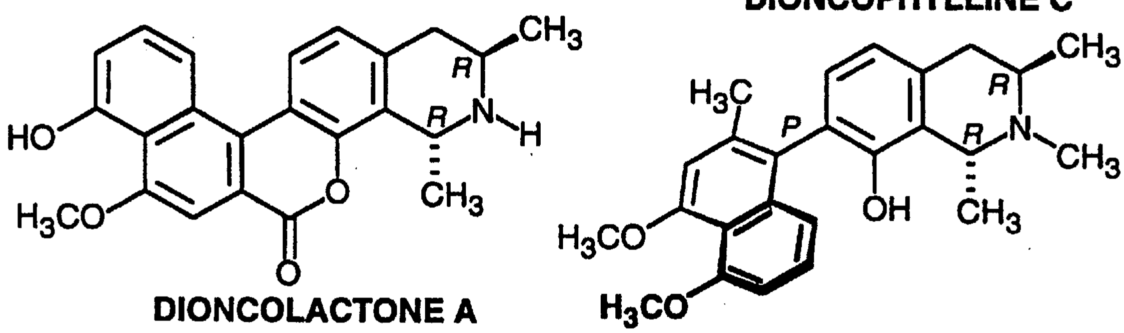

DIONCOLACTONE A

*N*-METHYL-DIONCOPHYLLINE A

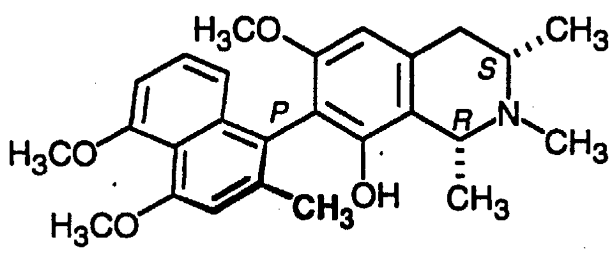

ANCISTROBREVINE D

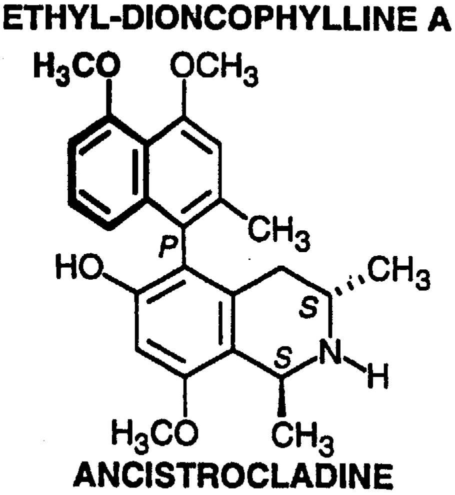

ANCISTROCLADINE

FIG. 1

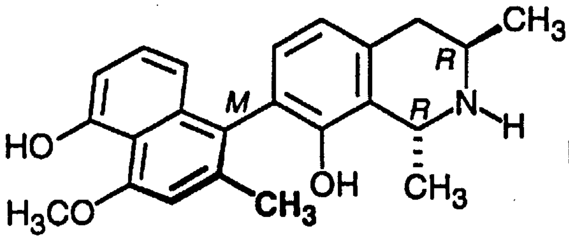

5'-*O*-DEMETHYL-
7-*EPI*-DIONCOPHYLLINE A

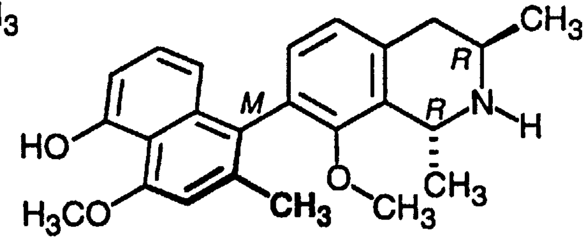

5'-*O*-DEMETHYL-8-*O*-METHYL
7-*EPI*-DIONCOPHYLLINE A

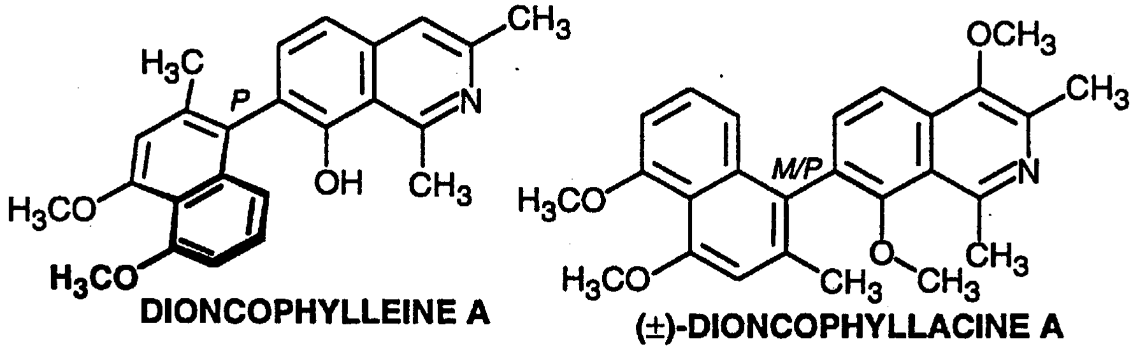

DIONCOPHYLLEINE A

(±)-DIONCOPHYLLACINE A

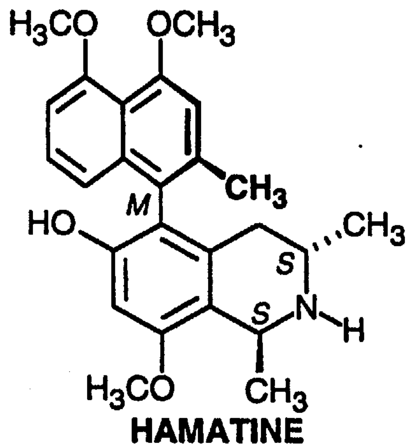

HAMATINE

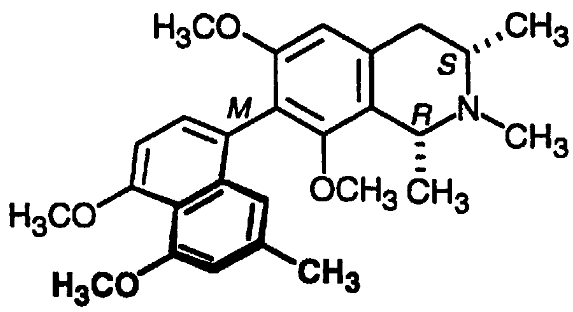

ANCISTROBREVINE A

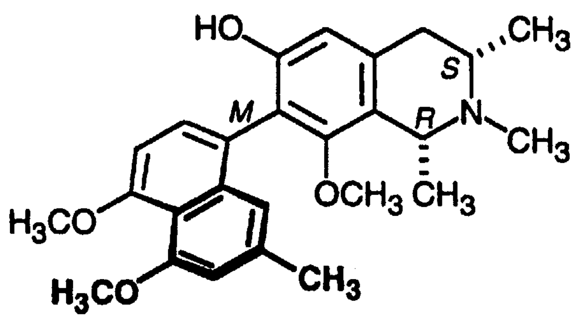

6-*O*-DEMETHYL-ANCISTROBREVINE A

**FIG. 1**
**(CONTINUED)**

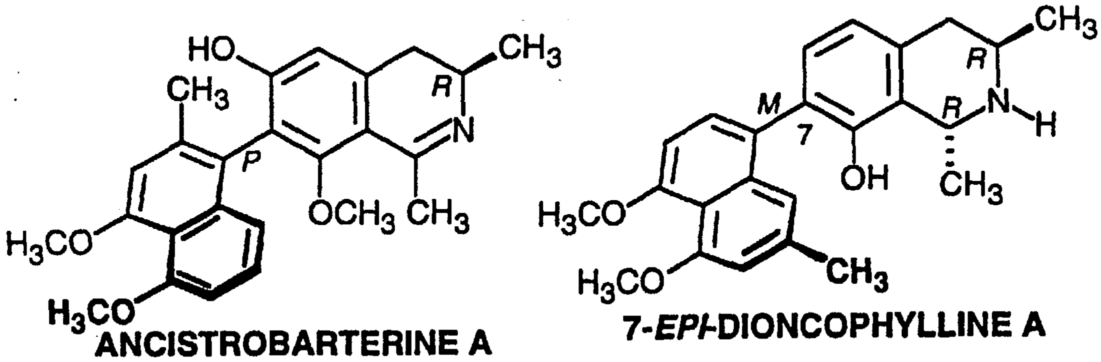

ANCISTROBARTERINE A
(6-*O*-DEMETHYL-8-*O*-METHYL-
7-*EPI*-ANCISTROBREVINE C)

7-*EPI*-DIONCOPHYLLINE A

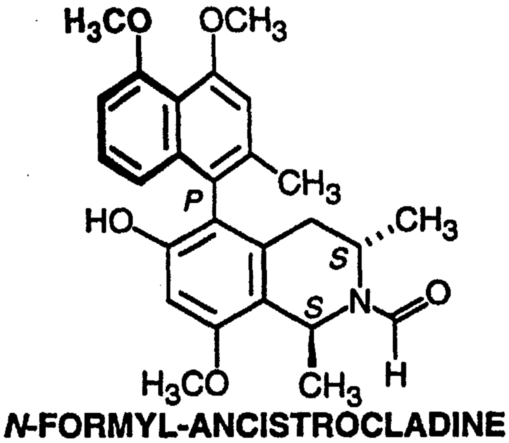

*N*-FORMYL-ANCISTROCLADINE

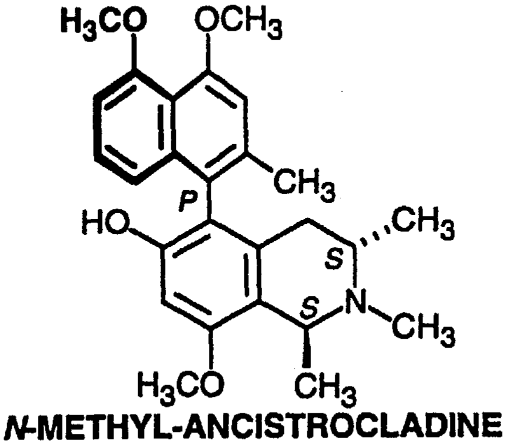

*N*-METHYL-ANCISTROCLADINE

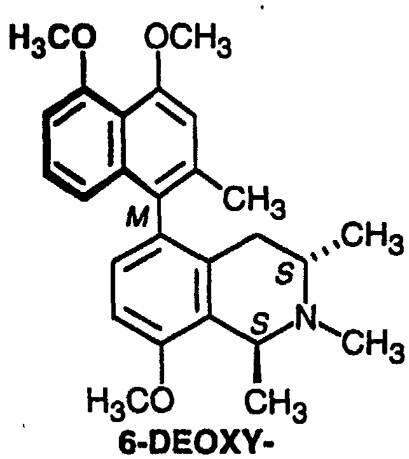

6-DEOXY-
*N*-METHYL-ANCISTROCLADINE

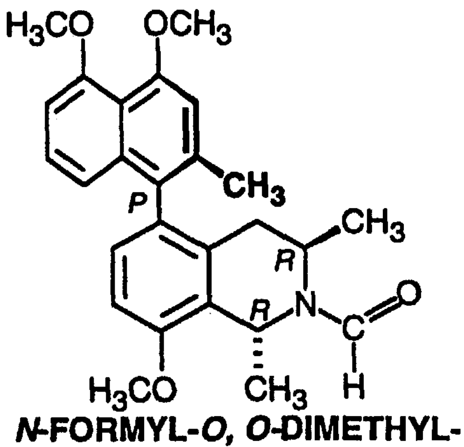

*N*-FORMYL-*O*, *O*-DIMETHYL-
DIONCOPHYLLINE C

**FIG. 1**
**(CONTINUED)**

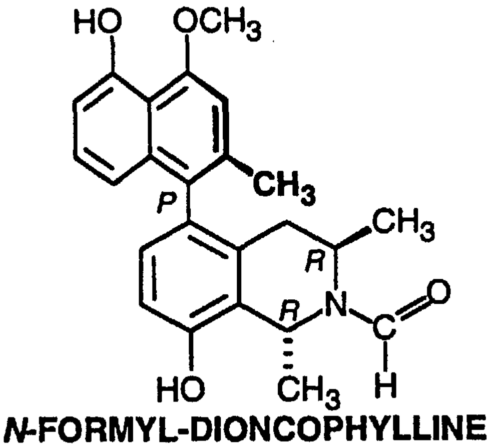

***N*-FORMYL-DIONCOPHYLLINE C**

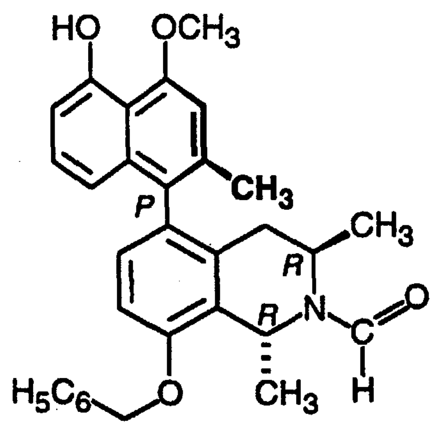

***N*-FORMYL-8-*O*-BENZYL-DIONCOPHYLLINE C**

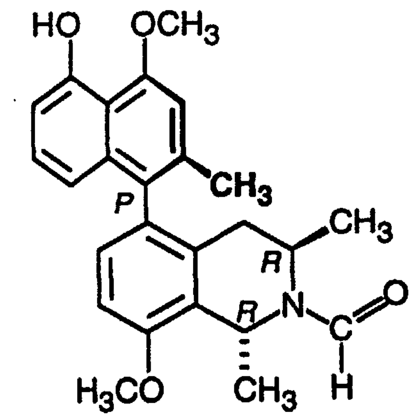

***N*-FORMYL-8-*O*-METHYL-DIONCOPHYLLINE C**

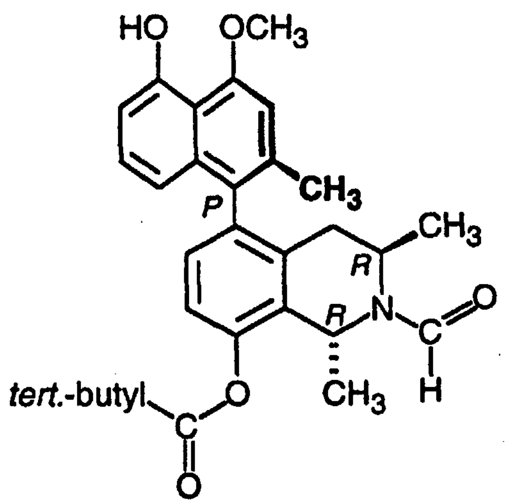

***N*-FORMYL-8-*O*-PIVALOYL-DIONCOPHYLLINE C**

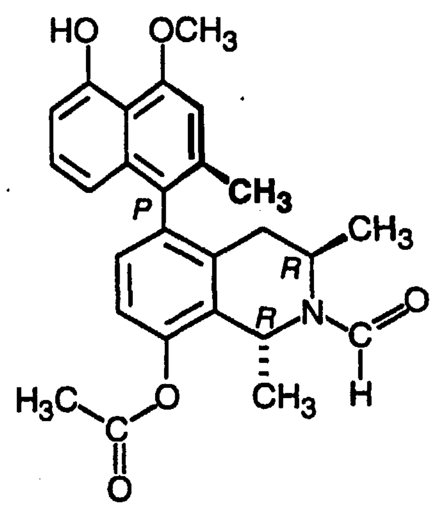

***N*-FORMYL-8-*O*-ACETYL-DIONCOPHYLLINE C**

**FIG. 1**
**(CONTINUED)**

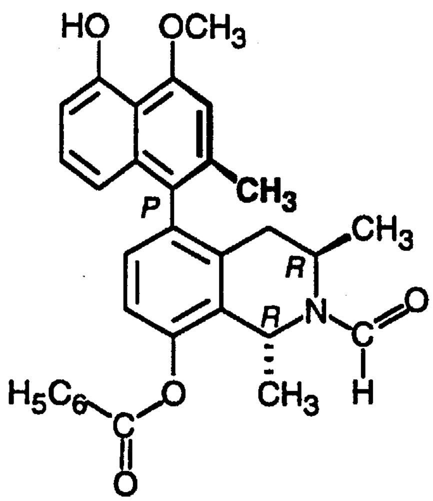

*N*-FORMYL-8-*O*-BENZOYL-
DIONCOPHYLLINE C

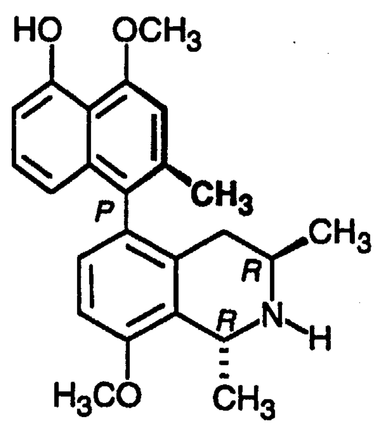

8-*O*-METHYL-
DIONCOPHYLLINE C

**FIG. 1**
**(CONTINUED)**

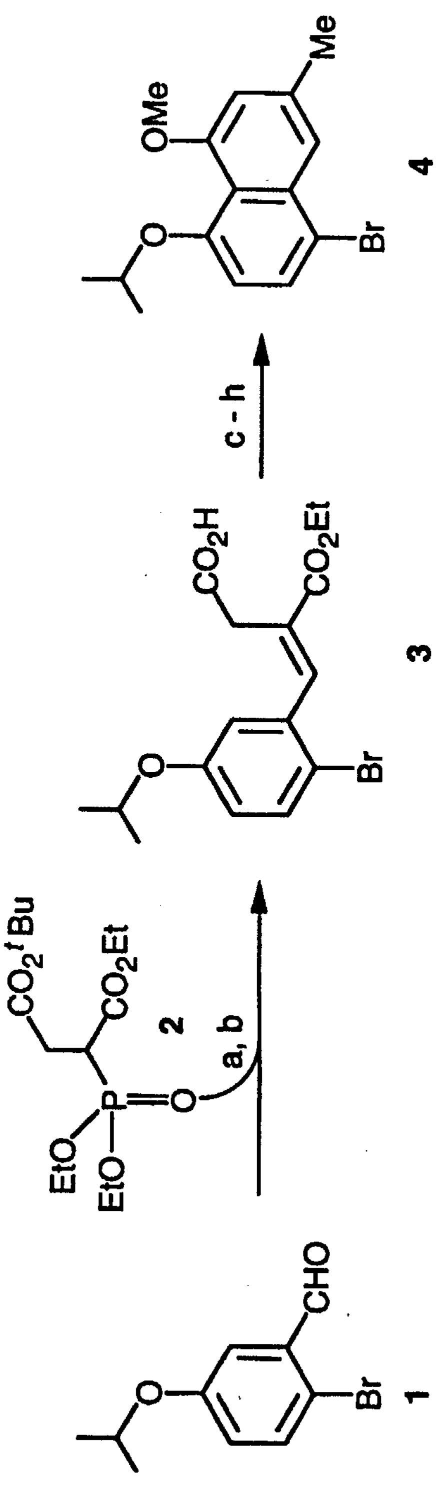


1 2 3 4

FIG. 2

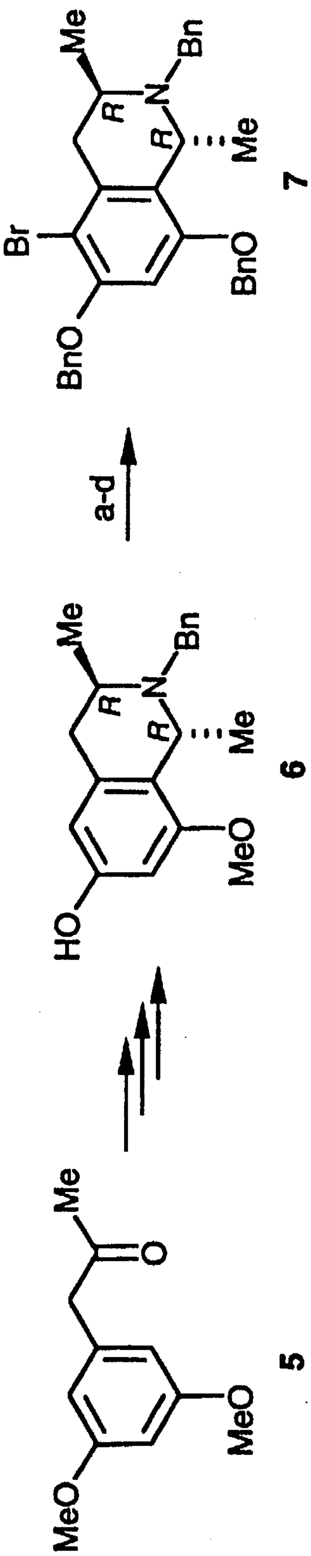


5 6 7

FIG. 3

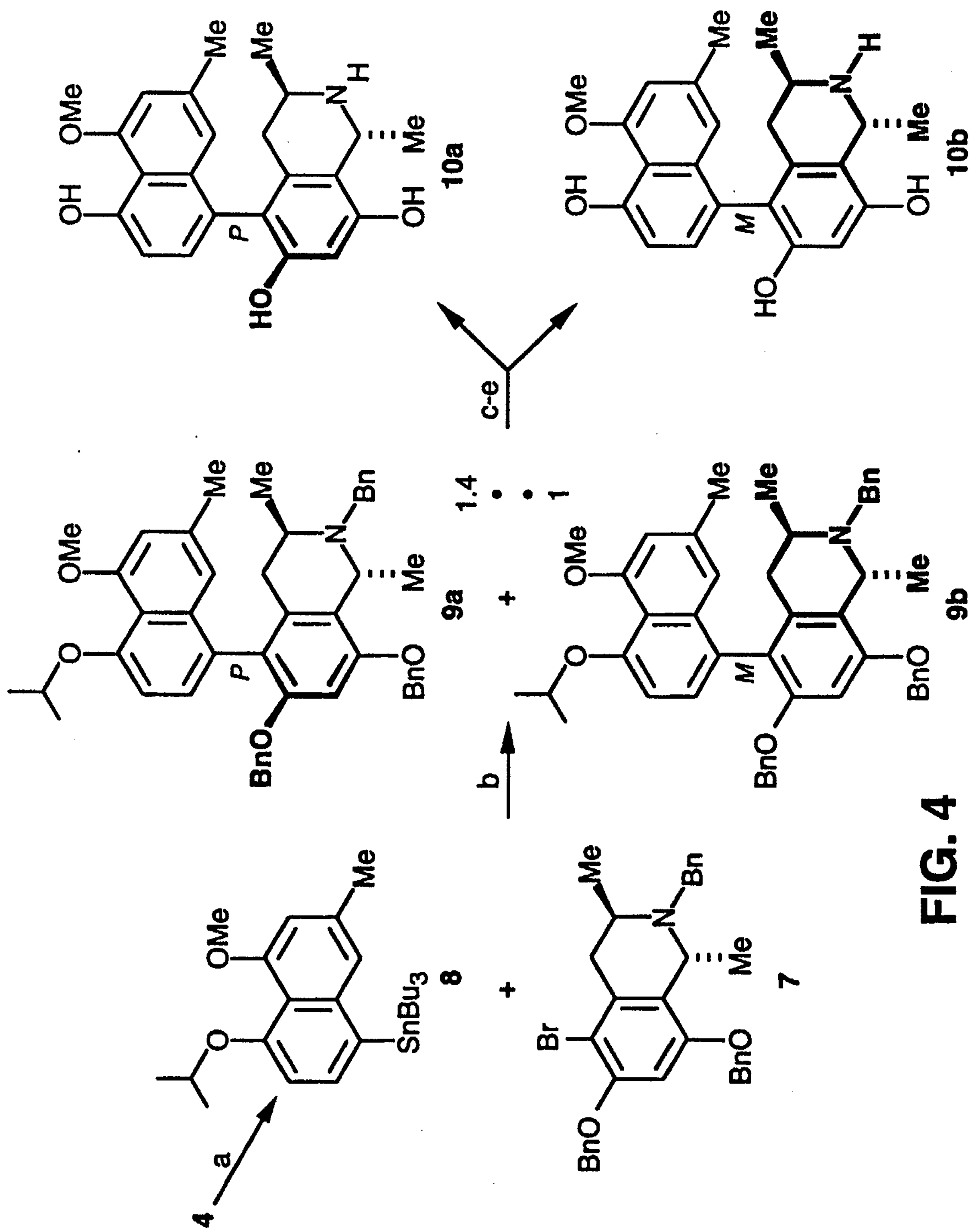


4

8

7

9a

9b

10a

10b

FIG. 4

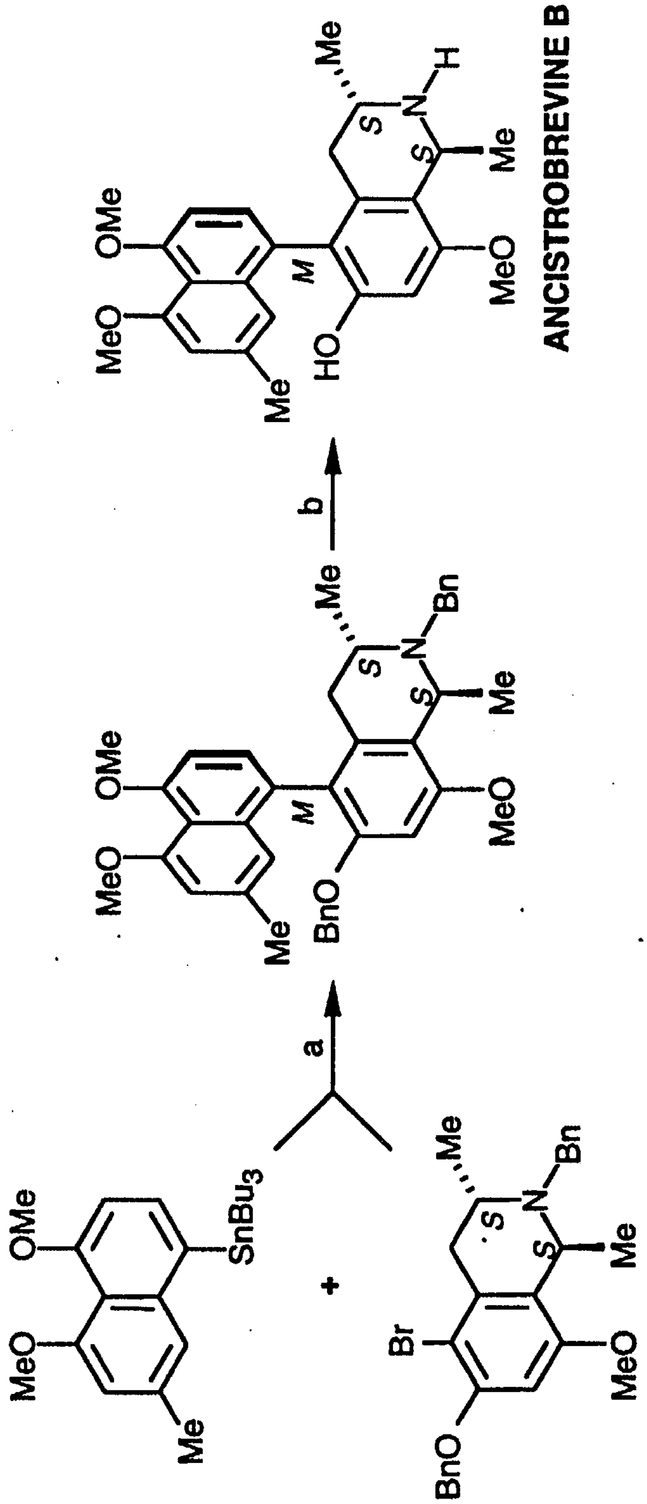


ANCISTROBREVINE B

FIG. 5

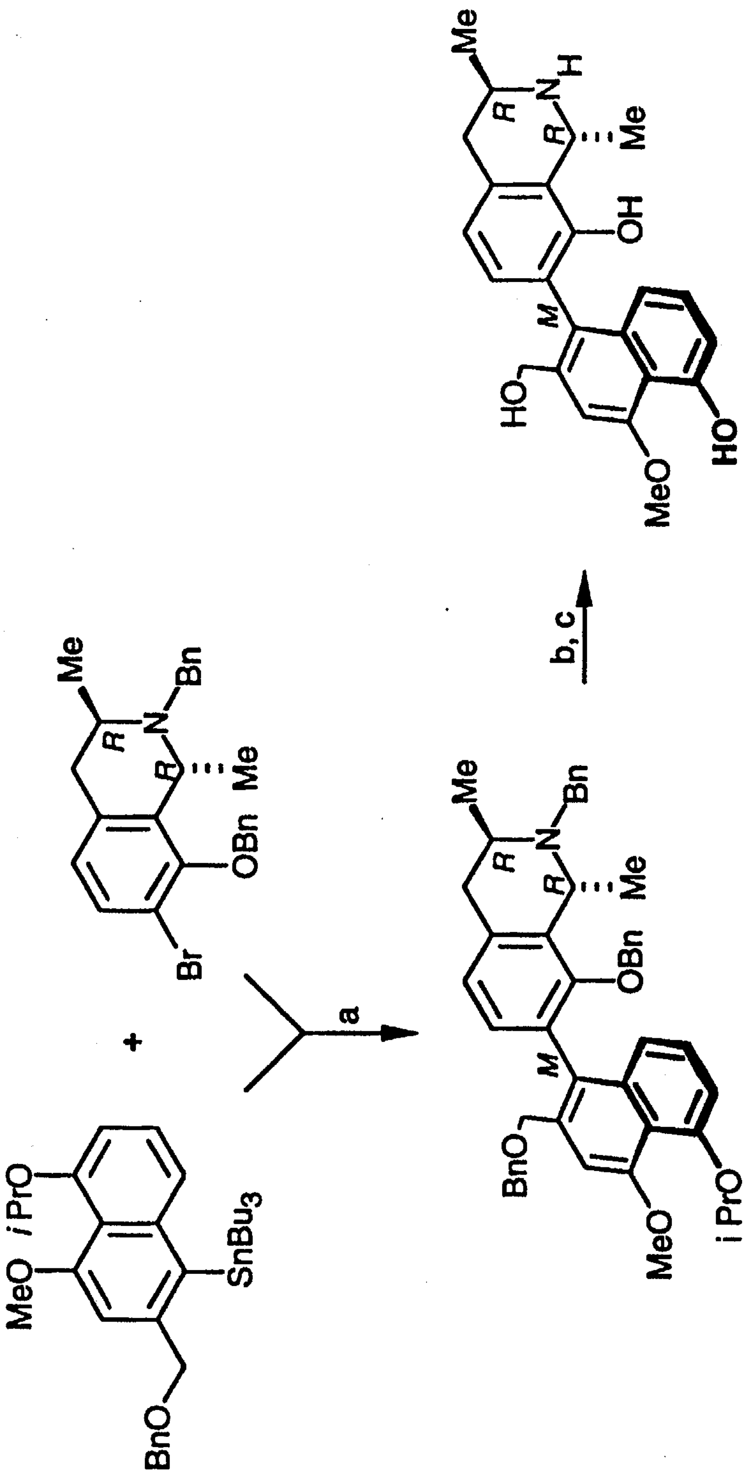


DIONCOPELTINE A

FIG. 6

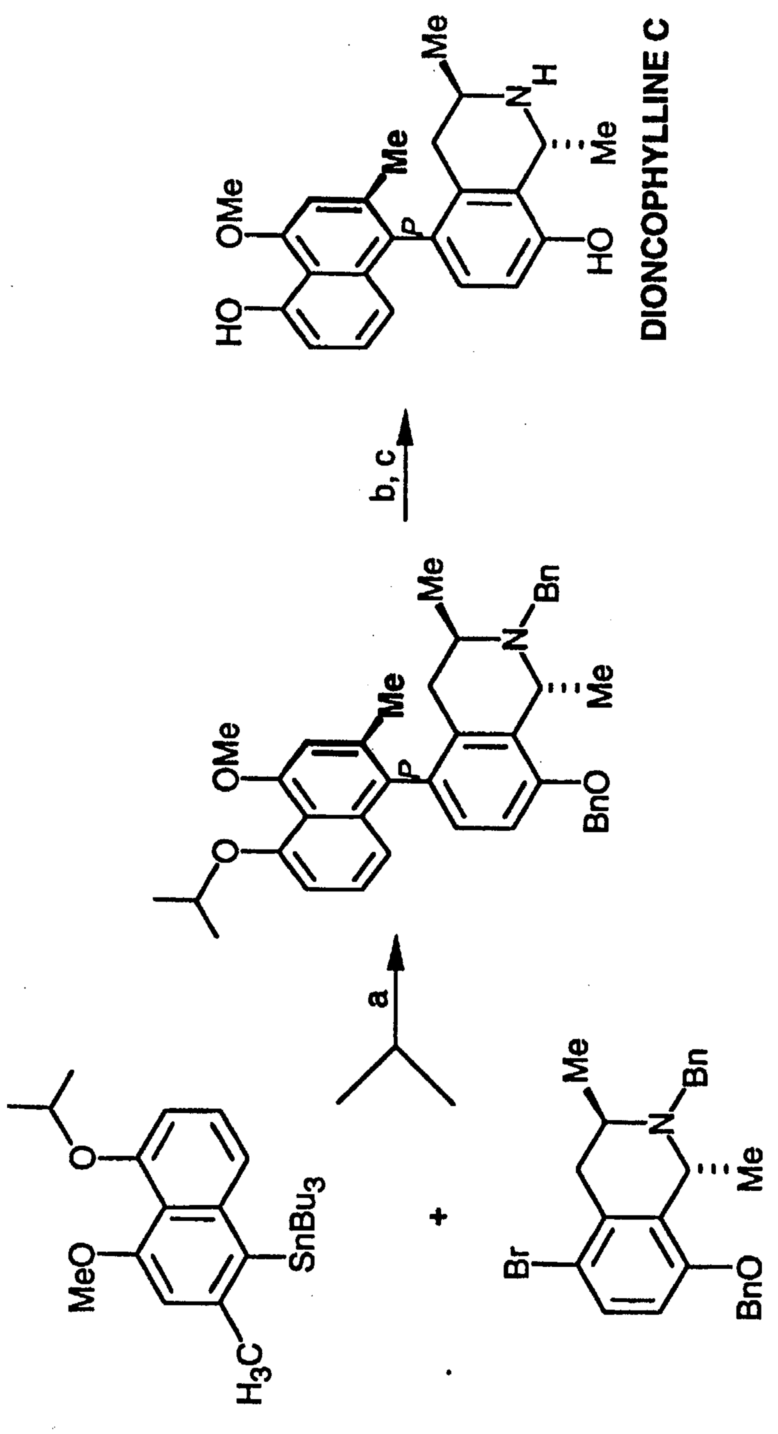


DIONCOPHYLLINE C

FIG. 7

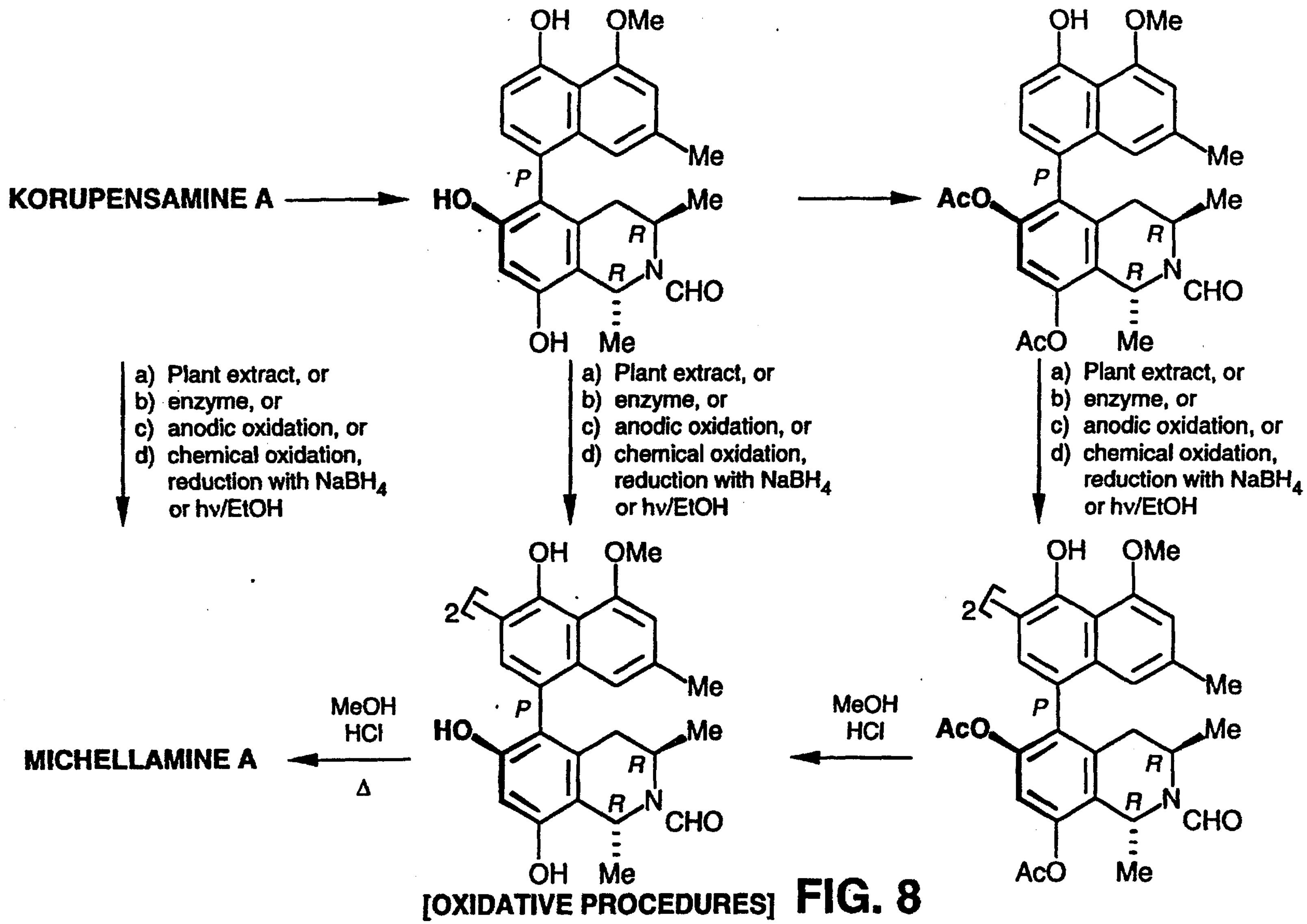


[OXIDATIVE PROCEDURES] FIG. 8

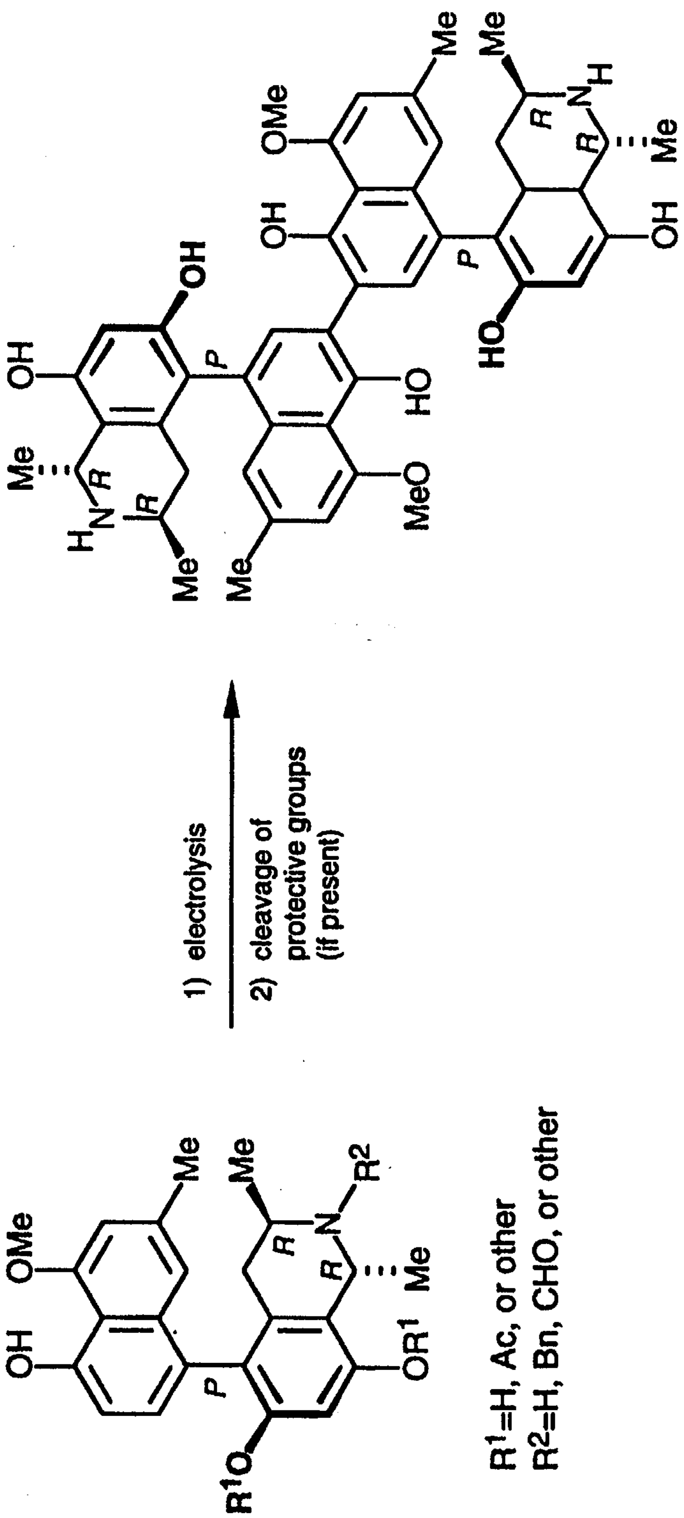
1) electrolysis
2) cleavage of protective groups (if present)
$R^1$=H, Ac, or other
$R^2$=H, Bn, CHO, or other

FIG. 9

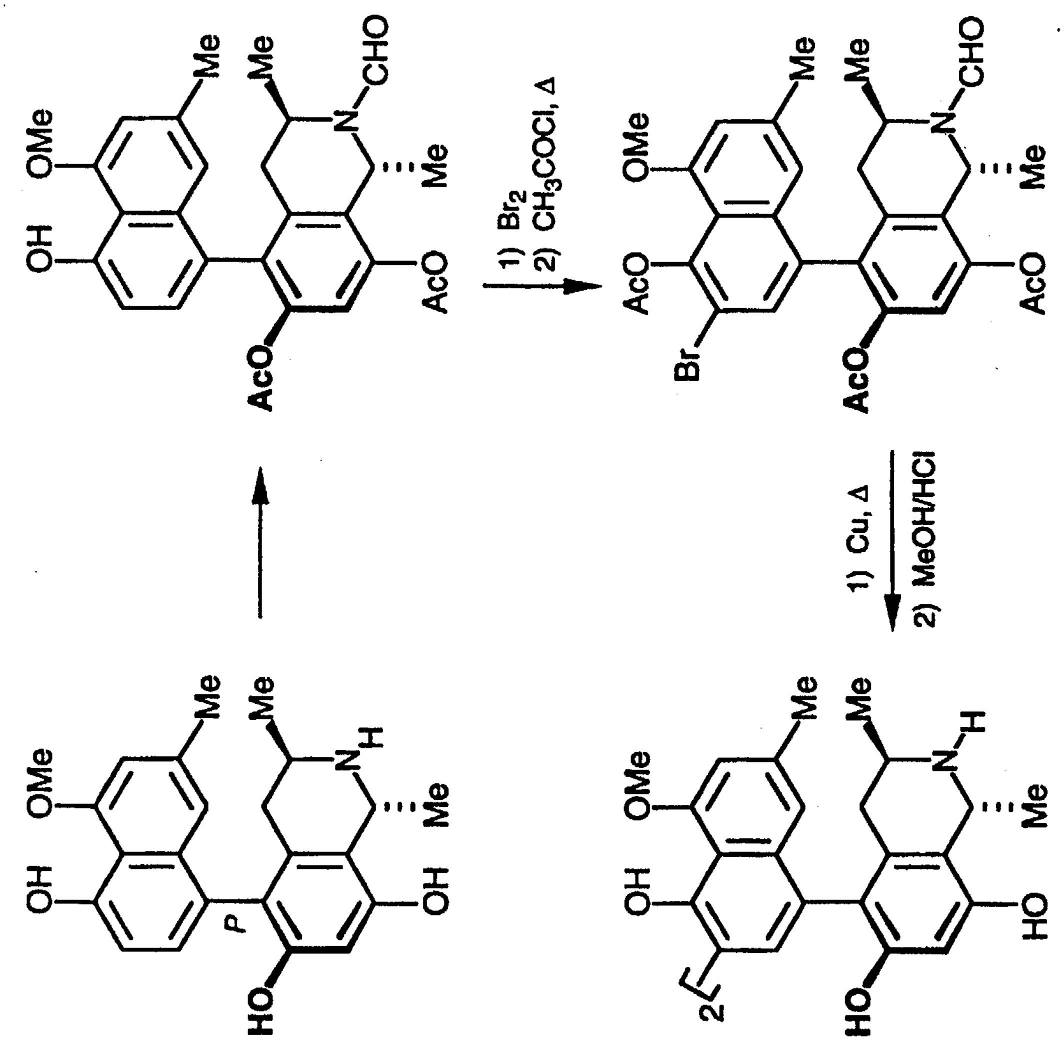


FIG. 10

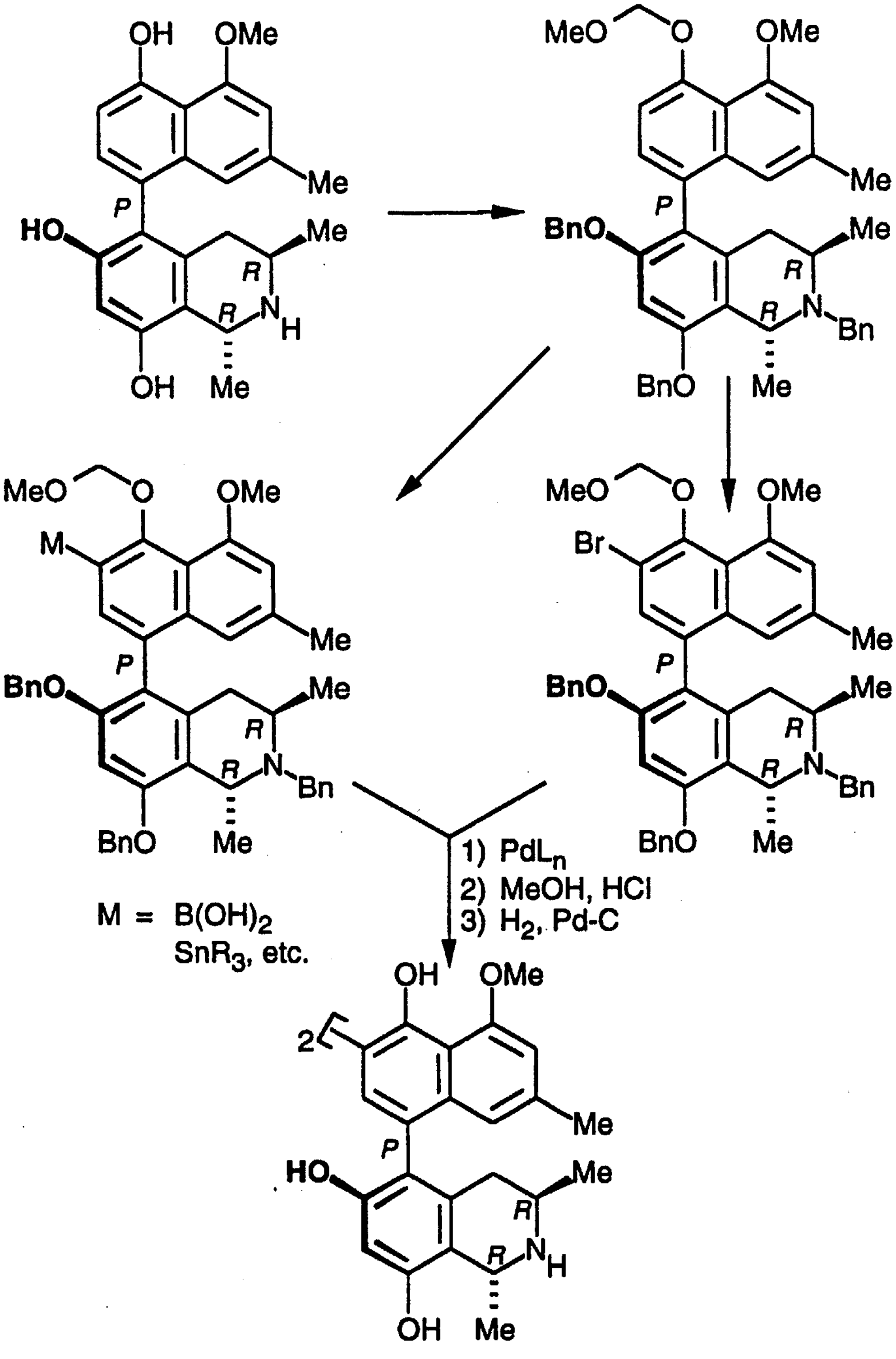


["REDOX-NEUTRAL" PROCEDURES]

**FIG. 11**

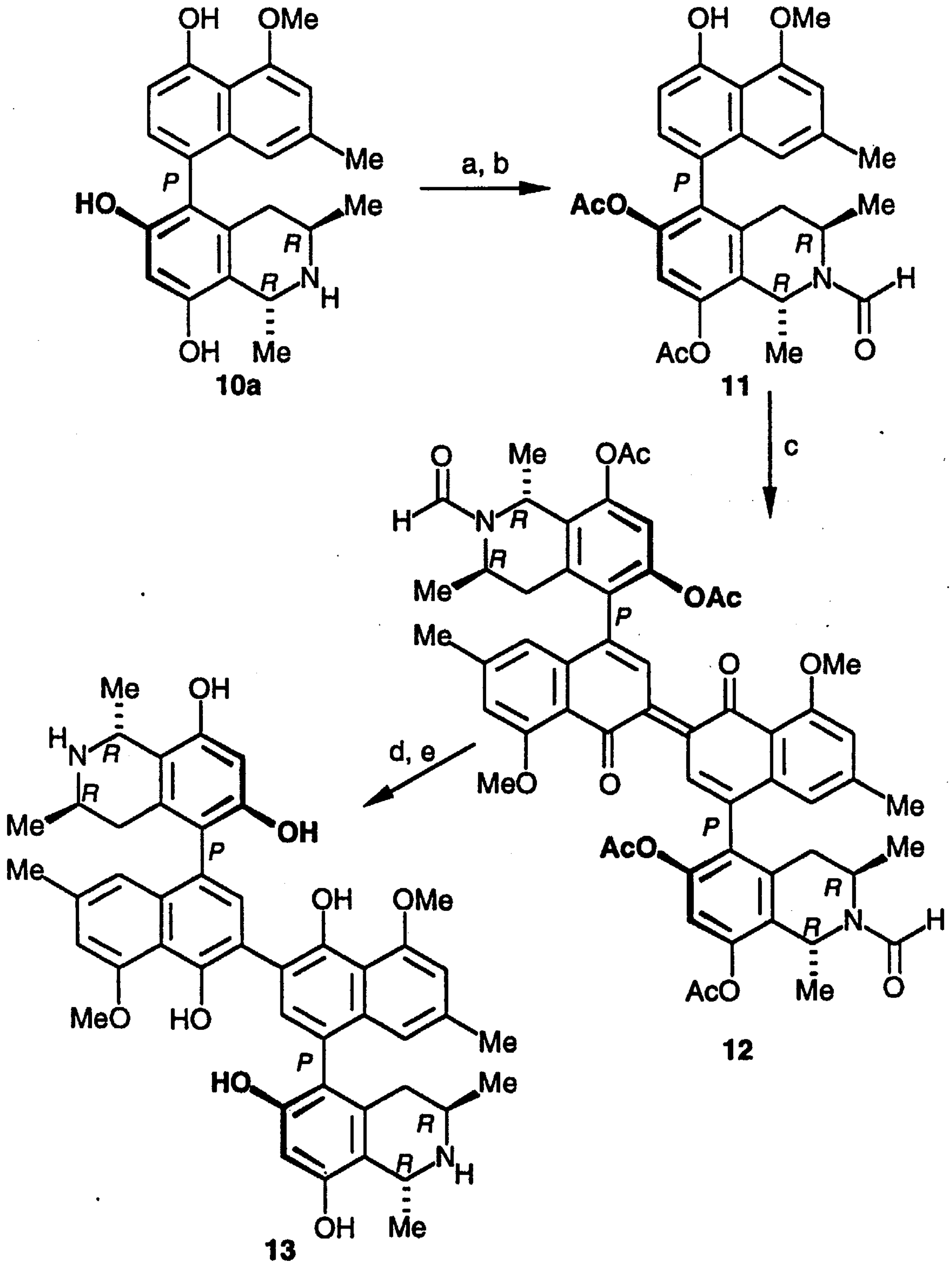


10a

11

12

13

FIG. 12

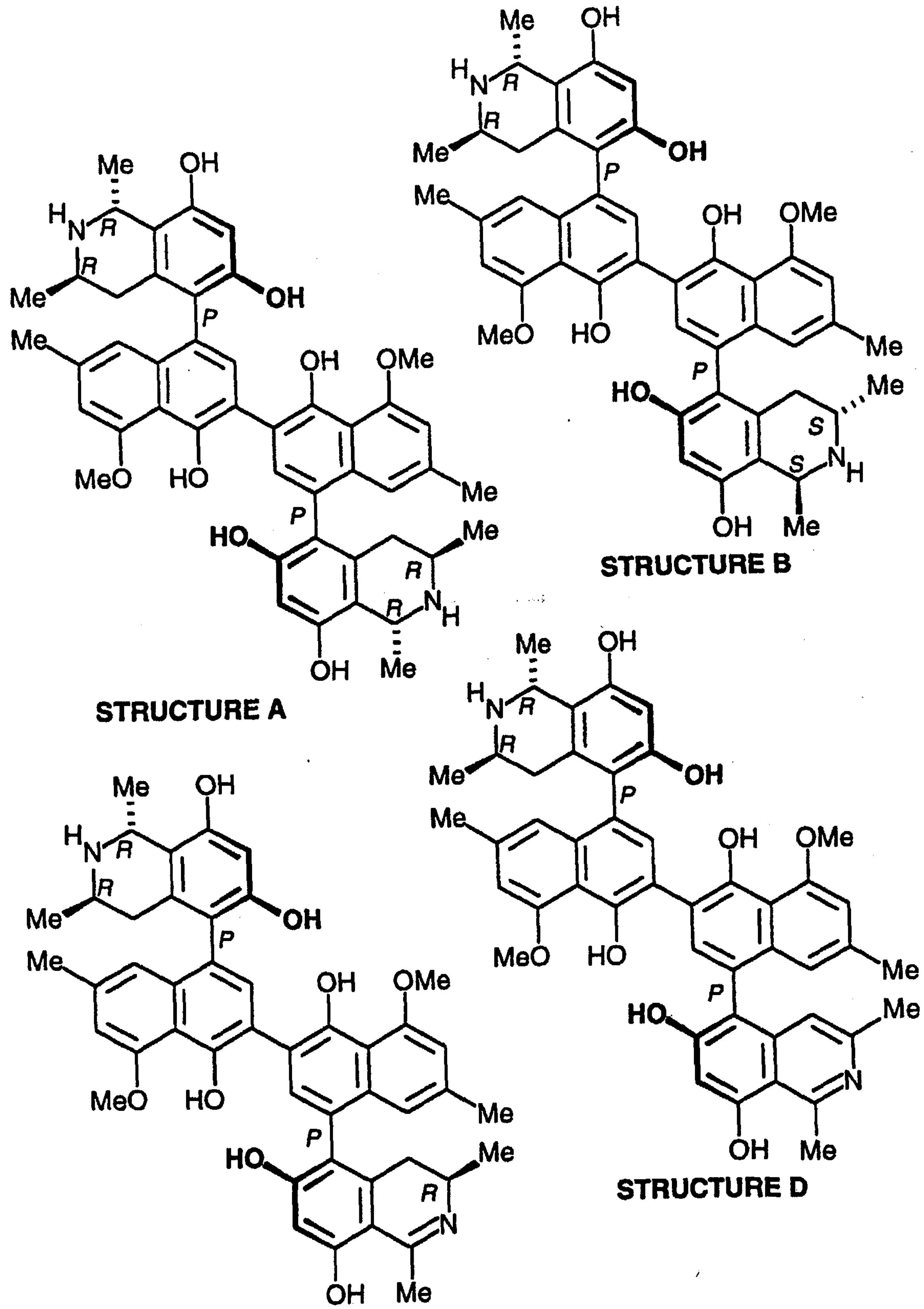

STRUCTURE A

STRUCTURE B

STRUCTURE C

STRUCTURE D

FIG. 13

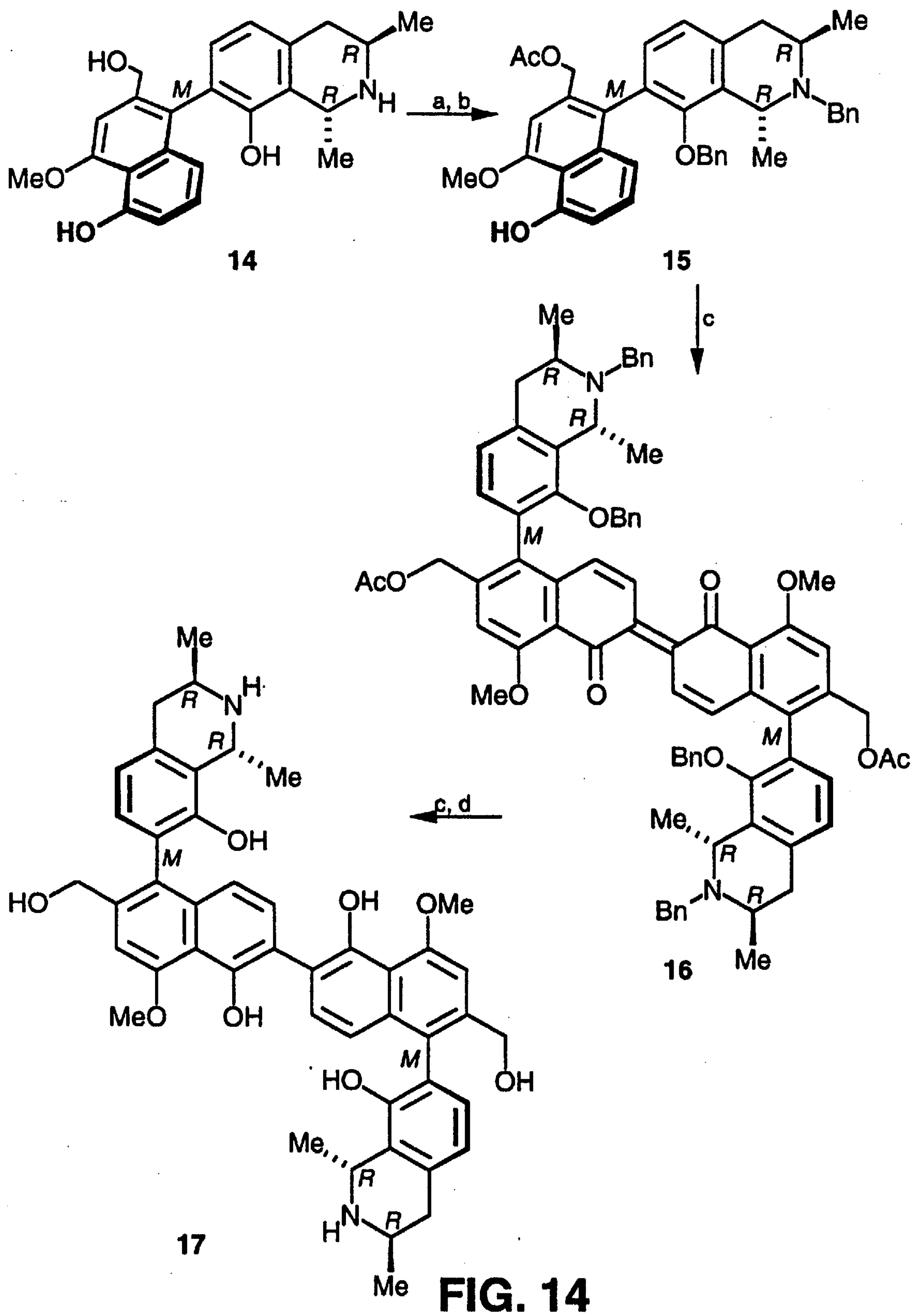


14

15

16

17

FIG. 14

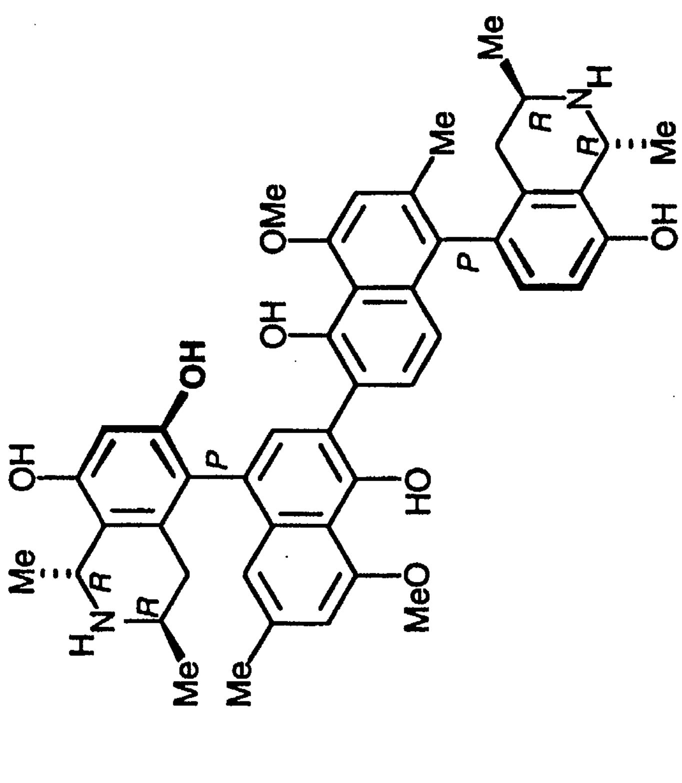

STRUCTURE A

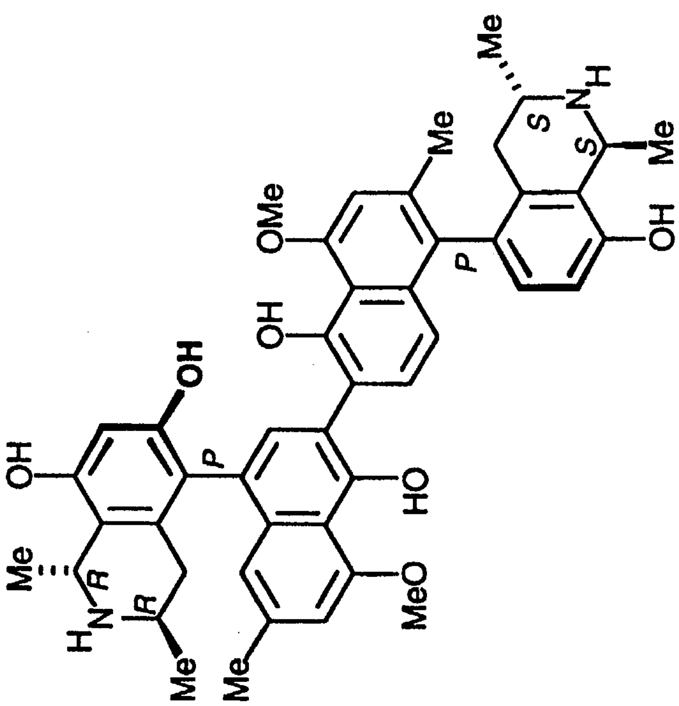

STRUCTURE B

FIG. 15

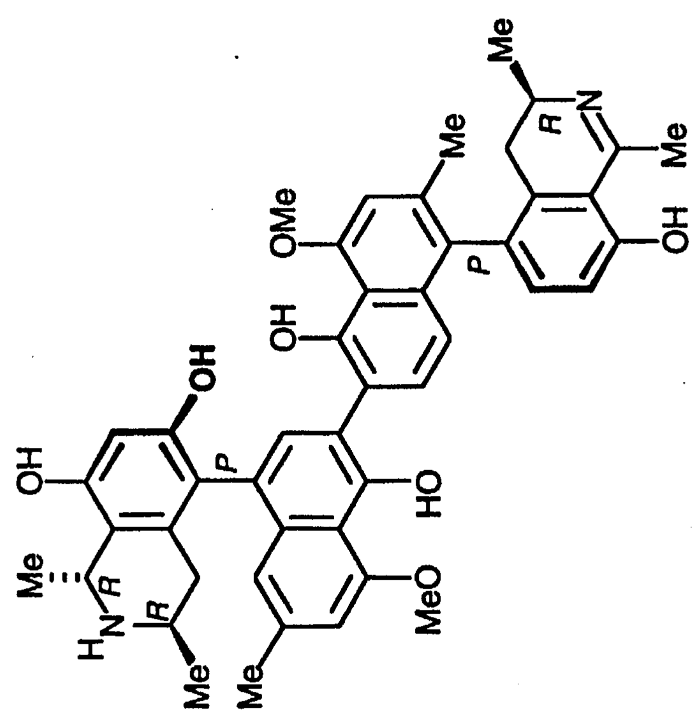

STRUCTURE C

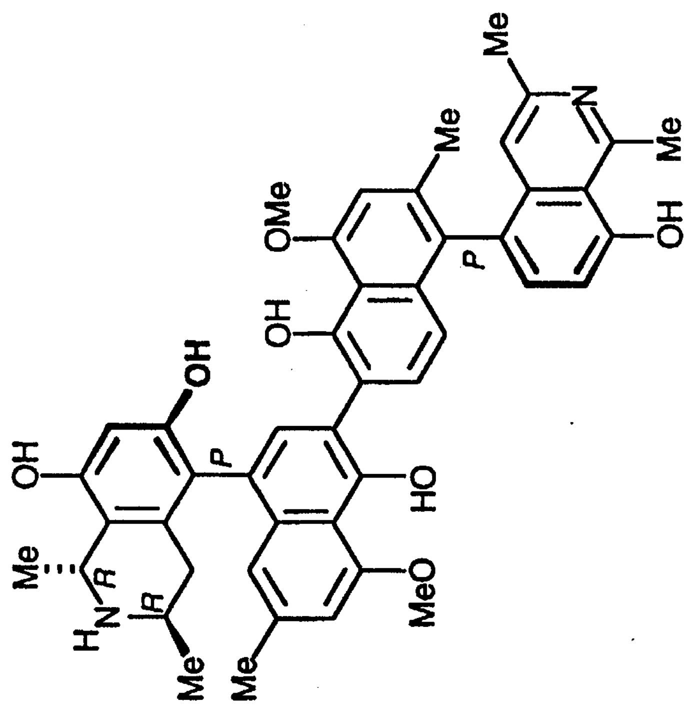

STRUCTURE D

FIG. 15
(CONTINUED)

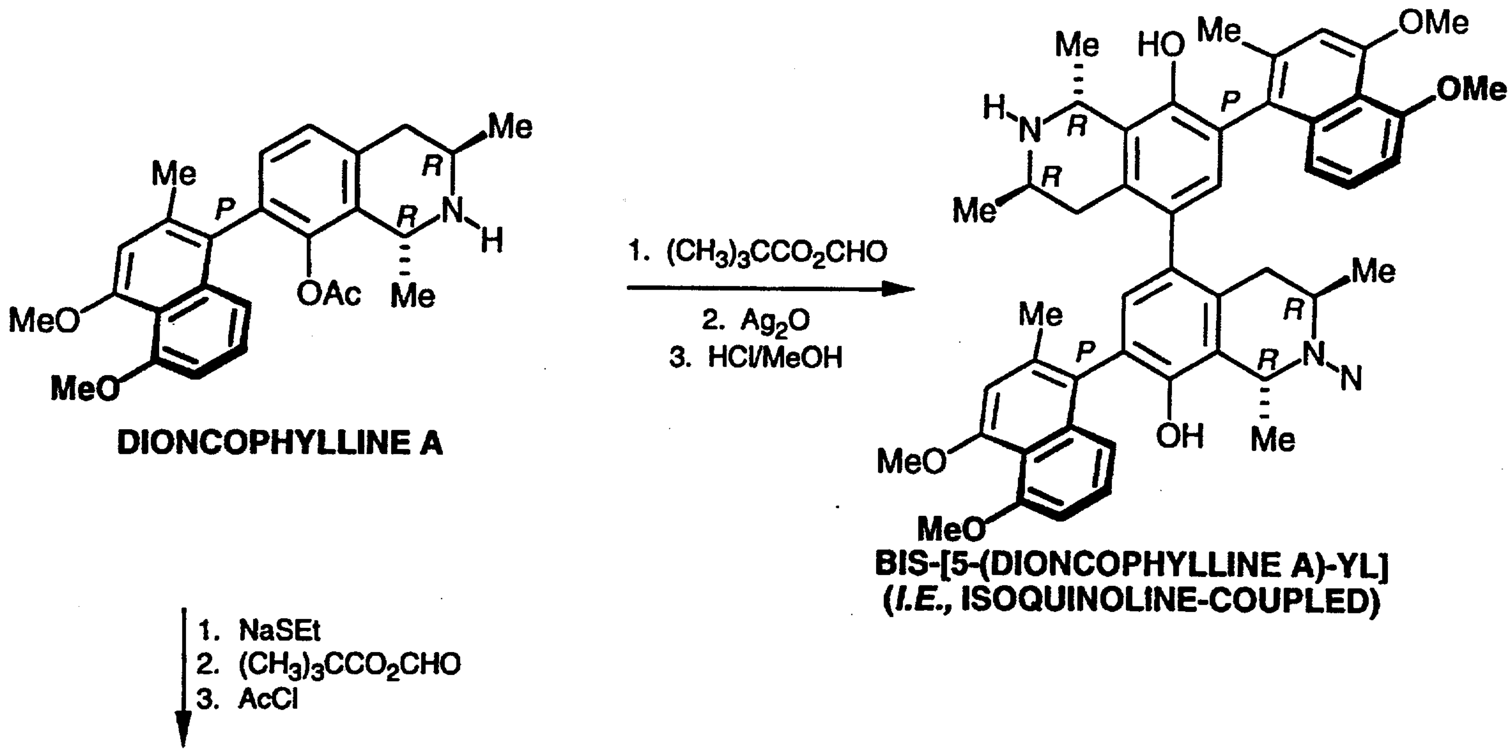


DIONCOPHYLLINE A

BIS-[5-(DIONCOPHYLLINE A)-YL]
(*I.E.*, ISOQUINOLINE-COUPLED)

FIG. 16

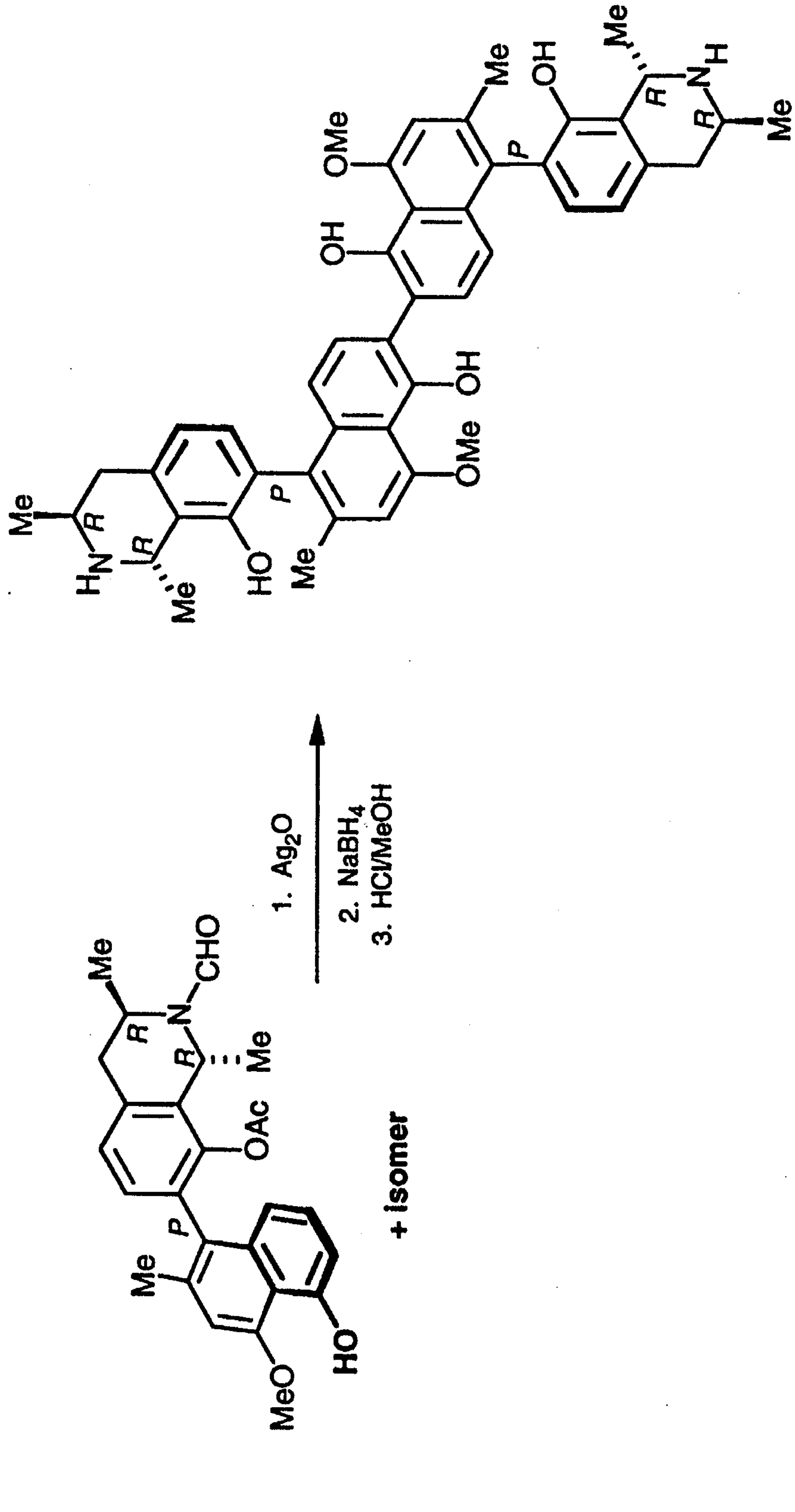


BIS-[6'-(DIONCOPHYLLINE A)-YL]
(*I.E.*, NAPHTHALENE-COUPLED)

**FIG. 16**
(CONTINUED)

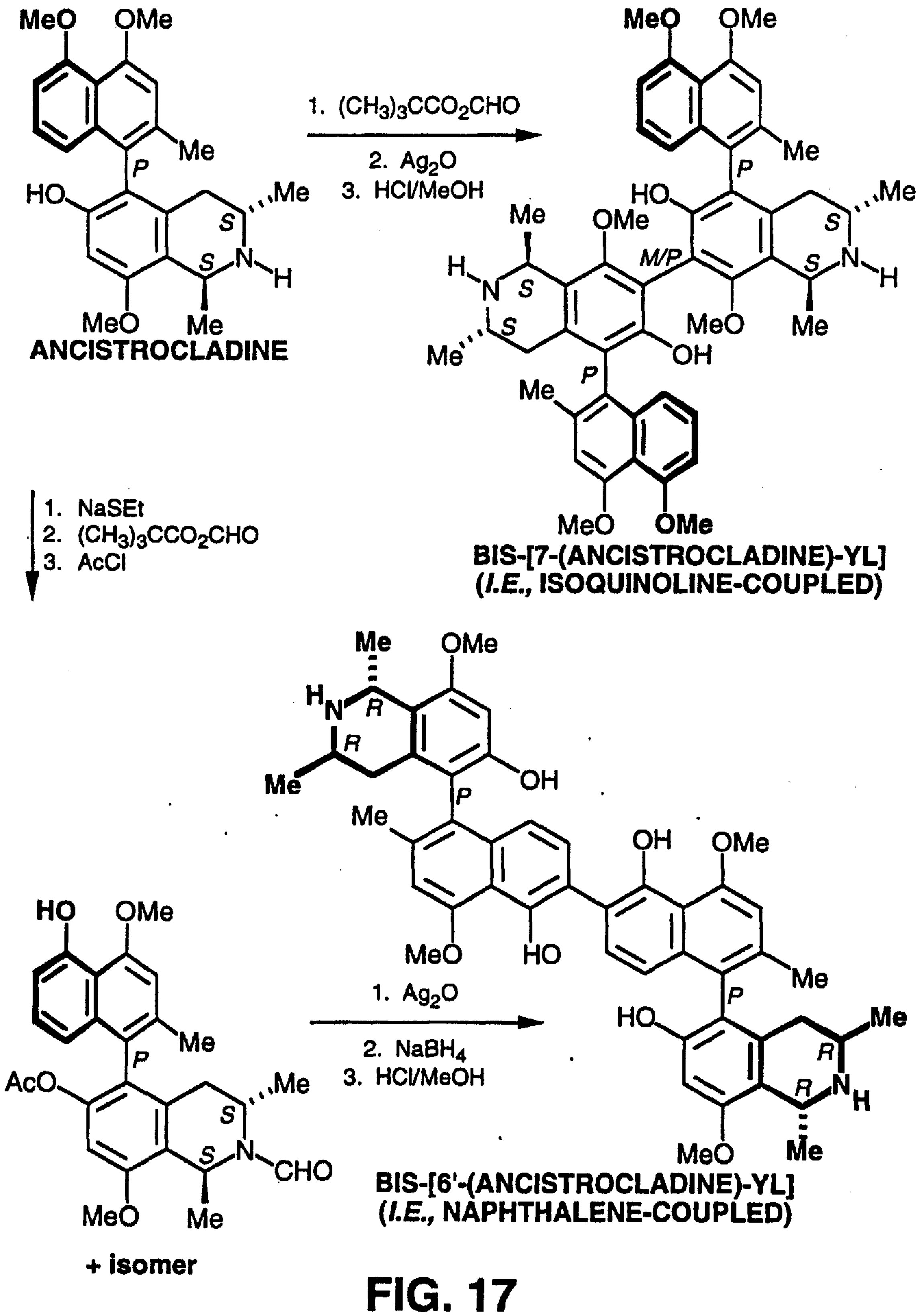


ANCISTROCLADINE

**BIS-[7-(ANCISTROCLADINE)-YL] (*I.E.,* ISOQUINOLINE-COUPLED)**

**+ isomer**

**BIS-[6'-(ANCISTROCLADINE)-YL] (*I.E.,* NAPHTHALENE-COUPLED)**

**FIG. 17**

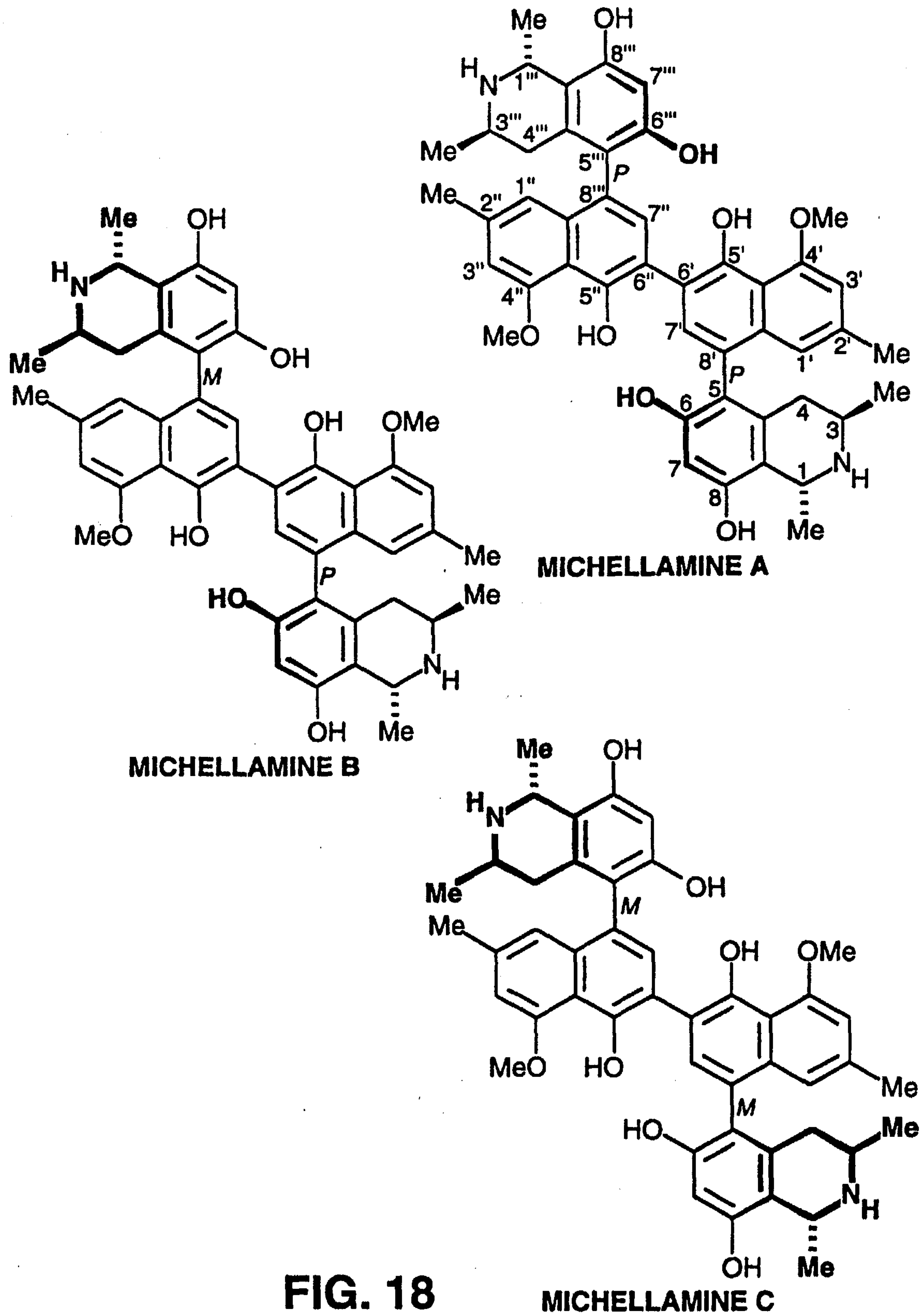

MICHELLAMINE A

MICHELLAMINE B

FIG. 18

MICHELLAMINE C

European Patent Office

# EUROPEAN SEARCH REPORT

Application Number
EP 03 00 5979

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 92 18125 A (US HEALTH)<br>29 October 1992 (1992-10-29)<br>* claims *<br>--- | 1-6 | C07D217/02<br>C07D217/04 |
| A | EP 0 515 856 A (BASF AG)<br>2 December 1992 (1992-12-02)<br>* page 2, line 1 - line 30 *<br>--- | 1,6,7 | |
| P,A | US 5 409 938 A (BOYD MICHAEL R ET AL)<br>25 April 1995 (1995-04-25)<br>* claims *<br>----- | 1-11 | |

TECHNICAL FIELDS SEARCHED (Int.Cl.7)
C07D

The present search report has been drawn up for all claims

| Place of search | Date of completion of the search | Examiner |
|---|---|---|
| THE HAGUE | 8 May 2003 | Chouly, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03 82 (P04C01)

## ANNEX TO THE EUROPEAN SEARCH REPORT ON EUROPEAN PATENT APPLICATION NO. EP 03 00 5979

This annex lists the patent family membersrelating to the patent documents cited in the above-mentioned European search report.
The members are as contained in the European Patent Office EDP file on
The European Patent Office is in no way liable for these particulars which are merely given for the purpose of information.

08-05-2003

| Patent document cited in search report | Publication date | Patent family member(s) | Publication date |
|---|---|---|---|
| WO 9218125 A | 29-10-1992 | AT 213231 T | 15-02-2002 |
| | | AU 657549 B2 | 16-03-1995 |
| | | AU 1880592 A | 17-11-1992 |
| | | CA 2100066 A1 | 13-10-1992 |
| | | DE 69232417 D1 | 21-03-2002 |
| | | DE 69232417 T2 | 24-10-2002 |
| | | EP 0594795 A1 | 04-05-1994 |
| | | JP 1957368 C | 10-08-1995 |
| | | JP 6076383 B | 28-09-1994 |
| | | JP 6502186 T | 10-03-1994 |
| | | US 5455251 A | 03-10-1995 |
| | | WO 9218125 A1 | 29-10-1992 |
| | | US 5654432 A | 05-08-1997 |
| EP 0515856 A | 02-12-1992 | DE 4117080 A1 | 26-11-1992 |
| | | EP 0515856 A1 | 02-12-1992 |
| | | JP 5148108 A | 15-06-1993 |
| | | US 5260315 A | 09-11-1993 |
| US 5409938 A | 25-04-1995 | AU 690640 B2 | 30-04-1998 |
| | | AU 1747695 A | 29-08-1995 |
| | | CA 2183247 A1 | 17-08-1995 |
| | | EP 0745066 A1 | 04-12-1996 |
| | | JP 10501518 T | 10-02-1998 |
| | | WO 9521826 A1 | 17-08-1995 |

EPO FORM P0459

For more details about this annex : see Official Journal of the European Patent Office, No. 12/82